# EUROPÄISCHE PATENTSCHRIFT

(11) **EP 1 519 756 B1**
(45) Veröffentlichungstag und Bekanntmachung des Hinweises auf die Patenterteilung: **02.05.2007**
(21) Anmeldenummer: 03740431.6
(22) Anmeldetag: 05.07.2003
(51) Int. Cl.: A61K 49/08, A61K 49/10

(54) **VERWENDUNG VON PERFLUORALKYLHALTIGEN METALLKOMPLEXEN ALS KONTRASTMITTEL IM MR-IMAGING ZUR DARSTELLUNG VON INTRAVASALEN THROMBEN**
USE OF METAL COMPLEXES CONTAINING PERFLUOROALKYL AS CONTRAST AGENTS IN MAGNETIC RESONANCE IMAGING FOR REPRESENTING INTRAVASCULAR THROMBI
UTILISATION DE COMPLEXES METALLIQUES CONTENANT DU PERFLUOROALKYLE EN TANT QU'AGENTS DE CONTRASTE EN IMAGERIE PAR RESONANCE MAGNETIQUE POUR LA REPRESENTATION DE THROMBUS INTRAVASCULAIRES

(30) Priorität: 10.07.2002 DE 10231799
(43) Veröffentlichungstag der Anmeldung: 06.04.2005
(73) Patentinhaber: Bayer Schering Pharma Aktiengesellschaft, 13353 Berlin (DE)
(72) Erfinder: MISSELWITZ, Bernd, 16548 Glienicke (DE); PLATZEK, Johannes, 12621 Berlin (DE); KAWATA, Yoko, Takarazuka, 665-0883 (JP); WEINMANN, Hanns-Joachim, 14129 Berlin (DE); YOKAWA, Takishi, Tokyo, 154-0014 (JP); NIEDBALLA, Ulrich, 14195 Berlin (DE)
(86) Internationale Anmeldenummer: PCT/EP2003/007274
(87) Internationale Veröffentlichungsnummer: WO 2004/006965

(56) Entgegenhaltungen:
- EP-A- 1 088 558
- WO-A-00/56723
- WO-A-01/08712
- WO-A-01/77102
- WO-A-02/13874
- WO-A-02/13875
- WO-A-02/14309
- WO-A-95/33494
- WO-A-97/26017
- WO-A-98/16256
- WO-A-99/01161
- US-A- 6 019 959
- YU XIN ET AL: "High-resolution MRI characterization of human thrombus using a novel fibrin-targeted paramagnetic nanoparticle contrast agent" MAGNETIC RESONANCE IN MEDICINE, Bd. 44, Nr. 6, Dezember 2000 (2000-12), Seiten 867-872, XP001155744 ISSN: 0740-3194

## Beschreibung

Die Erfindung betrifft den in den Patentansprüchen gekennzeichneten Gegenstand, das heißt die Verwendung von perfluoralkylhaltigen Metallkomplexen, die eine kritische Mizellbildungskonzentration < 10⁻³ mol/l, einen hydro- dynamischen Mizelldurchmesser (2 Rh) > 1 nm und eine Protonen-Relaxivity im Plasma (R¹) > 10 l /mmol·s aufweisen, als Kontrastmittel im MR-Imaging zur Darstellung von intravasalen Thromben.

Unter Thrombose versteht man die Bildung eines Blutgerinnsels (Thrombus) in einem Blutgefäß und die dadurch hervorgerufene Einengung bzw. Verstopfung dieses Gefäßes. Am häufigsten finden sich Thrombosen in den Venen (Phlebothrombose). Betroffen sind hier bevorzugt die Venen der unteren Körperhälfte (tiefe Bein- und Beckenvenen). Andere Teile des Kreislaufsystems können jedoch ebenfalls betroffen sein: Herzklappen, Herzspitze, Herzkranzgefäße, Hirn-gefäße, Arterien im Bereich des Darmes, Beinarterien sowie Venen von Bein und Becken, des Mastdarms (Hämorrhoiden) und des Armes. Durch Verschleppung des Thrombus kann es zu einer Lungenembolie kommen, die im ungünstigsten Fall tödlich endet.

Thrombosen der tiefen Leitvenen stellen ein großes sozialmedizinisches Problem dar. In Deutschland werden pro Jahr 60.000 Menschen wegen Thrombosen und ihrer Folgeerscheinungen behandelt. In den USA tritt jährlich bei 48 von 100.000 Einwohnern eine akute Thrombose der tiefen Leitvenen des Beines und des Beckens auf. Etwa 12% aller stationären Patienten entwickeln klinisch erkannte tiefe Bein- oder Beckenvenenthrombosen. Etwa 20 bis 30% aller allgemeinchirurgischer Patienten und mehr als 50% aller Patienten nach orthopädisch/unfallchirurgischen Eingriffen erleiden tiefe Beinvenenthrombosen, wobei bei ca. 1% dieser Patienten eine Lungenembolie mit klinischer Symptomatik auftritt (Leitlinien zu Diagnostik und Therapie in der Gefäßchirurgie, herausgegeben vom Vorstand der Dt. Ges. f. Gefäßchirurgie; Deutscher Ärzteverlag, Köln 1998).

Die entscheidenden, eine Thrombose verursachenden Mechanismen, wurden schon 1856 von Rudolf Virchow beschrieben und als Virchow'sche Trias nach ihm benannt. Dabei handelt es sich um eine Schädigung der Gefäßwand, die Verlangsamung des Blutflusses und eine erhöhte Gerinnungsneigung des Blutes infolge einer Veränderung der Blutzusammensetzung. Während für die venöse Thrombose (Phlebothrombose) die Verlangsamung des Blutflusses und eine erhöhte Gerinnungsneigung im Vordergrund stehen, ist bei der Entstehung der selteneren arteriellen Thrombose die Schädigung der Gefäßwand, meist als Folge der Arteriosklerose, mit der Ablagerung von Blutplättchen (Thrombozyten) von entscheidender Bedeutung.

Der Thrombus bleibt nur wenige Tage in seiner ursprünglichen Form erhalten. Nach einem Strukturwandel ist er in seinem Endzustand narbig umgewandelt und das Gefäß teilweise wieder durchgängig (rekanalisiert). Das Ziel der Therapie ist in erster Linie die Wiederherstellung des Blutflusses. Diese Therapie ist vom Alter des Thrombus abhängig und nur innerhalb der ersten 10 Tage nach Entstehung des Thrombus erfolgreich. Die Wiederherstellung des Blutflusses kann zum einen durch eine medikamentöse Auflösung der Thromben (Thrombolyse) erfolgen. Zum anderen stehen chirurgische Methoden zur Verfügung, entweder die Beseitigung des Verschlusses durch Entfernung des Gerinnsels (Thrombektomie) oder die Überbrückung des verschlossenen Gefäßabschnittes durch eine Gefäßplastik (Bypass). In zweiter Linie zielt die Therapie der Thrombose darauf ab, ein weiteres Wachstum des Thrombus zu verhindern und Spätfolgen bzw. Komplikationen zu vermeiden.

Die Diagnose von Thrombosen in der klinischen Praxis erfolgt hauptsächlich durch bildgebende Verfahren. Eine sehr gut geeignete Methode zum Nachweis einer Thrombose sowie zur Feststellung ihrer Ausdehnung ist die röntgenologische Kontrastmitteluntersuchung (Phlebographie). Nachteile sind die Exposition mit ionisierenden Strahlen und die mit jodhaltigen Kontrastmitteln verbundenen Nebenwirkungen. Daher ist die initiale Untersuchungsmethode bei Verdacht auf tiefe Beinvenenthrombose in vielen klinischen Einrichtungen die farbkodierte Duplexsonographie (B-Scan plus PW-Doppier), die jedoch extrem untersucherabhängig ist. Weitere nicht-invasive, bildgebende Verfahren zur Darstellung von luminalen Gefäßveränderungen sind die Arteriographie, CT-Angiographie und MR-Angiographie, sowie Methoden der Nuklearmedizin.

So können Thromben durch mit Indium-111 markierten Blutkörperchen als imaging agens dargestellt werden (Thakur et al., Thromb. Res. 9: 345, 1976; Powers et al., Neurology 32: 938, 1982). Auch die Jodisotope J-125 und J-131 sind für Imagingzwecke geeignet (Pang, US 5,011,686, 1991). Eine weite Verbreitung als label hat das Technetiumisotop Tc-99m. Mit ihm werden Peptide und besonders monoklonale Antikörper gelabelt ( Berger, US 5,024,829, 1991; Dean et al., US 4,980,148, 1990; US 5,508,020, 1996; US 5,645,815, 1997; WO 00/61195; US 6,171,578, 2001; EP 1171166, 2002). Verbindungen, die sowohl für die Szintigraphie als auch für das MR-imaging geeignet sind, werden von Abelman (US 5,656,600, 1997) beschrieben. DuPont Pharmaceuticals beschreibt in WO 01/77102 Konjugate aus Metallkomkomplex und Pyridinonen, die als Kontrastmittel zur Diagnose von Thrombosen mit Hilfe der Szintigraphie, der Computertomographie oder des MR-imaging geeignet sind.

Einen weiten Umfang hat die Literatur zur MR-Angiographie zur Darstellung intravasaler Thromben. In der Anmeldung WO 95/09013 beschreibt Cytogen Polypeptide als Komplexbildner für paramagnetische Metallionen.

Nycomed nennt in der Anmeldung WO 95/24225 polymere Komplexbildner für das Thrombus-imaging. An ein Backbone - beispielsweise Polylysin - sind Komplexbildner wie DOTA oder DO3A gebunden.

Sandoz beschreibt in der WO 95/20603 paramagnetische DTPA-Konjugate, die für das Thrombus-imaging geeignet sind.

In dem Barne-Jewish Hospital Patent US 5,780,010 werden spezifisch bindende (Biotin-Avidin-Komplexe) Konjugate als Kontrastmittel für das Thrombus-imaging beschrieben. Auch das Burnham Institute beschreibt in WO 98/16256 spezifisch (an Integrin) bindende Reste, die ein Thrombus-imaging ermöglichen. In diesen Konjugaten sind paramagnetische Komplexe von DTPA, EDTA oder DOTA als signalgebende Reste enthalten.

Konjugate aus einem Guanidinderivat und paramagnetischen Komplexen werden von 3-Dimensional Pharmaceuticals als Kontrastmittel für das Thrombus-imaging in WO 01/04117 beschrieben.

Konjugate aus Komplexen der DTPA, DOTA oder DO3A und Polypeptiden werden von EPIX in WO 01/09188 und EP 1203026 als imaging agents für das Thrombus-imaging beschrieben.

In EP 885545 nennt Pilgrimm superparamagnetische Eisenoxide als Kontrastmittel für die Thrombosediagnostik mit Hilfe des MRI.

Ebenfalls partikulär (USPIO) ist das MR-Kontrastmittel, das in WO 02/22011 zur Diagnose von Thromben beschrieben wird.

Nachteilig bei den Konjugaten ist, dass sie neben dem diagnostisch wirksamen Teil einen weiteren Bestandteil (Peptid oder Pharmakon) enthalten, so dass Nebenwirkungen, wie beispielsweise eine verringerte Verträglichkeit, öfters auftreten.

Von den partikulären Kontrastmitteln wird die Thrombusdarstellung von EP 885545 zwar beschrieben, aber nicht experimentell belegt.

In WO 02/22011 werden Bilder gezeigt, allerdings werden diese nach T2* gewichteten Flash-Sequenzen erhalten, so dass die Thromben nach Kontrastmittelgabe nur signalarm sind.

Die Untersuchung des Patientenblutes auf das Vorhandensein einer erhöhten Konzentration von D-Dimeren hat in jüngerer Zeit größere klinische Bedeutung erlangt. Nach eingehenderen Studien schließt eine Konzentration von weniger als 500 µg/l D-Dimeren im Blut das Vorhandensein einer Thromboembolie mit sehr hoher Wahrscheinlichkeit aus (Wells PS, Brill-Edwards P, Stevens P, et al. A novel and rapid whole blood assay for D-dimer in patients with clinical suspected deep vein thrombosis. Circulation 1995; 91: 2184 -2187 ). Die Spezifität des D-Dimer-Nachweises ist aber so gering, dass aus einer Erhöhung der Konzentration im Blut nicht auf eine Thrombose geschlossen werden kann.

Die Bildgewinnung mit Hilfe der Kernmagnetischen Resonanz (MRI) ist ein modernes, nicht-invasives radiologisches Verfahren, das mit einer sehr guten räumlichen und zeitlichen Auflösung die Darstellung von physiologischen und pathophysiologischen Strukturen ermöglicht. In der Diagnostik der tiefen Bein- und Beckenvenenthrombose ist die MR-Venographie (MRV) als Alternative zur Phlebographie und farbkodierten Dopplersonographie (FKDS) im Bereich der suprapoplitealen Venen seit längerem methodisch etabliert. In den letzten Jahren wurden auch Studien zur MRV der Unterschenkelvenen publiziert.

Grundsätzlich lässt sich aus dem Datensatz einer kontrastverstärkten 3D-MR-Angiographie das venöse System durch Perfusionsphasensubtraktion selektiv darstellen und nach Injektion eines verdünnten paramagnetischen Kontrastmittels über eine Fußrückenvene direkt visualisieren. Bei der MR Angiographie mit herkömmlichen, extrazellulären, paramagnetischen Substanzen wird nicht immer ein homogener Gefäßkontrast erzielt, was im Einzelfall eine Beurteilung erschwert. Mit dem erwarteten Einsatz höher konzentrierter Kontrastmittel oder sog. »Blood Pool Agents« könnte jedoch die schnellere, kontrastmittelgestützte 3D-MRA von Vorteil sein. Mit T2-Turbo-Spinecho (TSE)- und Time-of-Flight (TOF)-Sequenzen (ohne Kontrastmittel) lässt sich auch am offenen Niederfeldtomographen ein ausreichendes Signalniveau erzielen (König C. et al., MR-Venographie am offenen Niederfeldtomographen unter Verwendung manueller Flussaugmentation; Rofo, Fortschr. Geb. Röntgenstr. Neuen Bildgeb. Verfahr. 2001; 173: 810-814). Fluss-sensitive MRA-Techniken sind dagegen für die Diagnostik von Thrombosen wenig geeignet, da in Venen, insbesondere in solchen mit einer Thrombose, die Flussgeschwindigkeit im nicht-thrombosierten Anteil oft zu gering ist.

Die Verwendung spezifischer Kontrastmittel mit selektiver Anreicherung in bestimmten Geweben und Organen könnte den diagnostischen Wert der MR-Bildgebung bedeutend erhöhen. Kontrastmittelzubereitungen mit selektiver Anreicherung in intravasalen Thromben könnten Lokalisation und Grad der Erkrankung zu einem frühen Zeitpunkt erfassen und damit eine zielgerichtete Therapie und Prophylaxe ermöglichen.

Es besteht daher weiterhin das Bedürfnis nach einem verträglichen, leistungsstarken Kontrastmittel zur Darstellung von arteriellen und venösen Thrombosen.

Aufgabe der vorliegenden Erfindung war es daher, Kontrastmittel für die Darstellung von intravasalen Thromben im MR-Imaging zur Verfügung zu stellen, die die Anforderungen nach
selektiver Anreicherung
hoher Verträglichkeit
starkem Enhancement
vollständiger Ausscheidung
guter Wasserlöslichkeit
erfüllen.

Es wurde nun gefunden, dass überraschenderweise perfluoralkylhaltige Metallkomplexe, die eine kritische Mizellbildungskonzentration < 10⁻³ mol/l, einen hydrodynamischen Mizelldurchmesser (2 Rh > 1 nm) und eine Protonen-Relaxivity im Plasma (R¹) > 10 l/mmol·s aufweisen, als Kontrastmittel im MR-Imaging zur Darstellung von intravasalen Thromben sehr gut geeignet sind.

Verbindungen mit diesen Eigenschaften sind bereits in der WO 02/13874 als diagnostische Mittel für das Plaque-imaging mit Hilfe der MR-Technik beschrieben worden.

Die MR-Aufnahmen zeigen aber deutlich, dass Plaques und Thromben klar voneinander unterscheidbar sind. Dieses ist deshalb so wichtig, da Thromben in jungem Stadium mobil sein können und zu letal verlaufenden Embolien führen können.

Für die folgenden Versuche wurden die Gadoliniumkomplexe eingesetzt, da das Gadolinium von allen paramagnetischen Ionen den größten Einfluß auf die Signalverstärkung im MRI hat.

In einem in-vitro-Test (Bindung an ein Fibrin-Gel) konnte nachgewiesen werden, dass die erfindungsgemäßen Verbindungen bei einer Konzentration von 0.01 mmol Gd/I zu 79 % und bei einer Konzentration von 0.1 mmol Gd/l zu 39 % an das Fibrin-Gel binden und so eine sichere Unterscheidung zu Plaques ermöglichen.

Daneben wurde auch das Kontrastverhalten der erfindungsgemäßen Verbindungen in-vivo untersucht. In Kaninchen mit photochemisch induziertem Thrombus (PIT; i.v.-Injektion von Rose-Bengal und Bestrahlung mit Xenon-Licht) konnte zu verschiedenen Zeitpunkten nach intravenöser Applikation von 0.1 mmol Gd/kg Körpergewicht erfindungsgemäßer Verbindung (2 bis 48 h p.i.) mit T1-gewichteten Sequenzen ein deutliches Enhancement im induzierten Thrombus beobachtet werden. Zum Zeitpunkt 24 h p.i. war die Gadolinium-Konzentration im Thrombus ca. 4-mal höher verglichen zum Blut.
Für die MRI-Bildgebung werden im Thrombus, wo die Anreicherung der Verbindung erfolgt, Gadoliniumkonzentrationen von mindestens 50 µmol/l und höchstens 2500 µmol/l benötigt. Die Bildgebung kann nach 15 Minuten oder bis zu 48 Stunden nach Injektion der erfindungsgemäßen Verbindungen erfolgen. Da mit den erfindungsgemäßen Gadoliniumkomplexen vor allem die T1-Relaxationszeiten des Gewebes beeinflusst werden, sind T1-gewichtete Sequenzen am besten in der Lage, ein Enhancement im Thrombus nachzuweisen.

Als für die erfindungsgemäße Verwendung geeignete perfluoralkylhaltige Metallkomplexe werden amphiphile Verbindungen verstanden, die als unpolaren Teil eine Perfluoralkylseitenkette im Molekül aufweisen, die ggf. über einen lipophilen Linker mit dem Gesamtmolekül verbunden ist. Der polare Teil der erfindungsgemäßen Verbindungen wird durch ein oder mehrere Metallkomplexe und gegebenenfalls vorhandene weitere polare Gruppen gebildet.

In wässrigen Systemen zeigen diese amphiphilen Moleküle die für klassische Tenside (wie z.B. Natriumdodecylsulfat, SDS) charakteristischen Eigenschaften. So setzen sie die Oberflächenspannung des Wassers herab. Durch Tensiometrie lässt sich die sogenannte CMC (Kritische Mizellbildungskonzentration in mol/l) bestimmen. Hierzu wird die Oberflächenspannung in Abhängigkeit zu der Konzentration des zu vermessenden Stoffes bestimmt. Die CMC lässt sich aus dem Verlauf der erhaltenen Funktion Oberflächenspannung (c) ausrechnen. Die kritische Mizellbildungskonzentration der erfindungsgemäßen Verbindungen muß < 10⁻³ mol/l sein, vorzugsweise < 10⁻⁴ mol/l.

Die erfindungsgemäßen amphiphilen Verbindungen sind in Lösung assoziiert und liegen als Aggregate vor. Die Größe (2 Rh) derartiger Aggregate (z.B. Mizellen, Stäbchen, Oblaten etc.) lässt sich mit Hilfe der Photon-Correction-Spectroscopy (PCS) bestimmen.

Als zweites Kriterium dient daher der hydrodynamische Mizelldurchmesser 2 Rh, der > 1 nm sein muß. Besonders sind erfindungsgemäß solche perfluoralkylhaltigen Metallkomplexe geeignet, deren 2 Rh ≥ 3 nm beträgt, ganz besonders bevorzugt > 4 nm.

Sowohl die Bestimmung der CMC als auch die Photonenkorrelationsspektroskopie werden in H.-D. Dörfler, "Grenzflächen- und Kolloidchemie", Weinheim, New York, Basel, Cambridge, Tokyo, VSH 1994 beschrieben.

Als drittes Kriterium dient die Protonen-Relaxivity in Plasma (R¹) bei 40°C und einer Feldstärke von 0,47 Tesla. Die Relaxivity, die in [l/mmol·s] angegeben wird, ist das quantitative Maß für die Verkürzung der Relaxationszeit T¹ der Protonen. Für den erfindungsgemäßen Zweck muß die Relaxivity möglichst hoch sein und > 10 l/mmol·s betragen, vorzugsweise > 13 l/mmol·s, besonders bevorzugt > 15 l/mmol·s.

Die Relaxivity R¹ [l/mmol·s] der erfindungsgemäßen MR-Kontrastmittel wurde mit dem Gerät Minispec P 20 der Fa. Bruker bestimmt. Die Messungen wurden bei 40 °C und einer Feldstärke von 0,47 Tesla durchgeführt. Von jeder T1-Sequenz : 180° -TI-90°, Inversion Recovery, wurden 8 Meßpunkte aufgenommen. Als Medium diente Rinderplasma der Fa. Kraeber. Die Kontrastmittelkonzentrationen [mmol/l] lagen in den Ansätzen zwischen 0,30 und 1,16.

In einer Ausführungsform der vorliegenden Erfindung werden als bevorzugte Verbindungen die Verbindungen der allgemeinen Formel I gemäß der Ansprüche 8 bis 11 eingesetzt. Dabei handelt es sich um bekannte Verbindungen, die in WO 97/26017 beschrieben sind. Auch deren Herstellung kann dieser WO-Schrift entnommen werden. Überraschenderweise hat sich gezeigt, dass diese Verbindungen auch als MRI-Kontrastmittel zur Darstellung von Thromben sehr gut geeignet sind. Als ganz besonders bevorzugte Verbindungen werden die Metallkomplexe MK 2, 3 und 4 sowie MK 8, 9, 10 und 11 (vgl. auch Tabelle 1) eingesetzt.
In einer weiteren Ausführungsform der vorliegenden Erfindung werden als bevorzugte Verbindungen solche der allgemeinen Formel la gemäß der Ansprüche 12 bis 21 eingesetzt. Diese Verbindungen sind bekannt und in WO 99/01161 beschrieben. Ihre Verwendung als MRI-Kontrastmittel zur Darstellung von Thromben wurde bisher noch nicht beschrieben. Von diesen Verbindungen kommt ganz besonders bevorzugt der Metallkomplex MK 12 (vgl. Tabelle 1) zur Anwendung.
In einer weiteren bevorzugten Ausführungsform der Erfindung können die makrocyclischen Perfluoralkylverbindungen der allgemeinen Formel Ib 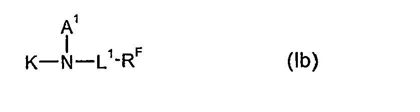 worin
- K: einen Komplexbildner oder einen Metallkomplex der allgemeinen Formel IIb 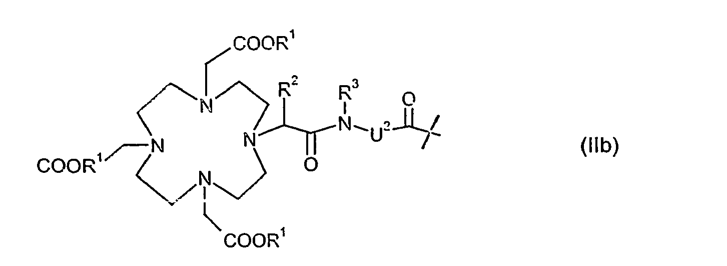 bedeutet,
wobei
R¹ für ein Wasserstoffatom oder ein Metallionenäquivalent der Ordnungszahlen 23-29, 42-46 oder 58-70,
R² und R³ für ein Wasserstoffatom, eine C₁-C₇-Alkylgruppe, eine Benzylgruppe, eine Phenylgruppe, -CH₂OH oder -CH₂-OCH₃, und
U² für den Rest L¹, wobei L¹ und U² unabhängig voneinander gleich oder verschieden sein können, steht,
- A¹: ein Wasserstoffatom, eine geradkettige oder verzweigte C₁-C₃₀-Alkylgruppe, die gegebenenfalls unterbrochen ist durch 1-15 Sauerstoffatome, und/oder gegebenenfalls substituiert ist mit 1-10 Hydroxygruppen, 1-2 COOH-Gruppen, einer Phenylgruppe, einer Benzylgruppe und/oder 1-5 - OR^{g}-Gruppen, mit R^{g} in der Bedeutung eines Wasserstoffatoms oder eines C₁-C₇-Alkylrestes, oder -L¹-R^{F} bedeutet,
- L¹: eine geradkettige oder verzweigte C₁-C₃₀-Alkylengruppe, die gegebenenfalls unterbrochen ist durch 1-10 Sauerstoffatome, 1-5 -NH-CO-Gruppen, 1-5 -CO-NH- Gruppen, durch eine gegebenenfalls durch eine COOH-Gruppe substituierte Phenylengruppe, 1-3 Schwefelatome, 1-2 -N(B¹)-SO₂- Gruppen, und/oder 1-2 -SO₂-N(B¹)- Gruppen mit B¹ in der Bedeutung von A¹, eine NHCO-Gruppe, eine CONH-Gruppe, eine N(B¹)-SO₂- Gruppe, oder eine -SO₂-N(B¹)- Gruppe und/oder gegebenenfalls substituiert ist mit dem Rest R^{F},
bedeutet und
- R^{F}: einen geradkettigen oder verzweigten perfluorierten Alkylrest der Formel CₙF₂ₙE,
wobei n für die Zahlen 4-30 steht und
E für ein endständiges Fluoratom, Chloratom, Bromatom, lodatom oder ein Wasserstoffatom steht, bedeutet,
und gegebenenfalls vorhandene Säuregruppen gegebenenfalls als Salze organischer und/oder anorganischer Basen oder Aminosäuren oder Aminosäureamide vorliegen können, wie sie und ihre Herstellung in der WO 02/13874 offenbart und definiert sind, eingesetzt werden.

Erfindungsgemäß werden ganz besonders bevorzugt die Metallkomplexe MK 17, MK 18 , MK 19, MK 21 und MK 23 (vgl. Tabelle 1) eingesetzt.

Diese Verbindungen der allgemeinen Formel Ib sind als MRI-Kontrastmittel zur Darstellung von Thromben sehr gut geeignet.

In einer anderen bevorzugten Ausführungsform der Erfindung können die perfluoralkylhaltigen Komplexe mit Zuckerresten der allgemeinen Formel Ic (siehe WO 02/13874) 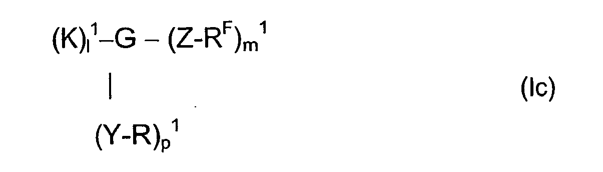 in der
- R: einen über die 1-OH- oder 1-SH-Position gebundenen Mono- oder Oligosaccharidrest darstellt,
- R^{F}: eine perfluorierte, geradkettige oder verzweigte Kohlenstoffkette mit der Formel -CₙF₂ₙE ist, in der E ein endständiges Fluor-, Chlor-, Brom-, Jod- oder Wasserstoffatom darstellt und n für die Zahlen 4-30 steht,
- K: für einen Metallkomplex der allgemeinen Formel IIc steht, 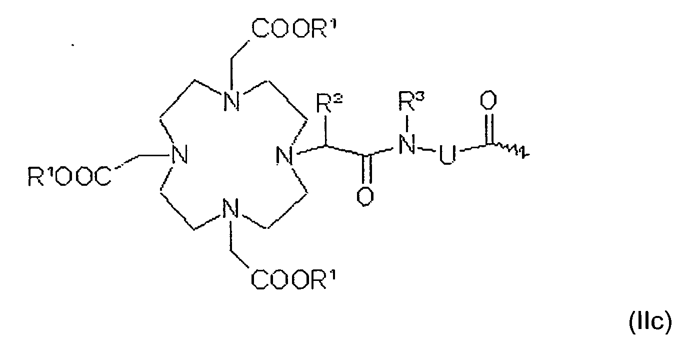 in der
- R¹: ein Wasserstoffatom oder ein Metallionenäquivalent der Ordnungszahlen 23-29, 42-46 oder 58-70 bedeutet,
mit der Maßgabe, dass mindestens zwei R¹ für Metall-ionenäquivalente stehen
- R² und R³: unabhängig voneinander Wasserstoff, C₁-C₇-Alkyl, Benzyl, Phenyl,-CH₂OH oder -CH₂OCH₃ darstellen und
- U: -C₆H₄-O-CH₂-ω-, -(CH₂)₁₋₅-ω, eine Phenylengruppe, -CH₂-NHCO-CH₂-CH(CH₂COOH)-C₆H₄-ω-, -C₆H₄-(OCH₂CH₂)₀₋₁-N(CH₂COOH)-CH₂-ω oder eine gegebenenfalls durch ein oder mehrere Sauerstoffatome, 1 bis 3-NHCO-, 1 bis 3 -CONH-gruppen unterbrochene und/oder mit 1 bis 3-(CH₂)₀₋₅COOH-Gruppen substituierte C₁-C₁₂-Alkylen- oder C₇-C₁₂-C₆H₄-O-Gruppe darstellt, wobei ω für die Bindungsstelle an -CO-steht,
oder
der allgemeinen Formel IIIc 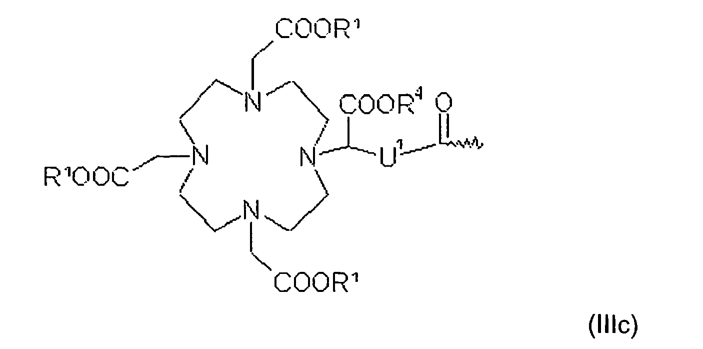 in der R¹ die oben genannte Bedeutung hat, R⁴ Wasserstoff oder ein unter R¹ genanntes Metallionenäquivalent darstellt und U¹ -C₆H₄-O-CH₂-ω- darstellt, wobei ω die Bindungsstelle an -CO- bedeutet
oder der allgemeinen Formel IVc 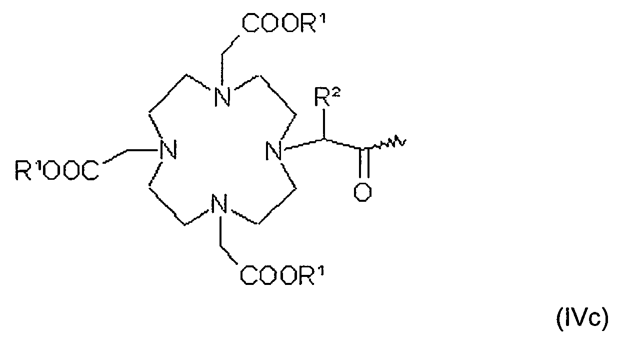 in der R¹ und R² die oben genannte Bedeutung haben
oder der allgemeinen Formel VcA oder VcB 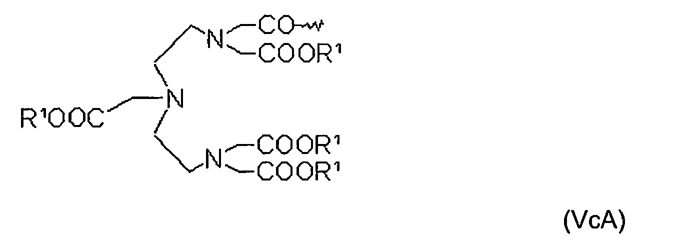 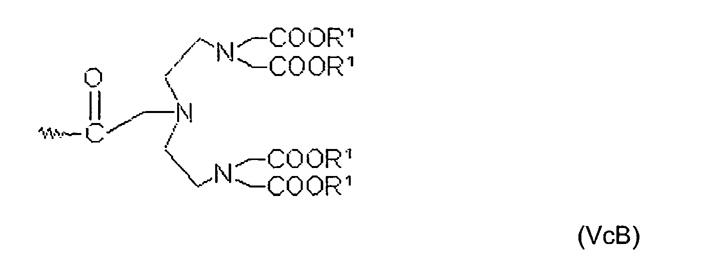 in der R¹ die oben genannte Bedeutung hat,
oder der allgemeinen Formel Vlc 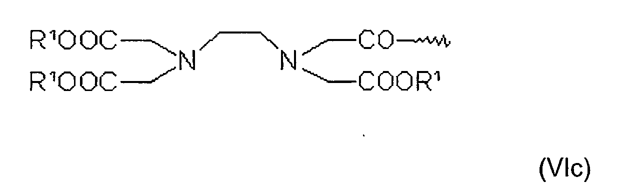 in der R¹ die oben genannte Bedeutung hat,
oder der allgemeinen Formel Vllc 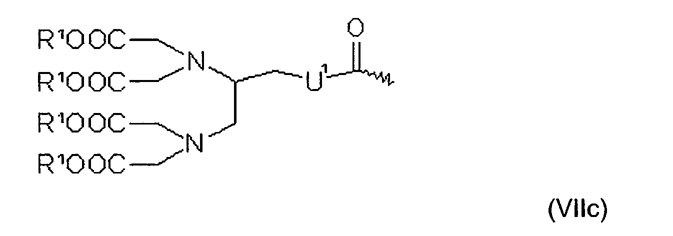 in der R¹ die oben genannte Bedeutung hat und
U¹ -C₆H₄-O-CH₂-ω- darstellt, wobei ω die Bindungsstelle an -CO- bedeutet
oder der allgemeinen Formel VIIIc 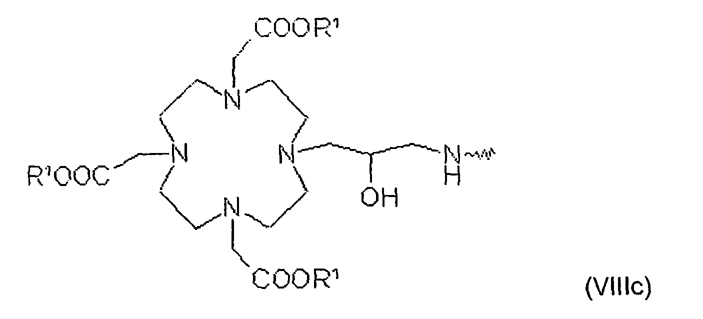 in der R¹ die oben genannte Bedeutung hat,
und im Rest K gegebenenfalls vorhandene freie Säuregruppen gegebenenfalls als Salze organischer und/oder anorganischer Basen oder Aminosäuren oder
Aminosäureamide vorliegen können,
- G: für den Fall, dass K die Metallkomplexe IIc bis VIIc bedeutet, einen mindestens dreifach funktionalisierten Rest ausgewählt aus den nachfolgenden Resten a) bis j) darstellt 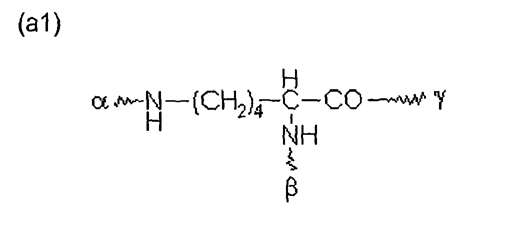 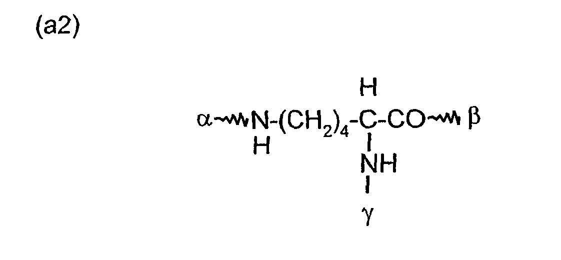 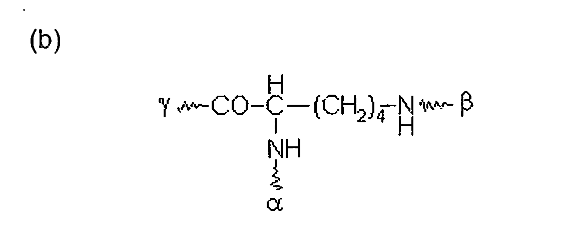 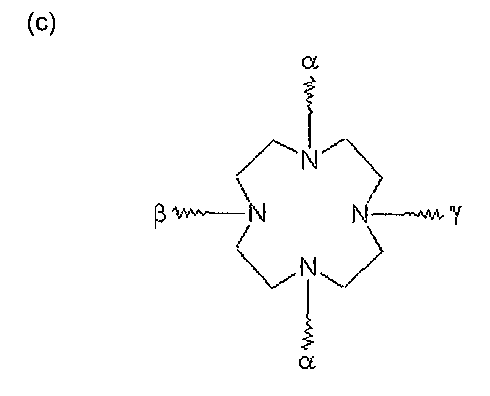 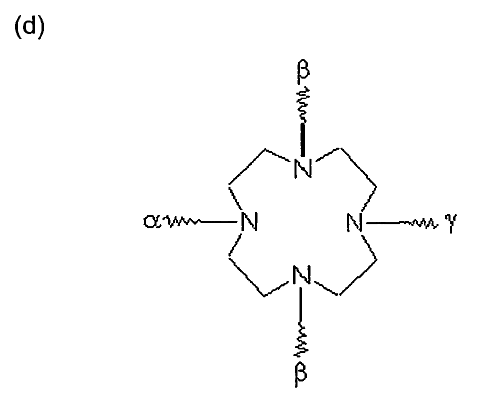 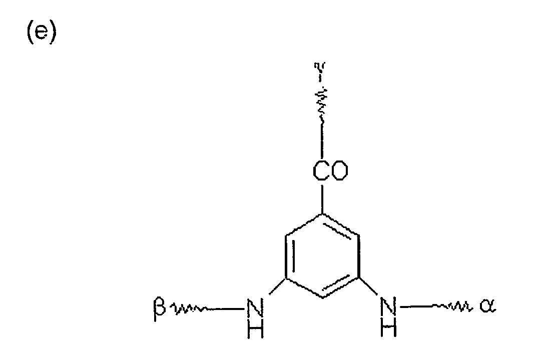 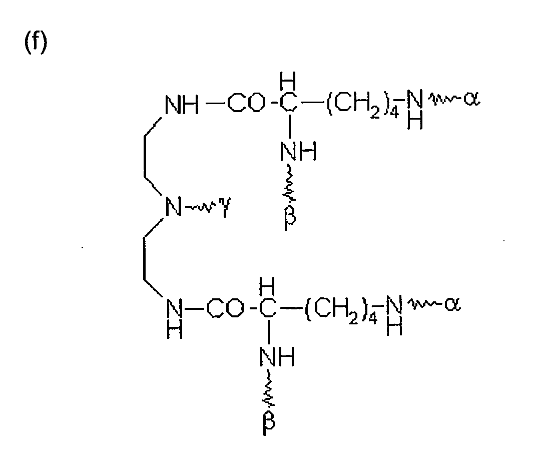 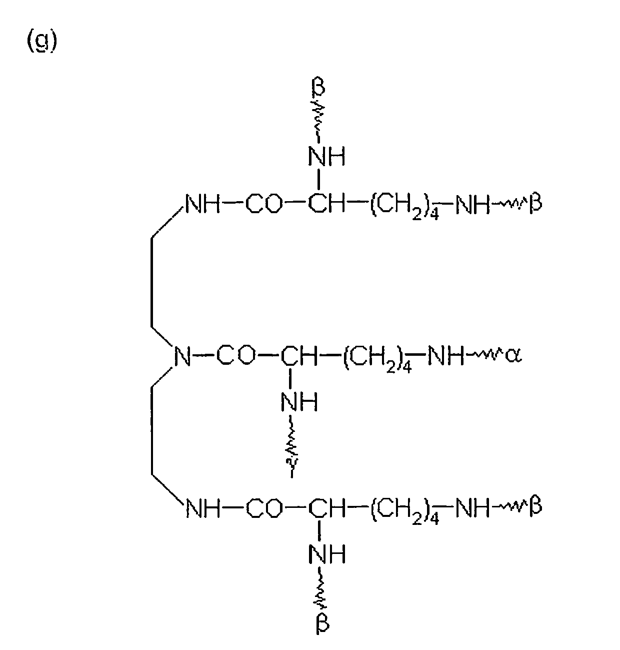 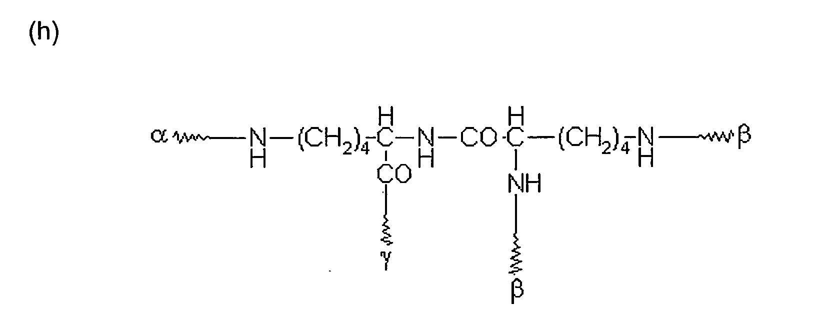 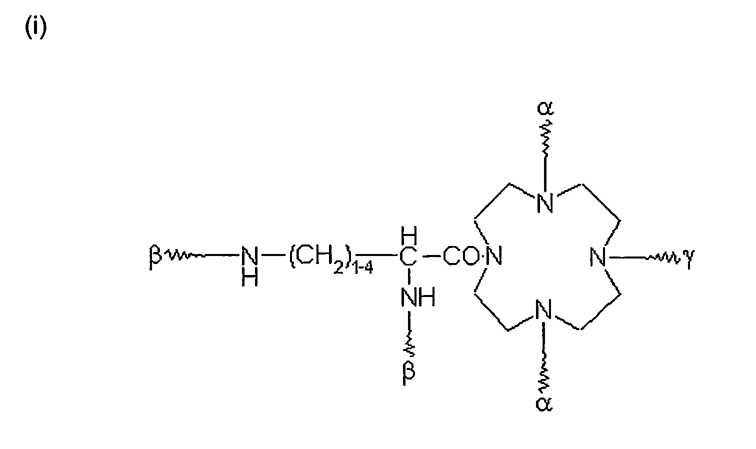 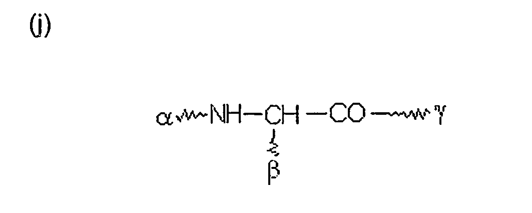 und
- G: für den Fall, dass K den Metallkomplex Vlllc bedeutet, einen mindestens dreifach funktionalisierten Rest ausgewählt aus k) oder l) darstellt, 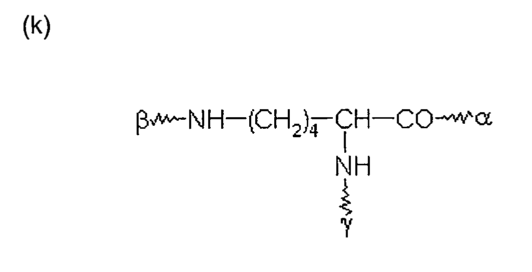 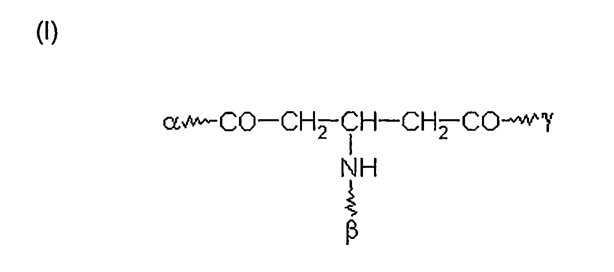
wobei α die Bindungsstelle von G an den Komplex K bedeutet, β die Bindungsstelle von G zum Rest Y ist und γ die Bindungsstelle von G zum Rest Z darstellt
- Y: -CH₂-, δ-(CH₂)₁₋₅CO-β, β-(CH₂)₁₋₅CO-δ, δ-CH₂-CHOH-CO-β oder δ-CH(CHOH-CH₂OH)-CHOH-CHOH-CO-β bedeutet, wobei δ die Bindungsstelle zum Zuckerrest R darstellt und β die Bindungsstelle zum Rest G ist
- Z: für 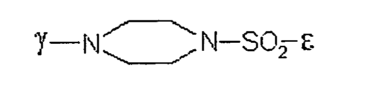 γ -COCH₂-N(C₂H₅)-SO₂-ε,
γ-COCH₂-O-(CH₂)₂-SO₂-ε, 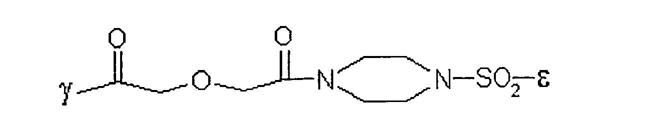 oder
γ-NHCH₂CH₂-O-CH₂CH₂-ε
steht, wobei γ die Bindungsstelle von Z zum Rest G darstellt und ε die Bindungsstelle von Z an den perfluorierten Rest R^{F} bedeutet
und
- l¹, m¹: unabhängig voneinander die ganzen Zahlen 1 oder 2 bedeuten und
- p¹: die ganzen Zahlen 1 bis 4 bedeutet,
eingesetzt werden.

Als ganz besonders bevorzugte Verbindungen der allgemeinen Formel Ic wird erfindungsgemäß der Metallkomplex MK 13 der Tab. 1 eingesetzt.

In einer weiteren bevorzugten Ausführungsform der Erfindung finden die perfluoralkylhaltigen Komplexe mit polaren Resten der allgemeinen Formel Id (siehe WO 02/13874) Anwendung 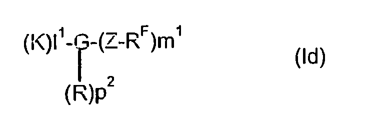 in der
- R^{F}: eine perfluorierte, geradkettige oder verzweigte Kohlenstoffkette mit der Formel -CₙF₂ₙE ist, in der E ein endständiges Fluor-, Chlor-, Brom-, Jod-oder Wasserstoffatom darstellt und n für die Zahlen 4-30 steht,
- K: für einen Metallkomplex der allgemeinen Formel IId steht, 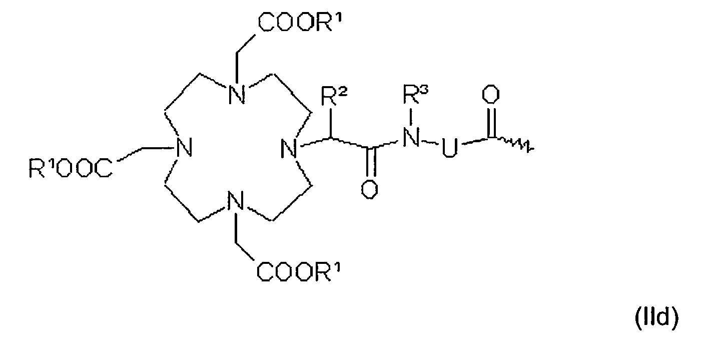
in der
- R¹: ein Wasserstoffatom oder ein Metallionenäquivalent der Ordnungszahlen 23-29, 42-46 oder 58-70 bedeutet,
mit der Maßgabe, dass mindestens zwei R¹ für Metall-ionenäquivalente stehen,
- R² und R³: unabhängig voneinander Wasserstoff, C₁-C₇-Alkyl, Benzyl, Phenyl,-CH₂OH oder -CH₂OCH₃ darstellen und
- U: -C₆H₄-O-CH₂-ω-, -(CH₂)₁₋₅-ω, eine Phenylengruppe, -CH₂-NHCO-CH₂-CH(CH₂COOH)-C₆H₄-ω-, -C₆H₄-(OCH₂CH₂)₀₋₁-N(CH₂COOH)-CH₂-ω oder eine gegebenenfalls durch ein oder mehrere Sauerstoffatome, 1 bis 3-NHCO-, 1 bis 3 -CONH-gruppen unterbrochene und/oder mit 1 bis 3 -(CH₂)₀₋₅COOH-Gruppen substituierte C₁-C₁₂-Alkylen- oder C₇-C₁₂-C₆H₄-0-Gruppe darstellt, wobei ω für die Bindungsstelle an -COsteht,
oder
der allgemeinen Formel IIId 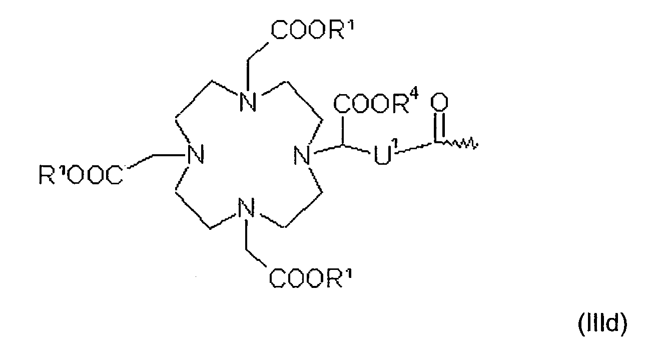 in der R¹ die oben genannte Bedeutung hat, R⁴ Wasserstoff oder ein unter R¹ genanntes Metallionenäquivalent darstellt und U¹ -C₆H₄-O-CH₂-ω- darstellt, wobei ω die Bindungsstelle an -CO- bedeutet
oder der allgemeinen Formel IVd 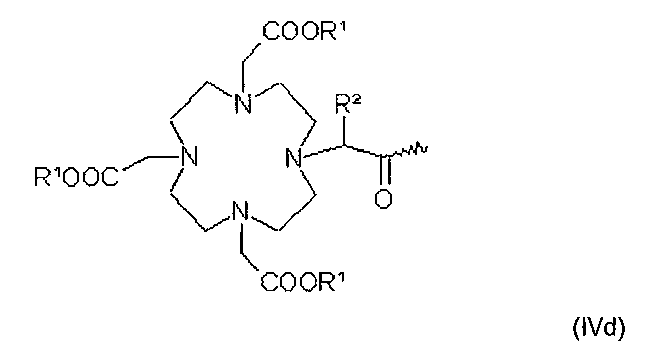 in der R¹ und R² die oben genannte Bedeutung haben
oder der allgemeinen Formel VdA oder VdB 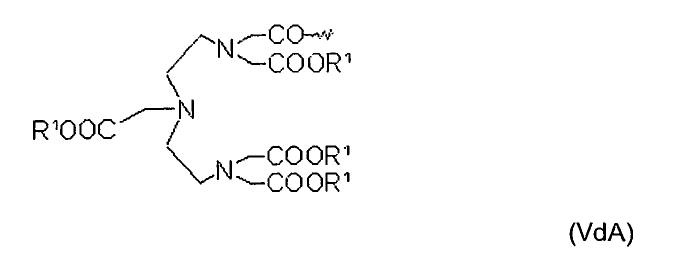 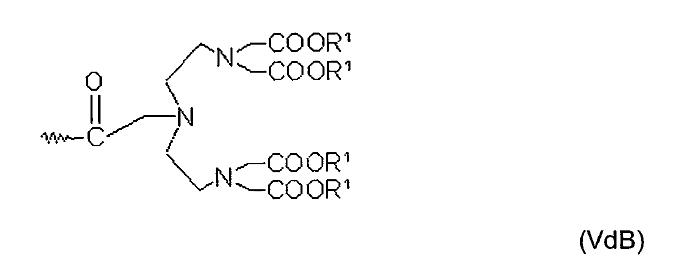 in der R¹ die oben genannte Bedeutung hat,
oder der allgemeinen Formel Vld 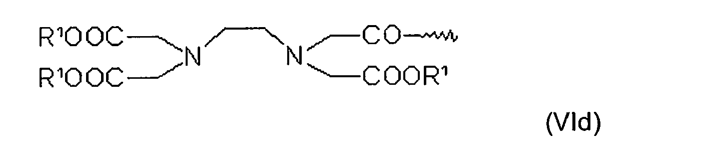 in der R¹ die oben genannte Bedeutung hat,
oder der allgemeinen Formel VIId 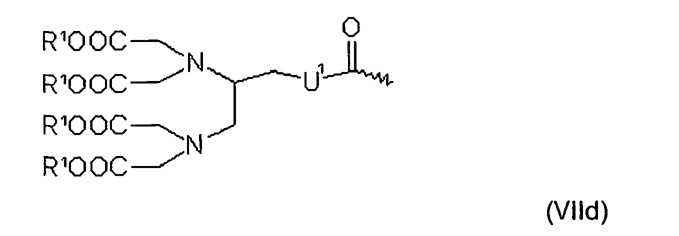 in der R¹ die oben genannte Bedeutung hat und
U¹ -C₆H₄-O-CH₂-ω- darstellt, wobei ω die Bindungsstelle an -CO- bedeutet
und im Rest K gegebenenfalls vorhandene freie Säuregruppen gegebenenfalls als Salze organischer und/oder anorganischer Basen oder Aminosäuren oder Aminosäureamide vorliegen können,
- G: einen mindestens dreifach funktionalisierten Rest ausgewählt aus den nachfolgenden Resten a) bis i) darstellt 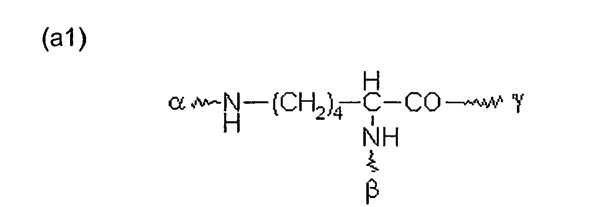 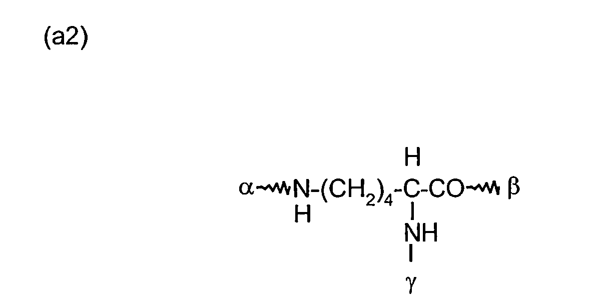 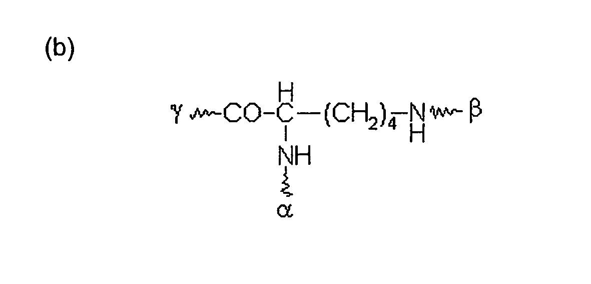 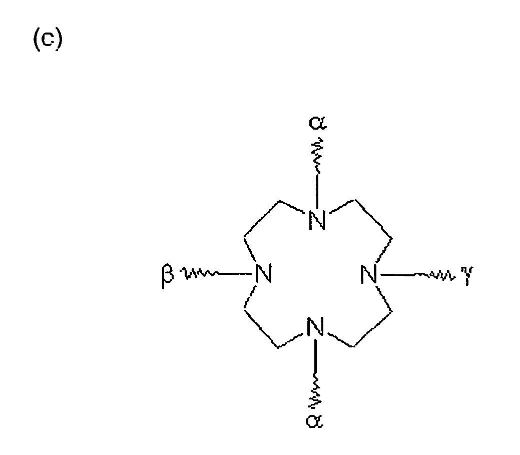 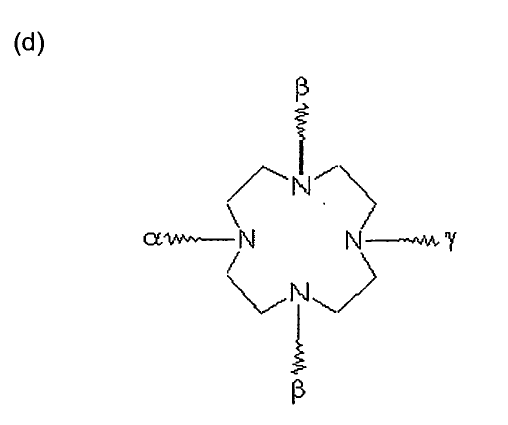 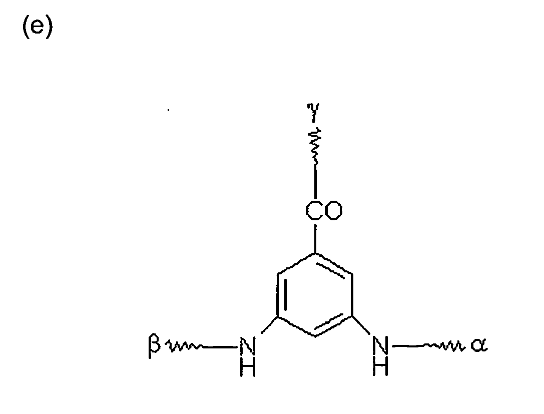 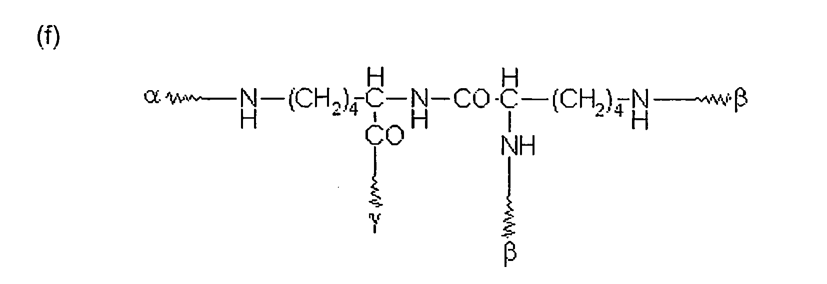 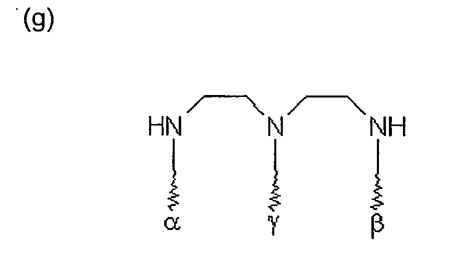 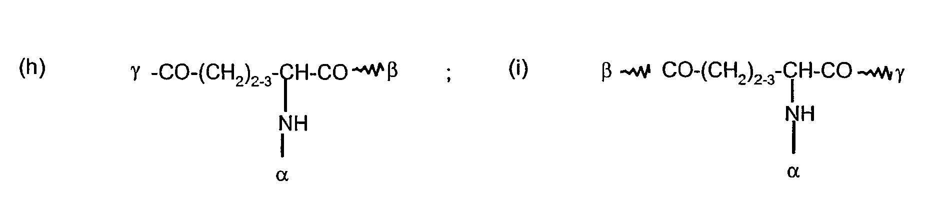 wobei α die Bindungsstelle von G an den Komplex K bedeutet, β die Bindungsstelle von G zum Rest R ist und γ die Bindungsstelle von G zum Rest Z darstellt
- Z: für 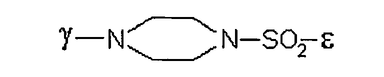 γ -C(O)CH₂O(CH₂)₂-ε, steht, wobei γ die Bindungsstelle von Z zum Rest G darstellt und ε die Bindungsstelle von Z an den perfluorierten Rest R^{F} bedeutet
- R: einen polaren Rest ausgewählt aus den Komplexen K der allgemeinen Formeln IId bis VIId darstellt, wobei R¹ hier ein Wasserstoffatom oder ein Metallionenäquivalent der Ordnungszahlen 20, 23-29, 42-46 oder 58-70 bedeutet,
und die Reste R², R³, R⁴, U und U¹ die oben angegebene Bedeutung aufweisen
oder
den Folsäurerest
oder
eine über -CO- , SO₂- oder eine direkte Bindung an den Rest G gebundene Kohlenstoffkette mit 2-30 C-Atomen bedeutet, geradlinig oder verzweigt, gesättigt oder ungesättigt,
gegebenenfalls unterbrochen durch 1-10 Sauerstoffatome, 1-5 - NHCO-Gruppen, 1-5 -CONH-Gruppen, 1-2 Schwefelatome, 1-5 -NH-Gruppen oder 1-2 Phenylengruppen, die gegebenenfalls mit 1-2 OH-Gruppen, 1-2 NH₂-Gruppen, 1-2 -COOH-Gruppen, oder 1-2 -SO₃H-Gruppen substituiert sein können
oder
gegebenenfalls substituiert mit 1-8 OH-Gruppen, 1-5 -COOH-Gruppen, 1-2 SO₃H-Gruppen, 1-5 NH₂-Gruppen, 1-5 C₁-C₄-Alkoxygruppen,
und
- l¹, m¹, p²: unabhängig voneinander die ganzen Zahlen 1 oder 2 bedeuten.

Besonders bevorzugte Verbindungender allgemeinen Formel Id sind solche mit dem Makrocyclus K der allgemeinen Formel IId, IIId, VdB oder VIId.

Als ganz besonders bevorzugte Verbindung der allgemeinen Formel Id wird erfindungsgemäß der Metallkomplex MK12 der Tab. 1 eingesetzt.

In einer anderen bevorzugten Ausführungsform der Erfindung können galenische Formulierungen eingesetzt werden, die paramagnetische und diamagnetische perfluoralkylhaltige Substanzen enthalten. Vorzugsweise liegen die paramagnetischen und diamagnetischen Substanzen in einem wässrigen Lösungsmittel gelöst vor.

Als paramagnetische perfluoralkylhaltige Verbindungen können in den Formulierungen erfindungsgemäß alle vorstehend genannten Metallkomplexe der allgemeinen Formeln I, Ia, Ib, Ic und/oder Id eingesetzt werden.

Die diamagnetischen perfluoralkylhaltigen Substanzen sind solche der allgemeinen Formel XX (siehe WO 02/13874):

R^{F}-L²-B² (XX)

worin R^{F} einen geradkettigen oder verzweigten Perfluoralkylrest mit 4 bis 30 Kohlenstoffatomen darstellt, L² für einen Linker und B² für eine hydrophile Gruppe steht. Der Linker L² ist eine direkte Bindung, eine -SO₂-Gruppe oder eine geradkettige oder verzweigte Kohlenstoffkette mit bis zu 20 Kohlenstoffatomen, welche mit ein oder mehreren -OH, -COO⁻, -SO₃-Gruppen substituiert sein kann und/oder gegebenenfalls ein oder mehrere -O-, -S-, -CO-, -CONH-, -NHCO-, - CONR⁹-, -NR⁹CO-, -SO₂-, -PO₄⁻-, -NH-, -NR⁹-Gruppen, einen Arylring oder ein Piperazin enthält, wobei R⁹ für einen C₁- bis C₂₀-Alkylrest steht, welcher wiederum ein oder mehrere O-Atome enthalten kann und/oder mit -COO⁻ oder SO₃-Gruppen substituiert sein kann.

Weitere geeignete diamagnetische perfluoralkylhaltige Verbindungen sind Konjugate aus Cyclodextrin und perfluoralkylhaltigen Verbindungen. Diese Konjugate bestehen aus α-,β - oder γ- Cyclodextrin und Verbindungen der Allgemeinen Formel XXII ( siehe WO 02/13874)

A¹-L³-R^{F} (XXII)

worin A¹ für ein Adamantan-, Biphenyl- oder Anthracenmolekül, L³ für einen Linker und R^{F} für einen geradkettigen oder verzweigten Perfluoralkylrest mit 4 Bis 30 Kohlenstoffatomen steht. Der Linker L³ ist eine geradkettige Kohlenwasserstoffkette mit 1 bis 20 Kohlenstoffatomen, welche durch ein oder mehrere Sauerstoffatome, ein oder mehrere CO-, SO2-, CONH-, NHCO-, CONR-, NRCO-, NH-, NR-Gruppen oder ein Piperazin unterbrochen sein kann, wobei R ein C₁-C₅-Alkylrest ist.

Zusammenfassend ist festzustellen, dass als ganz besonders bevorzugte Verbindungen die in Tabelle 1 aufgeführten Gadoliniumkomplexe MK 1-30 die erfindungsgemäßen Kriterien erfüllen. Die physikalischen Parameter dieser Metallkomplexe sind in Tabelle 1 aufgeführt.

Sowohl die erfindungsgemäßen paramagnetischen Verbindungen der allgemeinen Formeln I, Ia, Ib, Ic und Id als auch die erfindungsgemäßen Formulierungen aus paramagnetischen und diamagnetischen perfluoralkylhaltigen Substanzen eignen sich in hervorragender Weise als Kontrastmittel im MR-Imaging zur Darstellung von Thromben.

**Tabelle 1:**

| **Erfindungsgemäß ganz besonders bevorzugt verwendete Metallkomplexe (MK), ihre Herkunft und ihre physikochemischen Parameter** | | | | | |
|---|---|---|---|---|---|
| Komplex | Herkunft | Beispiel Nr. | R¹ [1 : mmol·s] | CMC[mol/l] | 2 Rh [nm] |
| MK 1 | WO 99/01161 | 18 | 23,0 | 1,5 10⁻⁴ | 3,5 |
| MK 2 | WO 97/26017 | 1 | 29,7 | 1,0 10⁻⁵ | 31,5 |
| MK 3 | WO 97/26017 | 2 | 33,0 | 2,3 10⁻⁵ | 14,0 |
| MK 4 | WO 97/26017 | 3 | 27,5 | 1,44 10⁻⁵ | 3,2 |
| MK 5 | WO 99/01161 | 25 | 15,1 | 3,1 10⁻⁵ | 7,0 |
| MK 6 | WO 97/26017 | 31 | 26,0 | 9,8 10⁻⁴ | 4,3 |
| MK 7 | WO 99/01161 | 12 | 21,4 | 1,81 10⁻⁶ | 4,2 |
| MK 8 | WO 97/26017 | 33 | 35,7 | 1,86·10⁻⁶ | 4,6 |
| MK 9 | WO 97/26017 | 35 | 34,0 | 3,25 ·10⁻⁶ | 4,3 |
| MK 10 | WO 97/26017 | 34 | 24,9 | 7,06·10⁻⁶ | 3,2 |
| MK 11 | WO 97/26017 | 32 | 24,8 | 2,88·10⁻⁶ | 35,5 |
| MK 12 | WO 99/01161 | 1 | 19,5 | 8,9·10⁻⁴ | 2,2 |
| MK 13 | WO 02/13874 | 21 | 15,9 | 2,5·10⁻⁶ | 4,4 |
| MK 14 | WO 02/13874 | 54 | 21,3 | 3,9 10⁻⁵ | 4,9 |
| MK 15 | WO 99/01161 | 14 | 19,3 | 8,7·10⁻⁶ | 3,2 |
| MK 16 | WO 00/56723 | 7 | 21,0 | 2,8·10⁻⁶ | 4,3 |
| MK 17 | WO 02/13874 | 6 | 13,3 | 2,65·10⁻⁶ | 6,0 |
| MK 18 | WO 02/13874 | 2 | 19,6 | 3,9 ·10⁻⁶ | 4,4 |
| MK 19 | WO 02/13874 | 5 | 30,3 | 5,2 ·10⁻⁵ | 3,0 |
| MK 20 | WO 00/56723 | 4 | 21,9 | 4,6 10⁻⁵ | 5,5 |
| MK 21 . | WO 02/13874 | 3 | 21,2 | 2,92 10⁻⁵ | 2,5 |
| MK 22 | WO 00/56723 | 7 | 27,8 | 4,4 ·10⁻⁶ | 5,7 |
| MK 23 | WO 02/13874 | 1 | 25,7 | 7,9 ·10⁻⁶ | 5,4 |
| MK 24 | WO 99/01161 | 1 | 13,9 | 6,3 ·10⁻⁶ | 10,0 |
| MK 25 | WO 99/01161 | 5 | 21,3 | 1,4 10⁻⁴ | 3,5 |
| MK 26 | WO 02/13874 | 57 | 22,8 | 4,3 ·10⁻⁶ | 5,2 |
| MK 27 | WO 97/25017 | 38 | 30,5 | 1,07 ·10⁻⁵ | 7,4 |
| MK 28 | diese Anm. | 1 | 27,9 | 8,1 ·10⁻⁶ | 4,7 |
| MK 29 | diese Anm. | 2 | 17,7 | 7,6 10⁻⁵ | 4,8 |
| MK 30 | diese Anm. | 3 | 27,9 | 7,0·10⁻⁶ | 7,9 |

| | | | | | |
|---|---|---|---|---|---|
| CMC: kritische Mizellbildungskonzentration 2 Rh: hydrodynamischer Mizelldurchmesser R¹: Relaxivity | | | | | |

Die Messungen wurden in Plasma bei 40 °C und einer Feldstärke von 0,47 Tesla durchgeführt.

### Beispiel 1

### a) 6-Benzyloxycarbonyl-2-N- 2H,2H, 4H,4H, 5H,5H-3-oxa-perfluortridecanoyl-L-lysinmethylester

Zu der Lösung von 50g (95,8 mmol) 2H,2H,4H,4H,5H,5H-3-Oxaperfluor - tridecan-säure (hergestellt aus 2H,2H,3H,3H-Perfluordecanol und Bromesssig - säure-t-butylester mit anschließender Esterspaltung) in 250 ml Thionylchlorid werden 2 Tropfen Dimethyl-formamid gegeben und man erwärmt 5 Stunden am Rückfluß.Dann engt man im Vakuum ein, nimmt den Rückstand in 250 ml Dichlormethan auf und tropft die Lösung bei 0° C unter Rühren zur Lösung von 34,74 g (105.0 mmol) 6-N-Benzyloxycarbonyl-L-Lysin -methylester, Hydrochlorid (Kaufware , Bachem) sowie 46,85 ml (350 mmol) Triethylamin in 400 ml Dichlormethan. Man läßt über Nacht rühren, versetzt mit 1 Liter 2N Salzsäure, schüttelt die organische Phase aus, extrahiert die Wasserphase zweimal mit je 100 ml Dichlormethan, trocknet die Lösung über Natriumsulfat, filtriert vom Trockenmittel ab und engt im Vakuum ein. Das Rohprodukt wird duch Chromatographie an Kieselgel gereinigt. Als Elutionsmittel dient ein Gemisch aus Dichlormethan mit 3 % Ethanolzusatz. Das Produkt wird nach dem Einengen als farbloses Gel erhalten.
Ausbeute: 67,0 g (87,6 % d. Th)

| Elementaranalyse | | | | |
|---|---|---|---|---|
| Ber.: | 40,61 C | 3,41 H | 40,45 F | 3,51 N |
| Gef.: | 40,48 C | 3,54 H | 40,61 F | 3,37 N. |

### b) 2-N-2H,2H,4H,4H,5H,5,H-3-Oxaperfluortridecanoyl-L-lysinmethylester, Hydrochlorid

Zu einer Lösung von 63,5 g (79,5 mmol) der Titelsubstanz aus Beispiel 1a) in einem Gemisch aus 500 ml Methanol und 90 ml 1 N Salzsäure gibt man 10 g Katalysator ( Pd 10 % /C) und hydriert bis zur Aufnahmne eines Equivalentes Wasserstoff bei Normaldruck und Raumtemperatur. Man filtriert vom Katalysator ab, wäscht diesen 3 mal mit je 50 ml heißem Methanol und engt die vereinigten Lösungen ein. Der Rückstand wird in Methanol gelöst und durch Zusatz von Diisopropylether zu Kristallisation gebracht.
Die Titelverbindung wird in farblosen Kristallen erhalten.
Ausbeute:: 55,70 g (quantitativ.)

| Elementaranalyse: | | | | | |
|---|---|---|---|---|---|
| Ber.: | 32,56 C | 3,16 H | 5.06 Cl | 46.09 F | 4,00 N |
| Gef.: | 32,44 C | 3,28 H | 4,95 Cl. | 46,21 F | 4.11 N |

### c) 6-N-3,6,9.12.15-Pentaoxa-hexadeoanoyl-2-N-2H,2H,4H,5H,5H-3-Oxaperfluortridecanoyl-I-lysin-methylester

13,31 g (50 ,0 mmol) 3.6.9.12.15-Pentaoxahexadecansäure (Kaufware) werden in 100 ml Thionylchlorid gelöst, mit zwei Tropfen Dimethylformamid versetzt und über Nacht bei Raumtemperatur gerührt. Dann erwärmt man eine Stunde auf 65 °C, ent-fernt überschüssiges Thionylchlorid am Rotations - verdampfer und nimmt den Rückstand in 150 ml Dichlormethan auf. Man tropft diese Lösung bei 0° C zu der Lösung von 35,04 g (50,0 mmol) der Titel - verbindung aus Beispiel 1b) und 15,18 g (150 mmol) Triethylamin in 350 ml Dichlormethan. Dann läßt man 72 Stunden bei Raumtemperatur rühren. Man engt ein und gewinnt das Produkt durch Säulenchromatographie an Kieselgel. Als Elutionsmittel dient ein Gemisch aus Dichlormethan/Ethanol 9:1. Die Titelverbindung wird als zähes, leicht gelbes Öl erhalten..
Ausbeute: 37,0 g (81,1% d. Th)

| Elementaranalyse: | | | | |
|---|---|---|---|---|
| Ber.: | 39,48 C | 4,53 H | 35,39 F | 3,07 N |
| Ber.: | 39,61 C: | 4,50 H | 35,50 F | 3,16 N |

### d) 6-N-3.6.9.12.15-Pentaoxahexadecanoyl-2-N-2H,2H,4H,4H.5H,5H-3-Oxa-perfluortridecanoyl-L-lysin

17,90 g der Titelverbindung aus Beispiel 1c) werden uber Nacht in einer Mischung aus 50 ml Methanol und 25 ml 2N Natronlauge gerührt. Man säuert mit 2 N Salzsäure an, engt im Vakuum ein und extrahiertr den Rückstand 5 mal mit je 50 ml Tetrahydrofuran/Essigester 2:1. Die vereinigten Extrakte werden über Natriumsulfat getrocknet . Man filtriert vom Trockenmittel ab und engt die Lösung ein. Der Rückstand wird durch Säulenchromatographie an Kieselgel gereinigt.Als Laufmittel dient ein Gemisch aus Dichlormethan/Methanol und Wasser im Verhältnis 160:40:1 , Die Titelverbindung wird als wachsartiger, leicht gelb gefärbter Rückstand erhalten.
Ausbeute: 14,7 g (83,4 % d. Th.)

| Elementaranalyse. | | | | |
|---|---|---|---|---|
| Ber.: | 38,76 C | 4,37 H | 35,94 F | 3,12 N |
| Gef.: | 38,87 C | 4,25 H | 36 07 F | 3,21 N |

### e) 6-N-3.6.9.12.15-Pentaoxahexadecanoyl-2-N-2H,2H,4H,4H,5H,5H-3-oxaperfluortridecanoyl-L-lysin-N- {1,4,7-tris[carboxylatomethyl]-1,4,7,10-tetraazacyclododecan-10-(2-hydroxy-3-yl), Gadoliniumkomplex}-amid

In 50 ml Dimethylformamid werden 8,0 g (8,9 mmol) der unter 1d) herge-stellten Säure sowie 2,05 g (17,8 mmol) Hydroxysuccinimid gelöst und bei 0° C mit 4,60 g (22,25 mmol) Dicyclohexylcarbodiimid versetzt. Man rührt noch 10 Minuten bei 0° C nach und dann noch weitere 2 Stunden bei Raumtemperatur. Nach erneutem Abkühlen auf 0° C wird eine Lösung aus 3,93 g (6,65 mmol) Gadolinium-Komplex von 10-(3-Amino-2-hydroxy-propyl)-1,4,7-tris-(carboxymethyl)-1,4,7,10-tetraazacyclododecan (WO 95/17451) sowie 0,58 g (13,7 mmol) Lithiumchlorid und 2,77 g (27,4 mmol) Triethylamin in 40 ml Dimethylsulfoxid zugegeben. Man läßt zwei Tage bei Raumtemperatur rühren, versetzt mit 650 ml Aceton und gießt die Suspension qauf 2 l Methyl-t-buthylether. Man rührt etwa 30 Minuten nach und saugt dann vom Feststoff ab. Der Feststoff wird in destilliertem Wasser gelöst und mit Aktivkohle behandelt. Die Lösung wird filtriert, im Vakuum eingeengt, und der Rückstand wird an Kieselgel chromatographiert. Als Elutionsmittel dient ein Gemisch aus Methanol und Dichlormethan im Verhältnis2.1
Ausbeute 5,47 g (54,5 % d. Th)
Wassergehalt: 7,3 %

| Elementaranalyse (bezogen auf wasserfreie Substanz): | | | | | |
|---|---|---|---|---|---|
| Ber.: | 37,97 C | 4,71 H | 10,81 Gd | 22,19 F | 6,74 N |
| Gef.: | 38,16 | 4,83 | 10,72 | 22,32 | 6,83 |

### Beipiel 2

### a) 10-(3-Carboxy-3-yl-propionsäure)-1,4,7,10-tetraazacyclododecan

150 g (761 mmol) Brombernsteinsäure werden mit Natronlauge (10 %) neutralisiert und die Lösung wird zur Trockne eingeengt. In 300 ml destilliertem Wasser werden 65,55 g (380 mmol) Cyclen gelöst und mit dem Binatriumsalz (aus 150 g Brombernsteinsäure= 761 mmol) versetzt. Man erwärmt auf 50 °C und läßt über Nacht rühren. Die Lösung wird dann zur Trockne eingeengt und mit Ethanol kodestilliert. Der Rückstand wird in Butanol aufgenommen und mit Wasser extrahiert. Die wäßrige Phase wird eingeengt und an Kieselgel chromatographiert. Als Elutionsmittel dienen Gemische aus Methanol mit Ammoniak (20:1 - 2:1). Die produkthaltigen Fraktionen werden vereinigt und zur
Trockne eingeengt.
Ausbeute: 54,8 g (50,4 % d. Th.)

| Elementaranalyse: | | | |
|---|---|---|---|
| ber.: | C 50,34 | H 7,74 | N 19,57 |
| gef. | C 50,46 | H 7,83 | N 19,69 |

### b) 1,4,7-Tris(carboxymethyl)-10-(3-carboxy-3-yl-propionsäure)-1,4,7,10-tetraazacyclododecan

In 60 ml destilliertem Wasser werden 11 g (38,14 mmol) 10-(3-Carboxy-3-yl-propionsäure)-1,4,7,10-tetraazacyclododecan gelöst und mit 18,03 g (190,74 mmol) Chloressigsäure versetzt. Man erwärmt dann auf 70 °C und hält den pH Wert durch Zugabe von Na-tronlauge (32 %) zwischen 9 und 10. Man läßt über Nacht bei 70 °C rühren, stellt dann erneut auf einen pH Wert von 10 ein und gibt 7,2 g (76,19 mmol) Chloressigsäure dazu. Es wird noch 3 Stunden bei 70 °C gerührt. Man engt zur Trockne ein, dampft mit Methanol ab, nimmt in Methanol auf und filtriert von den Salzen ab. Das Filtrat wird eingeengt und an einer lonenaustauschersäule Amberlite 252 C mit Wasser/Ammoniak als Elutionsmittel chromatographiert. Die produkthaltigen Fraktionen werden zusammengefaßt, eingeengt, erneut in destilliertem Wasser aufgenommen und gefriergetrocknet. Die Titelverbindung wird als weißer Schaum erhalten.
Ausbeute: 13,12 g (82,3 % d. Th.)
Wassergehalt: 9,6 %

| Elementaranalyse (bezogen auf wasserfreie Substanz): | | | |
|---|---|---|---|
| ber.: | C 46,75 | H 6,54 | N 12,12 |
| gef. | C 46,87 | H 6,62 | N 12,24 |

### c) Gadoliniumkomplex von 10-{1-carboxy-2-carbonyl-[piperazin-1-yl-4-(perfluoroctylsulfonyl)]}-ethyl-1,4,7-tris(carboxymethyl)-1,4,7,10-tetraazacyclododecan, Natriumsalz

In 100 ml Dimethylsulfoxid werden 10,0 g (16,21 mmol) Gadolinium-Komplex tetraazacyclododecan (hergestellt aus dem Liganden durch Komplexierung mit Gadoliniumoxid) und 3,0g Lithiumchlorid unter leichtem Erwärmen gelöst. Nach dem Abkühlen auf Raumtemperatur werden 9,21 g (16,21 mmol) Perfluoroctylsulfonylpiperazin zugegeben.. Dann wird auf 0° C gekühlt, und es werden 12,3 g (46,63 mmol) EEDQ (1,2-Dihydro-2-ethoxychinolin-1-carboncarbonsäure ethylester) zugegeben und es wird über Nacht bei Raumtemperatur gerührt.. Die Reaktion wird in eine Mischung aus 800 ml Methylt-butylether und 100 ml Aceton gegossen und gerührt. Der Niederschlag wird durch Chromatographie an Kieselgel gereinigt. Als Elutionsmittel dient eine Mischung aus Dichlormethan/Methanol und Ammoniak im Verhältnis 2: 2. 1.Die produkthaltigen Fraktionen werden vereinigt und eingeengt. Der Rückstand wirdin 200 ml destilliertem Wasser gelöst, mit Natronlauge auf einen pH Wert von 7,2 eingestellt und lyophilisiert. Die Titelverbindung wird als weißer Schaum erhalten.
Ausbeute: 7,64 g (39 % d. Th.)
Wassergehalt. 7,8 %

| Elementaranalyse (bezogen auf wasserfreie Substanz) | | | | | | | |
|---|---|---|---|---|---|---|---|
| Ber.: | 30,31 C | 2,80 H | 27,17 F | 13,23 Gd | 7,07 N | 1,93 Na | 2,70 S |
| Gef.: | 30,42 C | 2,91 H | 27,04 F | 13.29 Gd | 7,15 N | 2.04 Na | 2,59 S |

### Beispiel 3

### a)1,4,7-Tris(carboxylatomethyl)-1,4,7,10-tetraazacyclododecan-10-[2-hydroxy 3-(N-benzyloxycarbonyltriglycidyl)-)-amino]-propyl, Gadoliniumkomplex

In 100 ml Dimethylformamid werden 12,68 g (39,121 mmol) N-Benzyloxycarbonyltri-glycin (Kaufware, Bachem) gelöst und mit 9,03 g (78,42 mmol) N-Hydroxysuccinimidversetzt. Man kühlt auf 0° C ab und und gibt dann 32,36 g (156,84 mmol) Dicyclohexyl-carbodiimid dazu.Man rührt 20 Minuten bei 0° C und dann weitere 3 Stunden bei Raumtemperatur. Diese Suspension wird dann zu der auf auf 0° C gekühlten Lösung aus 15 g (26,14 mmol) Gadoliniumkom -plex aus 10-(3-Amino-2-hydroxy-propyl)-1,4,7,-tris(carboxymethyl)-1,4,7,10-tetraazacyclododecan (hergestellt nach WO 95/17451) in 40 ml destilliertem Wasser und 15 ml (14 mmol) Triethylamin in 60 ml Isopropanol unter Rühren gegeben. Nach beendeter Zugabe wird 3 Stunden bei Raumtemperatur gerührt.Dann filtriert man vom Harnstoff ab, wäscht mit n Butanol nach und engt im Vakuum ein.Der Rückstand wird mehrfach mit Wasser extrahiert. Die organische Phase wird über Natriumsulfat getrocknet und im Vakuum einge-engt. Der Rückstand wird an Kieselgel chromatographiert. Als Elutionsmittel dient ein Gemisch aus Dichlormethan, Methanol und Ammoniak.Die produkt-haltigen Fraktionen werden vereinigft, im Vakuum eingeengt, erneut in destilliertem Wasser aufgenommen und der Gefriertrocknung unterworfen. Die Titelverbindung wird als weißer Schaum erhalten.
Ausbeute: 12,94 g (56,3 % d. Th

| Elementaranalyse: | | | | |
|---|---|---|---|---|
| Ber.: | 42,36 C | 5,16 H | 17,89 Gd | 12,75 N |
| Gef.: | 42,44 C | 5,22 H | 17,78 Gd | 12,80 N |

### b) 1,4,7-Tris(carboxylatomethyl)-1,4,7,10-tetraazacyclododecan-10-(2-hydroxy-3-amino-triglycidyl)-propyl, Gadoliniumkomplex

In einem Gemisch aus 100 ml Ethanol und 30 ml destiliertem Wasser werden 8,53 g (9,7 mmol) der Titelverbindung aus Beispiel 3a) gelöst und mit 2 g Katalysator (Palladium 10% auf Aktivkohle) sowie 3 ml Essigsäure versetzt. Man hydriert bis zur Aufnahme eines Equivalents Wasserstoff. Dann saugt man vom Katalysator ab, wäscht mit Ethanol nach und engt die Lösung im Vakuum zur Trockene ein.
Die Titelverbindung wird als Schaum erhalten.
Ausbeute: 7,22 g (quantitativ)

| Elementaranalyse: | | | | |
|---|---|---|---|---|
| Ber.: | 37,08 C | 5,28 H | 21,11 Gd | 15,04 N |
| Gef.: | 37,21 C | 5,33 H | 21,25 Gd | 15,15 N |

### c) 1,4,7-Tris(carboxylatomethyl)-1,4,7,10-tetraazacyclododecan-10-{2-hydroxy-3-N-[triglycidyl-N-(2H,2H,4H,4H,5h,5H-3-oxa-perfluortridecanoyl)]amino}-propyl, Gadoliniumkomplex

In 90 ml Dimethylformamid werden 7,60 g (14,55 mmol) 2H,2H,4H,4H,5H,5H-3-oxa-perfluortridecansäure gelöst und mit 3,35 g (29,1 mmol) N-Hydroxsuccinimid versetzt. Man kühlt auf 0° C ab und versetzt dann mit 11,71 g (56,71 mmol) Dicyclohexylcarbo-diimid. Nach 20 Minuten entfernt man die Kühlung und rührt weitere 3 Stunden bei Raumtemperatur. Dann gibt man die entstandene Suspension unter Rühren zu der auf 0° C gekühlten Lösung von 7,22 g (9,7 mmol) der Titelverbindung aus Beispiel 3b) in einem Gemisch aus 5 ml (36,7 mmol) Triethylamin 20 ml destiliertem Wasser und 30 ml 2-Propanol.Man läßt über Nacht bei Raumtemperatur rühren, filtriert dann vom Dicyclohexylharnstoff ab, wäscht mit 2-Propanol /dest. Wasser 3:2 nach und engt die vereinigten Lösungen im Vakuum ein.Der Rückstand wird in einem Gemisch aus Wasser und Butanol gelöst und mit Butanol extrahiert. Die vereinigten organischen Lösungen werden getrocknet, und im Vakuum eingeengt.Der Rückstand wird durch Chromatographie an Kieselgel gereinigt. Als Elutionsmittel dient ein Gemisch aus
Ethanol/2-Propanol/ konz.
Ammoniak im Verhältnis 15 : 10 : 1. Die produkthaltigen Fraktionen werden vereinigt,
im Vakuum zur Trockene eingeengt , erneut in destiliertem Wasser gelöst und gefriergetrocknet. Die Titelverbindung wird als weißer Schaum erhalten.
Ausbeute: 7,52 g (62,1 % d. Th.)

| Elementaranalyse. | | | | | |
|---|---|---|---|---|---|
| Ber.: | 33.66 C | 3.55 H | 25,86 F | 12,59 Gd | 8,97 N |
| Gef.: | 33,55 C | 3,67 H | 25,99 F | 12,43 Gd | 9,09 N |

### Beispiel 4: Bindung an ein Fibrin-Gel

Durch Mischung von Fibrinogen mit Thrombin bildet sich nach 30-minütiger Inkubationszeit (Raumtemperatur) ein Koagulat (Fibrin-Gel). Dieses wird mit 0.5 ml PBS und mit 0.5 ml einer Lösung der Titelverbindung aus Beispiel 21, WO 02/13874, Mk 13, (0.01 und 0.1 mmol Gd/l) versetzt und über 16 Stunden bei Raumtemperatur inkubiert. Nach Entfernen des Überstandes auf dem Fibrin-Gel wird der ungebundene Anteil der erfindungsgemäßen Verbindung durch Ultrafiltration (1.200 g für 30 Minuten) vom Fibrin getrennt. Der Gadolinium-Gehalt im Fibrin-Gel wird mittels induktiv gekoppelter Plasma - Atom Emissions Spektroskopie (ICP-AES) ermittelt.
Die Bindung der erfindungsgemäßen Verbindung an das Fibrin-Gel betrug 79.1 % für die 0.01 mmol Gd/I-Lösung und 38.5 % für die 0.1 mmol Gd/I-Lösung.

### Beispiel 5: MRT-Darstellung (in-vivo) eines venösen Thrombus nach intravenöser Gabe des Kontrastmittels in Kaninchen

Die MR-Bildgebung erfolgte in Kaninchen mit photochemisch induziertem Thrombus (PIT). Durch Bestrahlung mit Xenon-Licht (540 nm, 1.100 klux, 25 min) nach i.v.-Injektion von Rose-Bengal (20 mg/kg) wurde in der linken Femoralvene die Thrombusbildung induziert. Der Blutfluss in der Femoralvene wurde mittels einer Ultraschallsonde kontrolliert. Das Imaging erfolgte mit einem Magnetom Harmony (Siemens, 1T) vor (baseline) sowie 25, 40 min, 1, 2, 3, 4, 24 und 48 Stunden nach intravenöser Applikation (ca. 1 h nach der Thrombus-Induktion) von 0.1 mmol Gd/kg der Titelverbindung aus Beispiel 21, WO 02/13874, unter Verwendung einer Phasenkontrast-Sequenz (TR/TE = 104/14ms) sowie von T1-gewichteten Gradientenecho-Sequenzen (MPRage: TR/TE/TI/α = 11/4/120ms/8°; und 3D-Flash: TR/TE/α = 5/2ms/50°).
Nach der Bildgebung wurde die linke Femoralvene (mit dem Thrombus) herauspräpariert, in Formalin fixiert, und zur histologischen Beurteilung mit Hämatoxylin / Eosin (HE) bzw. Phosphotungstic acid / Hämatoxylin (PTAH) gefärbt.

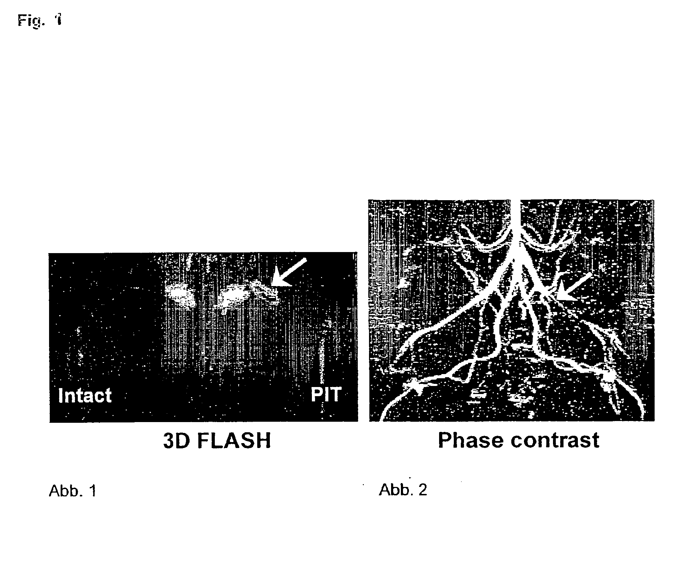

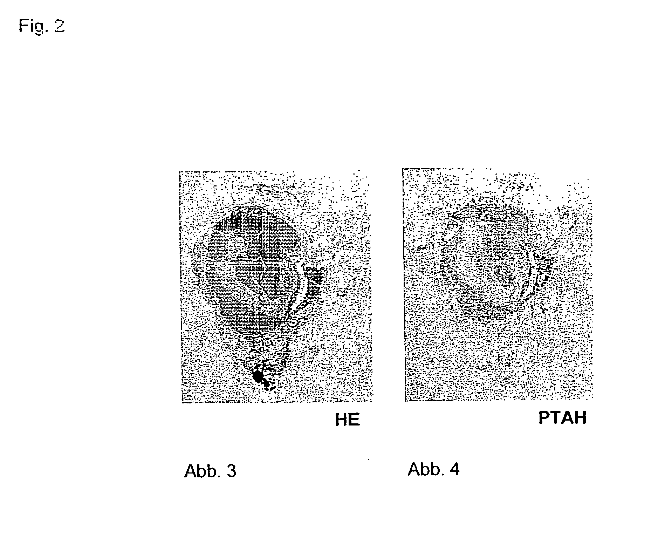

Im MR Imaging (MRA) war der Thrombus bereits frühzeitig (25 min p.i.) erkennbar. Die in Figur 1 angegebenen Abbildungen 1 und 2 zeigen MR-Aufnahmen der Beckenregion 24 h nach intravenöser Applikation von 0.1 mmol Gd/kg Körpergewicht der erfindungsgemäßen Verbindung im PIT-Kaninchen (photochemisch induzierter Thrombus). Die T₁-gewichtete 3D-Flash-Sequenz verdeutlicht einen starken Signalanstieg im Thrombus im Bereich der linken Femoralvene. Der Blutfluss in der linken Femoralvene ist deutlich reduziert (siehe MRI mit Phasenkontrast-Sequenz).

Mit beiden Färbetechniken (HE und PTAH (Fig. 2, Abb. 3 und 4)) konnten rote Blutgerinnsel (Thromben) im Bereich der linken Femoralvene nachgewiesen werden. Die Thromben füllen nahezu das gesamte Lumen des Blutgefäßes aus. Die Exfoliation der vaskulären Endothelzellen und die Adhäsion der Thromben ist deutlich sichtbar. Die Intima- und Adventitia-Kerne sind weitestgehend verschwunden.

Mit diesem Versuch konnte die Eignung der erfindungsgemäßen Verbindungen als Marker für venöse Thromben gezeigt werden.

### Beispiel 6 MRT-Darstellung (ex-vivo) eines venösen Thrombus nach intravenöser Gabe des Kontrastmittels in Kaninchen

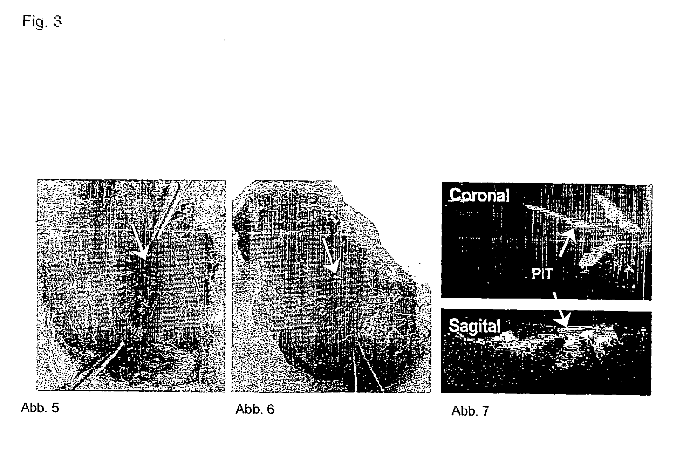

Die MR-Bildgebung erfolgte in Kaninchen mit photochemisch induziertem Thrombus (PIT). Durch Bestrahlung mit Xenon-Licht (540 nm, 1.100 klux, 25 min) nach i.v.-Injektion von Rose-Bengal (20 mg/kg) wurde in der linken Femoralvene die Thrombusbildung induziert. 24 Stunden nach intravenöser Applikation (ca. 1 h nach der Thrombus-Induktion) von 0.1 mmol Gd/kg der Titelverbindung aus Beispiel 21 WO 02/13874, wurde das Tier getötet und die linke Femoralvene (mit dem Thrombus) herauspräpariert. Das Imaging des geschädigten Venenabschnittes erfolgte mit einem Magnetom Harmony (Siemens, 1T) unter Verwendung einer T1-gewichteten Spinecho-Sequenz (TR/TE/α = 300/12ms/90°, mit und ohne Fettunterdrückung).
Der induzierte Thrombus ist im Präparat durch die Farbänderung deutlich sichtbar. Zusätzlich befinden sich auch Blutgerinnsel außerhalb des Gefäßes. In der ex-vivo MR-Bildgebung ist mit der T1-gewichteten Spinecho-Sequenz ein deutliches Enhancement der Thromben zu beobachten (siehe Fig. 3, Abbildungen 5 bis 7).

### Beispiel 7: Bestimmung der Gadolinium-Anreicherung im Thrombus nach intravenöser Gabe des Kontrastmittels in Kaninchen

Die Gehaltsbestimmung erfolgte in Kaninchen mit photochemisch induziertem Thrombus (PIT). Durch Bestrahlung mit Xenon-Licht (540 nm, 1.100 klux, 25 min) nach i.v.-Injektion von Rose-Bengal (20 mg/kg) wurde in der linken Femoralvene die Thrombusbildung induziert. 24 Stunden nach intravenöser Applikation (ca. 1 h nach der Thrombus-Induktion) von 0.1 mmol Gd/kg der Titelverbindung aus Beispiel 21 WO 02/13874, wurde das Tier getötet und verschiedene Organe und Gewebe zur Bestimmung des Gd-Gehaltes entnommen: Blut, Femoralvenen (mit und ohne Thrombus), Muskel. Nach Aufschluss der Gewebeproben wurde die Gadolinium-Konzentration (ppm) mittels ICP-AES gemessen.
In der linken Femoralvene (mit Thrombus) betrug die Gd-Konzentration 63 ppm, im Kontrollgefäß dagegen nur 35 ppm. Das Blutgerinnsel außerhalb des Gefäßes wies einen hohen Gd-Gehalt von 166 ppm auf. Im Blut war zum Zeitpunkt 24 h p.i. nur eine Gd-Konzentration von 15 ppm und im nicht-signal-verstärkten Muskel von 10 ppm nachweisbar.

## Patentansprüche

1. Verwendung von perfluoralkylhaltigen Metallkomplexen, die eine kritische Mizellbildungskonzentration <10⁻³ mol/l, einen hydrodynamischen Mizelldurchmesser (2 Rh) > 1 nm und eine Protonen-Relaxivity im Plasma (R¹) bei 40°C und einer Feldstärke von 0.47 Tesla > 10 l/mmol·s aufweisen, zur Herstellung eines Kontrastmittel im MR-Imaging zur Darstellung von intravasalen Thromben.

2. Verwendung nach Anspruch 1,
**dadurch gekennzeichnet, dass**
die Metallkomplexe als MRI-Kontrastmittel zur Darstellung von venösen Thromben eingesetzt werden.

3. Verwendung nach Anspruch 1 oder 2,
**dadurch gekennzeichnet, dass**
die Metallkomplexe als MRI-Kontrastmittel zur Darstellung von arteriellen Thromben eingesetzt werden.

4. Verwendung nach einem der Ansprüche 1 bis 3,
**dadurch gekennzeichnet, dass**
die Metallkomplexe als MRI-Kontrastmittel zur Frühbestimmung einer thrombotischen Verschlußerkrankung eingesetzt werden.

5. Verwendung nach Anspruch 1,
**dadurch gekennzeichnet, dass**
Metallkomplexe eingesetzt werden, deren Mizellbildungskonzentration < 10⁻⁴ mol/l ist.

6. Verwendung nach Anspruch 1,
**dadurch gekennzeichnet, dass**
Metallkomplexe eingesetzt werden, deren hydrodynamischer Mizelldurchmesser ≥ 3 nm ist, vorzugsweise > 4 nm.

7. Verwendung nach Anspruch 1,
**dadurch gekennzeichnet, dass**
Metallkomplexe eingesetzt werden, die eine Protonen-Relaxivity im Plasma von > 13 l/mmol·s, vorzugsweise > 15 l/mmol·s, aufweisen.

8. Verwendung nach einem der Ansprüche 1 bis 7,
**dadurch gekennzeichnet, dass**
als perfluoralkylhaltige Metallkomplexe die Verbindungen der allgemeinen Formel I
R^{F}-L-K I
worin
R^{F} eine perfluorierte, geradkettige oder verzweigte Kohlenstoffkette mit der Formel -CₙF₂ₙE ist, in der
E ein endständiges Fluor-, Chlor-, Brom-, Jod- oder Wasserstoffatom darstellt und n für die Zahlen 4-30 steht,
L eine direkte Bindung, eine Methylengruppe, eine -NHCO-Gruppe, eine Gruppe 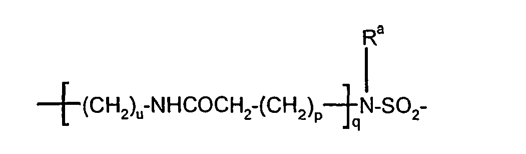 wobei p die Zahlen 0 bis 10, q und u unabhängig voneinander die Zahlen 0 oder 1 und
R^{a} ein Wasserstoffatom, eine Methylgruppe, eine Benzylgruppe, eine Phenylgruppe, eine -CH₂-OH-, CH₂OCH₃-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen, eine Gruppe -SO₃H- bedeuten,
oder eine geradkettige, verzweigte, gesättigte oder ungesättigte C₂-C₃₀- Kohlenstoffkette ist, die gegebenenfalls 1 bis 10 Sauerstoffatome, 1 bis 3 -NR^{a}-Gruppen, 1 bis 2 Schwefelatome, ein Piperazin, eine -CONR^{a}-Gruppe, eine bis sechs -NR^{a}CO-Gruppe, eine -SO₂-Gruppe, eine -NR^{a}-CO₂-Gruppe, 1 bis 2 -CO-Gruppen, eine Gruppe 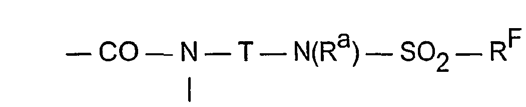 oder 1 bis 2 gegebenenfalls substituierte Aryle enthält und/oder durch diese Gruppen unterbrochen ist, und/oder gegebenenfalls substituiert ist mit 1 bis 3 -OR^{a}-Gruppen, 1 bis 2 Oxogruppen, 1 bis 2 -NH-COR^{a}-Gruppen, 1 bis 2 -CONHR^{a}-Gruppen, 1 bis 2 -(CH₂)ₚ-CO₂H-Gruppen, 1 bis 2 Gruppen -(CH₂)ₚ-(O)_{q}-CH₂CH₂-R^{F}, wobei
R^{a}, R^{F} und p und q die oben angegebenen Bedeutungen haben und
T eine C₂-C₁₀-Kette bedeutet, die gegebenenfalls durch 1 bis 2 Sauerstoffatome oder 1 bis 2 -NHCO-Gruppen unterbrochen ist,
K für einen Komplexbildner oder Metallkomplex oder deren Salze organischer und/oder anorganischer Basen oder Aminosäuren oder Aminosäureamide steht, und zwar für einen Komplexbildner oder Komplex der allgemeinen Formel II 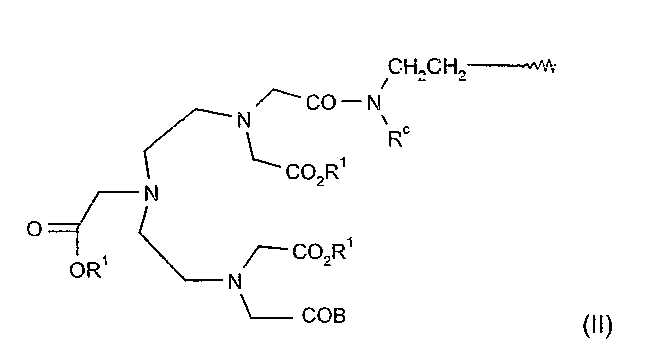 in der R^{C}, R¹ und B unabhängig voneinander sind und
R^{C} die Bedeutung von R^{a} hat oder -(CH₂)ₘ-L-R^{F} bedeutet, wobei m 0, 1 oder 2 ist und L und R^{F} die o.g. Bedeutung haben,
R¹ unabhängig voneinander ein Wasserstoffatom oder ein Metallionenäquivalent der Ordnungszahlen 22 - 29, 42-46 oder 58-70 bedeutet,
B -OR¹ oder 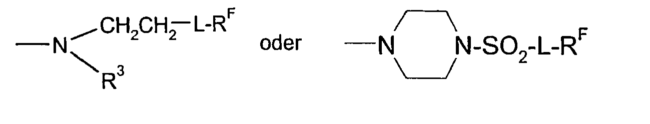
bedeutet, wobei R¹, L, R^{F} und R^{C} die o. g. Bedeutungen haben,
oder
für einen Komplexbildner oder Komplex der allgemeinen Formel III 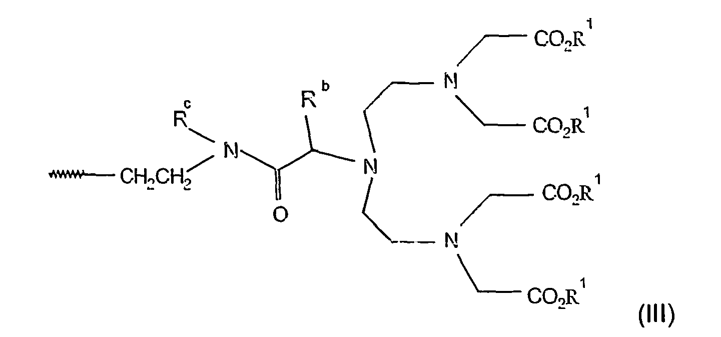 in der R^{c} und R¹ die oben genannten Bedeutungen aufweisen, R^{b} die Bedeutung von R^{a} hat
oder
für einen Komplexbildner oder Komplex der allgemeinen Formel IV 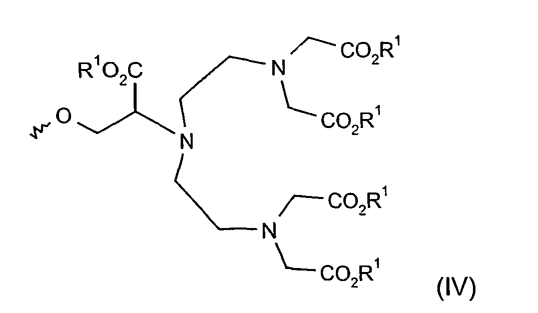 in der R¹ die oben genannte Bedeutung hat
oder
für einen Komplexbildner oder Komplex der allgemeinen Formel V 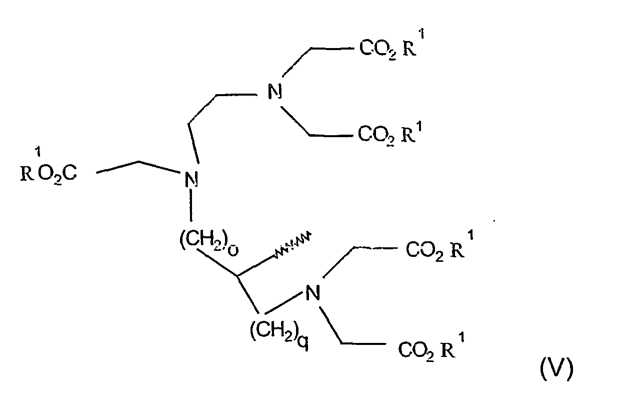 in der R¹ die oben genannte Bedeutung hat und o und q für die Ziffern
O oder 1 stehen und die Summe o + q = 1 ergibt,
oder
für einen Komplexbildner oder Komplex der allgemeinen Formel VI 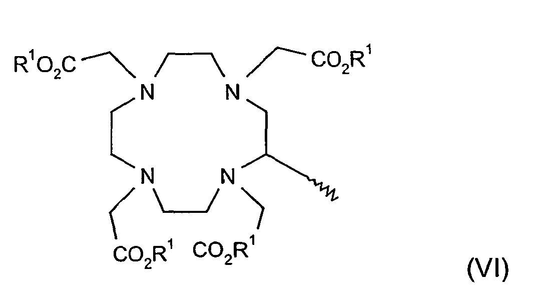 in der R¹ die oben genannte Bedeutung hat
oder
für einen Komplexbildner oder Komplex der allgemeinen Formel VII 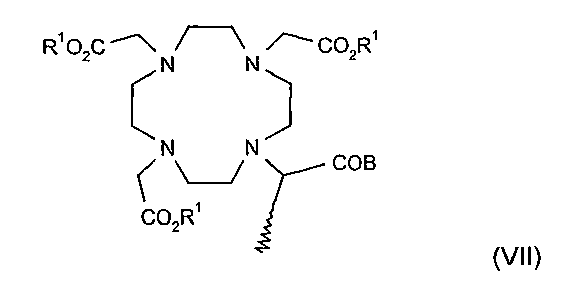 in der R¹ und B die oben genannten Bedeutungen haben
oder
für einen Komplexbildner oder Komplex der allgemeinen Formel VIII 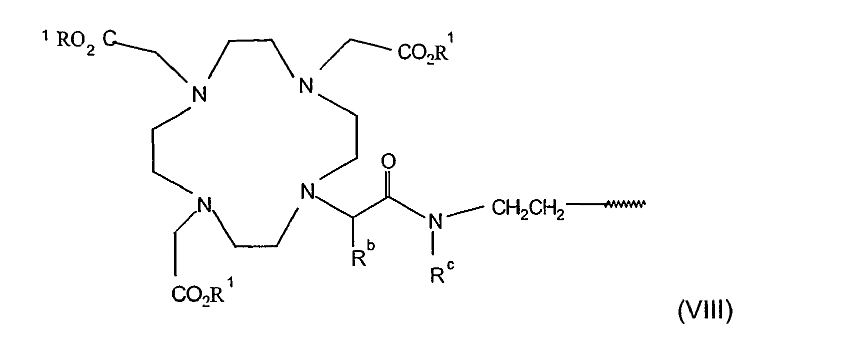 in der R^{c} und R¹ die oben genannten Bedeutungen haben und R^{b} die o.g. Bedeutung von R^{a} hat.
oder
für einen Komplexbildner oder Komplex der allgemeinen Formel IX 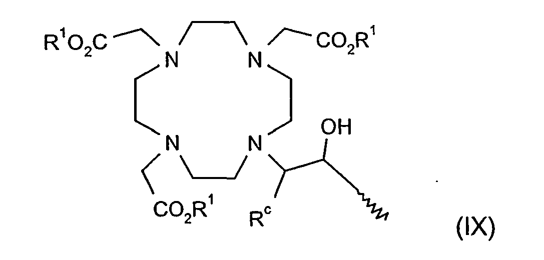 in der R^{c} und R¹ die oben genannten Bedeutungen haben,
oder
für einen Komplexbildner oder Komplex der allgemeinen Formel X 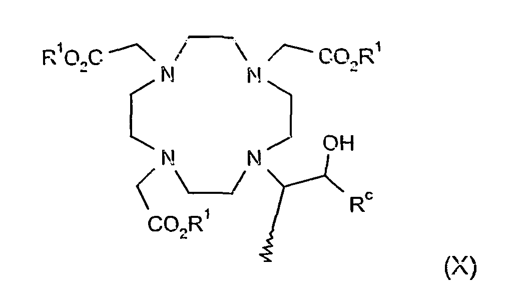 in der R^{c} und R¹ die oben genannten Bedeutungen haben,
oder
für einen Komplexbildner oder Komplex der allgemeinen Formel XI 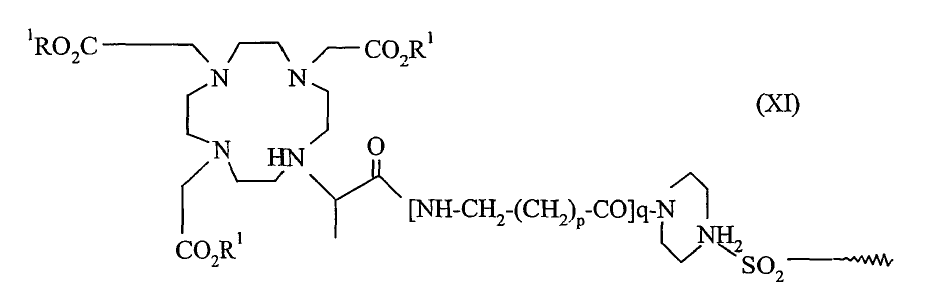 in der R¹ , p und q die oben genannte Bedeutung haben und R^{b} die Bedeutung von R^{a} hat.
oder
für einen Komplexbildner oder Komplex der allgemeinen Formel XII 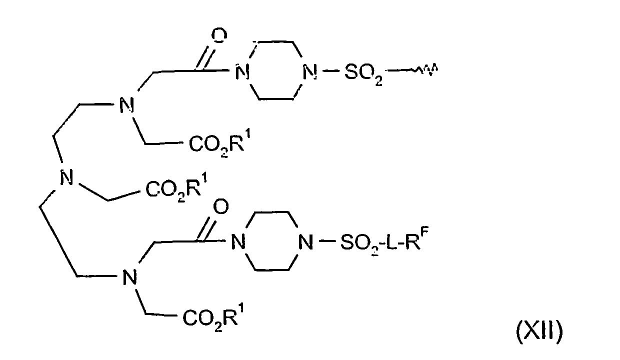 in der L, R^{F} und Z¹ die oben genannten Bedeutungen haben, oder
für einen Komplexbildner oder Komplex der allgemeinen Formel XIII 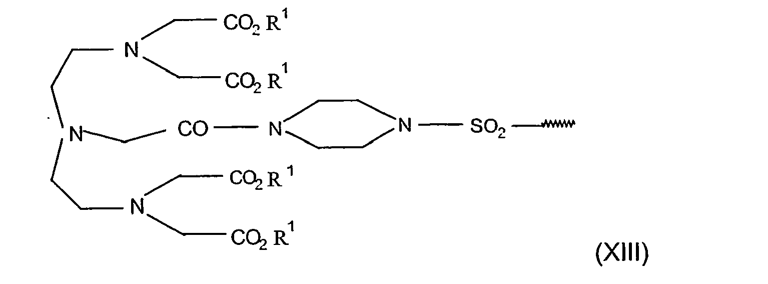 in der R¹ die oben genannte Bedeutung hat,
eingesetzt werden.

9. Verwendung nach Anspruch 8,
**dadurch gekennzeichnet, dass**
die Verbindungen der allgemeinen Formel I eingesetzt werden, in der L für
α-CH₂-β
α-CH₂CH₂-β
α-(CH₂)ₛ-β s = 3 - 15
α-CH₂-O-CH₂CH₂-β
α-CH₂-(O-CH₂-CH₂-)ₜ-β t = 2 - 6
α-CH₂-NH-CO-β
α-CH₂-NH-CO-CH₂-N(CH₂COOH)-SO₂-β
α-CH₂-NH-CO-CH₂-N(C₂H₅)-SO₂-β
α-CH₂-NH-CO-CH₂-N(C₁₀H₂₁)-SO₂-β
α-CH₂-NH-CO-CH₂-N(C₆H₁₃)-SO₂-β
α-CH₂-NH-CO-(CH₂)₁₀-N(C₂H₅)-SO₂-β
α-CH₂-NH-CO-CH₂-N(-CH₂-C₆H₅)-SO₂-β
α-CH₂-NH-CO-CH₂-N(-CH₂-CH₂-OH)SO₂-β
α-CH₂-NHCO-(CH₂)₁₀-S-CH₂CH₂-β
α-CH₂NHCOCH₂-O-CH₂CH₂-β
α-CH₂NHCO(CH₂)₁₀-O-CH₂CH₂-β
α-CH₂-C₆H₄-O-CH₂CH₂-β
α-CH₂-O-CH₂-C(CH₂-OCH₂CH₂-C₆F₁₃)₂-CH₂-OCH₂-CH₂-β 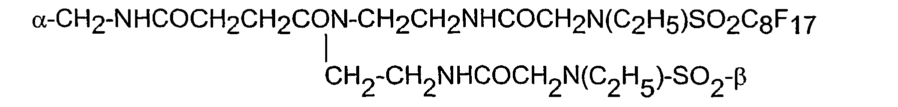 α -CH₂-O-CH₂-CH(OC₁₀H₂₁)-CH₂-O-CH₂CH₂-β
α-(CH₂NHCO)₄-CH₂O-CH₂CH₂-β
α-(CH₂NHCO)₃-CH₂O-CH₂CH₂-β
α-CH₂-OCH₂C(CH₂OH)₂-CH₂-O-CH₂CH₂-β 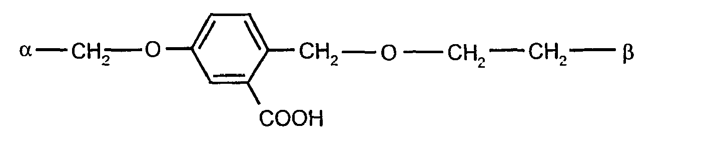 α -CH₂NHCOCH₂N(C₆H₅)-SO₂-β
α-NHCO-CH₂-CH₂-β
α-NHCO-CH₂-O-CH₂CH₂-β
α-NH-CO-β
α-NH-CO-CH₂-N(CH₂COOH)-SO₂-β
α-NH-CO-CH₂-N(C₂H₅)-SO₂-β
α-NH-CO-CH₂-N(C₁₀H₂₁)-SO₂-β
α-NH-CO-CH₂-N(C₆H₁₃)-SO₂-β
α-NH-CO-(CH₂)₁₀-N(C₂H₅)-SO₂-β
α-NH-CO-CH₂-N(-CH₂-C₆H₅)-SO₂-β
α-NH-CO-CH₂-N(-CH₂-CH₂-OH)SO₂-β
α-NH-CO-CH₂-β
α-CH₂-O-C₆H₄-O-CH₂-CH₂-β
α-CH₂-C₆H₄-O-CH₂-CH₂-β
α-N(C₂H₅)-SO₂-β
α-N(C₆H₅)-SO₂-β
α-N(C₁₀H₂₁)-SO₂-β
α-N(C₆H₁₃)-SO₂-β
α-N(C₂H₄OH)-SO₂-β
α-N(CH₂COOH)-SO₂-β
α-N(CH₂C₆H₅)-SO₂-β
α-N-[CH(CH₂OH)₂]-SO₂-β
α-N-[CH(CH₂OH)CH(CH₂OH)]-SO₂-β
steht und worin α die Bindungsstelle zum Komplexbildner oder Metallkomplex K und β die Bindungsstelle zum Fluorrest darstellt.

10. Verwendung nach Anspruch 8 oder 9,
**dadurch gekennzeichnet, dass**
die Verbindungen der Formel I eingesetzt werden, in der n in der Formel - CₙF₂ₙE für die Zahlen 4-15 steht und/oder E in dieser Formel ein Fluoratom bedeutet.

11. Verwendung nach einem der Ansprüche 8 bis 10,
**dadurch gekennzeichnet, dass**
die folgenden Verbindungen eingesetzt werden:
- Gadolinium-Komplex von 10-[1-Methyl-2-oxo-3-aza-5-oxo-{4-perfluoro-octylsulfonyl-piperazin-1-yl}-pentyl]-1,4,7-tris(carboxymethyl)-1,4,7,10-tetraazacyclododecan,
- Gadolinium-Komplex von 10-[2-Hydroxy-4-aza-5-oxo-7-oxa-10,10,11,11, 12,12, 13,13,14,14,15,15,16,16,17,17-heptadecafluorheptacecyl]-1,4,7-tris(carboxymethyl)-1,4,7,10-tetraazacyclododecan,
- Gadolinium-Komplex von 10-[2-Hydroxy-4-aza-5,9-dioxo-9-{4-perfluoroctyl)-piperazin-1-yl}-nonyl]-1,4,7-tris(carboxymethyl)-1,4,7,10-tetraazacyclododecan,
- Gadolinium-Komplex von 10-[2-Hydroxy-4-aza-5-oxo-7-aza-7-(perfluoroctylsulfonyl)-nonyl]-1,4,7-tris(carboxymethyl)-1,4,7,10-tetraazacyclododecan,
- Gadolinium-Komplex von 10-[2-Hydroxy-4-oxa-1H,1H,2H,3H,3H,5H,5H, 6H,6H-perfluor-tetradecyl]-1,4,7-tris(carboxymethyl)-1,4,7,10-tetraazacyclododecan,
- Gadolinium-Komplex von 10-[2-Hydroxy-4-aza-5-oxo-7-oxa-10,10,11,11, 12,12,13,13,14,14,15,15,16,16,17,17,18,18,19,19-henicosafluor-nonadecyl]-1,4,7-tris(carboxymethyl)-1,4,7,10-tetraazacyclododecan,
- Gadolinium-Komplex von 10-[2-Hydroxy-4-aza-5-oxo-11-aza-11-(perfluoroctylsulfonyl)-tridecyl]-1-4-7-tris(carboxymethyl) 1,4,7,10-tetraazacyclododecan,
- Gadolinium-Komplex von 10-[2-Hydroxy-4-aza-5-oxo-7-aza-7-(perfluoroctylsulfonyl)-8-phenyl-octyl]-1-4-7-tris(carboxymethyl)-1,4,7,10-tetraazacyclododecan.

12. Verwendung nach einem der Ansprüche 1-7,
**dadurch gekennzeichnet, dass**
als perfluoralkylhaltige Metallkomplexe die Verbindungen der allgemeinen Formel la
A-R^{F} (Ia)
worin
• A ein Molekülteil ist, der 2 - 6 Metallkomplexe enthält, die direkt oder über einen Linker an ein Stickstoffatom einer ringförmigen Gerüstkette gebunden sind
und
• R^{F} eine perfluorierte, geradkettige oder verzweigte Kohlenstoffkette mit der Formel -CₙF₂ₙE ist, in der
E ein endständiges Fluor-, Chlor-, Brom-, Jod- oder Wasserstoffatom darstellt
und n für die Zahlen 4 - 30 steht,
wobei das Molekülteil A die folgende Struktur aufweist: 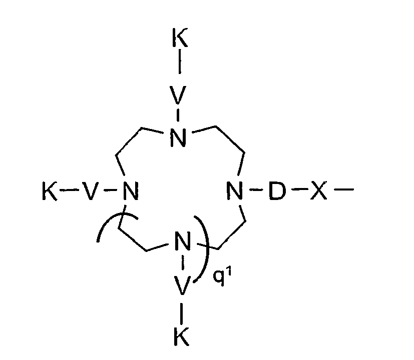 wobei
• q¹ eine Zahl 0, 1, 2 oder 3 ist,
• K für einen Komplexbildner oder Metallkomplex oder deren Salze organischer und/oder anorganischer Basen oder Aminosäuren oder Aminosäureamide steht,
• X eine direkte Bindung zur Perfluoralkylgruppe, eine Phenylengruppe oder eine C₁-C₁₀-Alkylenkette ist, die gegebenenfalls 1 - 15 Sauerstoff-, 1 - 5 Schwefelatome, 1 - 10 Carbonyl-, 1 - 10 (NR^{d})-, 1 - 2 NR^{d}SO₂-, 1 - 10 CONR^{d}-, 1 Piperidin-, 1 - 3 SO₂-, 1 - 2 Phenylengruppen enthält oder gegebenenfalls durch 1 - 3 Reste R^{F} substituiert ist, worin R^{d} für ein Wasserstoffatom, eine Phenyl-, Benzyl- oder eine C₁-C₁₅-Alkylgruppe steht, die gegebenenfalls 1 - 2 NHCO-, 1 - 2 CO-Gruppen, 1 - 5 Sauerstoffatome enthält und gegebenenfalls durch 1 - 5 Hydroxy-, 1-5 Methoxy-, 1 - 3 Carboxy-, 1 - 3 R^{F}-Reste substituiert ist
• V eine direkte Bindung oder eine Kette der allgemeinen Formel IIa oder IIIa ist: 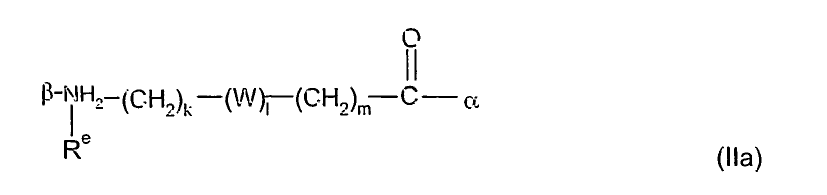 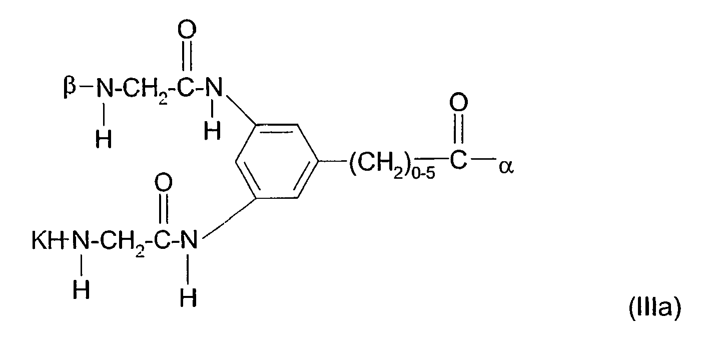
worin
■ R^{e} ein Wasserstoffatom, eine Phenylgruppe, eine Benzylgruppe oder eine C₁-C₇ Alkylgruppe ist, die gegebenenfalls substituiert ist mit einer Carboxy-, einer Methoxy- oder einer Hydroxygruppe,
■ W eine direkte Bindung, eine Polyglycolethergruppe mit bis zu 5 Glycoleinheiten oder ein
Molekülteil der allgemeinen Formel IVa ist
-CH(R^{h})- (IVa)
worin R^{h} eine C₁-C₇-Carbonsäure, eine Phenylgruppe, eine Benzylgruppe oder eine -(CH₂)₁₋₅-NH-K-Gruppe ist,
■ α die Bindung an das Stickstoffatom der Gerüstkette, β die Bindung zum Komplexbildner oder Metallkomplex K darstellt,
■ und in der die Variablen k und m für natürliche Zahlen zwischen 0 und 10 und I für 0 oder 1 stehen,
und wobei
o D eine CO- oder SO₂-Gruppe ist,
eingesetzt werden.

13. Verwendung nach Anspruch 12,
**dadurch gekennzeichnet, dass**
die Verbindungen der allgemeinen Formel la eingesetzt werden, in der q die Zahl 1 ist.

14. Verwendung nach Anspruch 12,
**dadurch gekennzeichnet, dass**
die Verbindungen der allgemeinen Formel la eingesetzt werden, in der der Molekülteil X eine Alkylenkette ist, die 1 - 10 CH₂CH₂O- oder 1 - 5 COCH₂NH-Gruppen enthält, eine direkte Bindung oder eine der folgenden Strukturen
γ-CH₂-O-(CH₂)₂-δ, 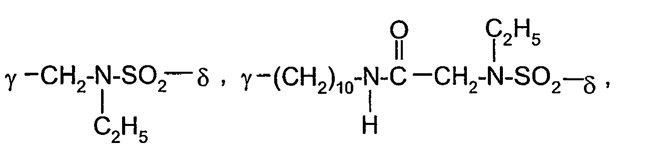 γ -(CH₂)₁₀-O-(CH₂)₂-δ , 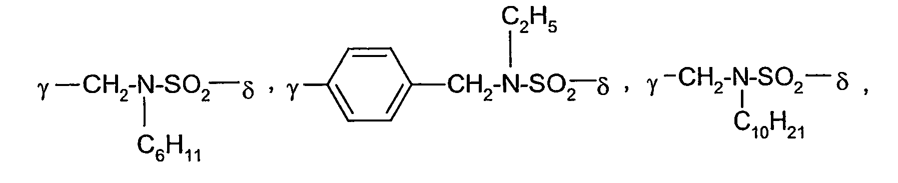 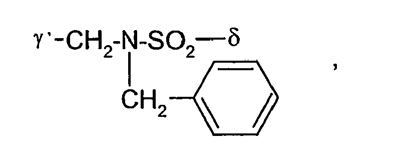 wobei
γ an D und δ an R^{F} bindet.

15. Verwendung nach Anspruch 12,
**dadurch gekennzeichnet, dass**
die Verbindungen der allgemeinen Formel la eingesetzt werden, in der V ein Molekülteil mit einer der folgenden Strukturen ist

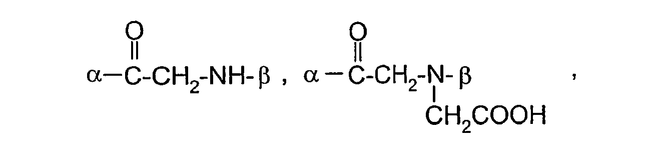

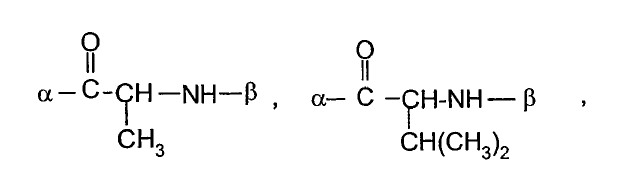

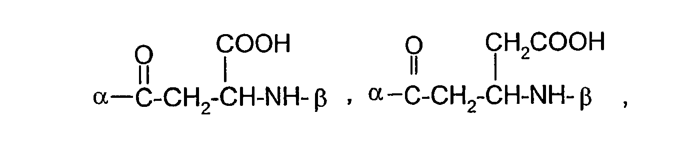

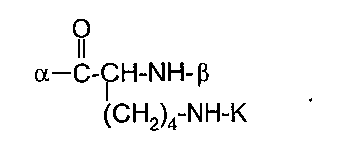

16. Verwendung nach Anspruch 12,
**dadurch gekennzeichnet, dass**
die Verbindungen der allgemeinen Formel Ia eingesetzt werden, in der K einen Komplex der allgemeinen Formel Va, VIa, VIIa oder VIIIa darstellt 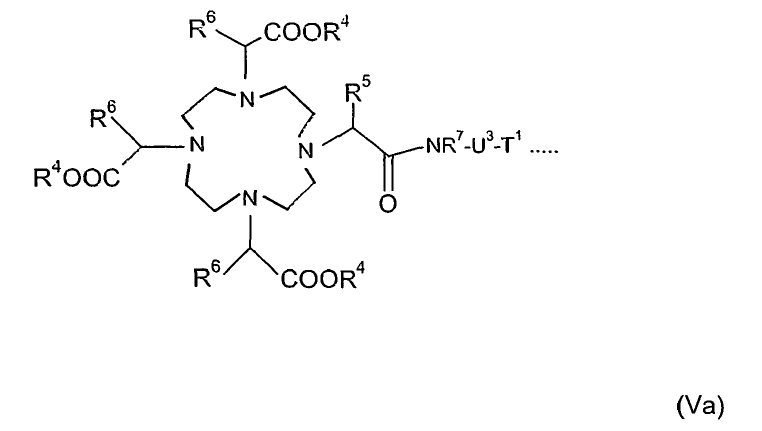 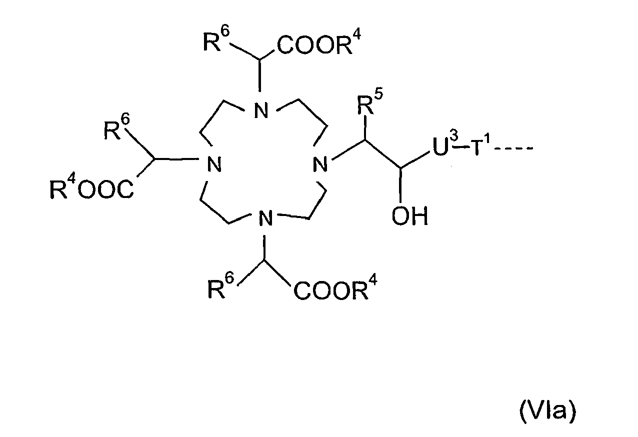 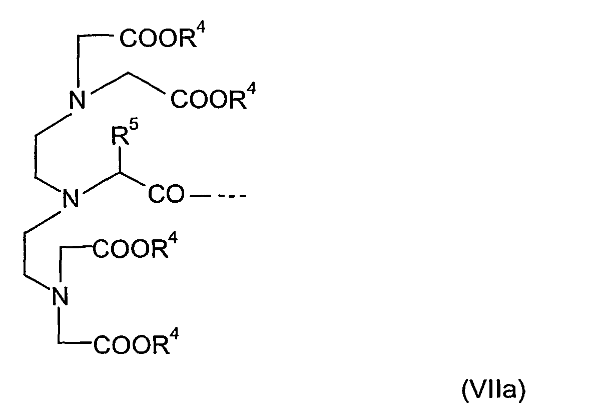 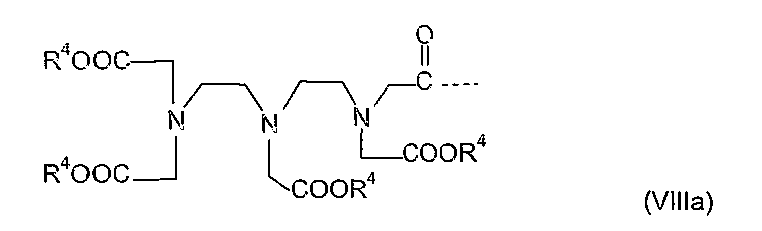 wobei
• R⁴ unabhängig voneinander ein Wasserstoffatom oder ein Metallionenäquivalent der Elemente der Ordnungszahlen 23-29, 42-46 oder 58-70 ist,
• R⁵ ein Wasserstoffatom oder eine geradkettige, verzweigte, gesättigte oder ungesättigte C₁-C₃₀-Alkylkette ist, die gegebenenfalls substituiert ist durch 1 -5 Hydroxy-, 1 - 3 Carboxy- oder 1 Phenylgruppe(n) und/oder gegebenenfalls durch 1 - 10 Sauerstoffatome, 1 Phenylen- oder 1 Phenylenoxygruppe unterbrochen ist
• R⁶ ein Wasserstoffatom, ein geradkettiger oder verzweigter C₁-C₇-Alkylrest, ein Phenyl- oder Benzylrest ist,
• R⁷ ein Wasserstoffatom, eine Methyl- oder Ethylgruppe ist, die gegebenenfalls substituiert ist durch eine Hydroxy- oder Carboxygruppe,
• U³ eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-,
1 Harnstoff-, 1 Thioharnstoff-, 1-2 Carboxyalkylimino-, 1-2 Estergruppen, 1 - 10 Sauerstoff-, 1-5 Schwefel- und/oder 1-5 Stickstoffatome enthaltende und/oder gegebenenfalls durch 1 - 5 Hydroxy-, 1-2 Mercapto-, 1 - 5 Oxo-, 1 - 5 Thioxo-,
1 - 3 Carboxy-, 1 - 5 Carboxyalkyl-, 1 - 5 Ester- und/oder 1 - 3 Aminogruppen substituierte, geradkettige, verzweigte, gesättigte oder ungesättigte C₁-C₂₀-Alkylengruppe ist, wobei die gegebenenfalls enthaltenen Phenylengruppen durch 1 - 2 Carboxy-, 1 - 2 Sulfon- oder 1 - 2 Hydroxygruppen substituiert sein können
• T¹ für eine -CO-β, -NHCO-β oder -NHCS-β-Gruppe steht, wobei β die Bindungsstelle an V darstellt.

17. Verwendung nach Anspruch 16,
**dadurch gekennzeichnet, dass**
die für U³ stehende C₁-C₂₀-Alkylenkette die Gruppen -CH₂NHCO-, -NHCOCH₂O-, -NHCOCH₂OC₆H₄-, -N(CH₂CO₂H)-, -CH₂OCH₂-,
-NHCOCH₂C₆H₄-, -NHCSNHC₆H₄-, -CH₂OC₆H₄-, -CH₂CH₂O-enthält und/oder durch die Gruppen -COOH, -CH₂COOH substituiert ist.

18. Verwendung nach Anspruch 16,
**dadurch gekennzeichnet, dass**
U³ für eine
-CH₂-, -CH₂CH₂-, -CH₂CH₂CH₂-, -C₆H₄-, -C₆H₁₀-, -CH₂C₆H₄-,
-CH₂NHCOCH₂CH(CH₂CO₂H)-C₆H₄-,
-CH₂NHCOCH₂OCH₂-,
-CH₂NHCOCH₂C₆H₄-gruppe steht.

19. Verwendung nach Anspruch 12,
**dadurch gekennzeichnet, dass**
die Verbindungen der allgemeinen Formel la eingesetzt werden, in der K eine der folgenden Strukturen aufweist:

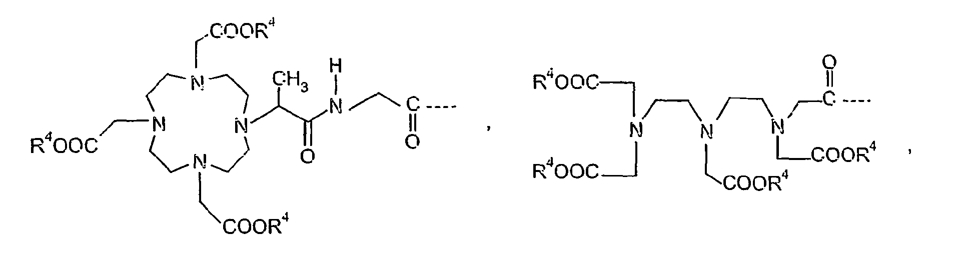

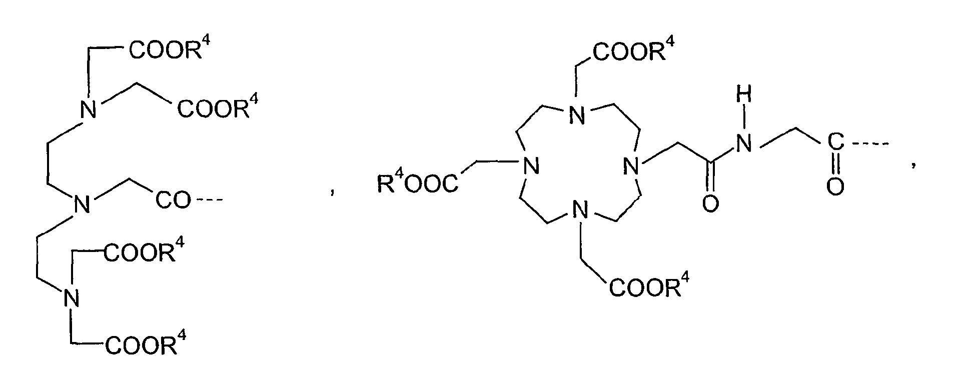

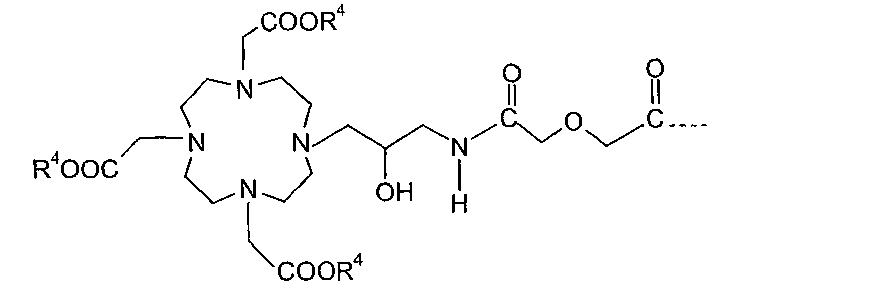

20. Verwendung nach einem der Ansprüche 12 bis 19,
**dadurch gekennzeichnet, dass**
die Verbindungen der allgemeinen Formel la eingesetzt werden, in der die Perfluoralkylkette R^{F} -C₆F₁₃, -C₈F₁₇, -C₁₀F₂₁ oder -C₁₂F₂₅ ist.

21. Verwendung nach einem der Ansprüche 12 bis 20,
**dadurch gekennzeichnet, dass**
der Gadolinium-Komplex von 1,4,7-Tris{1,4,7-tris(N-(carboxylatomethyl)-10-[N-1-methyl-3,8-diaza-2,5,8-trioxooctan-1,8-diyl)]-1,4,7,10-tetraazacyclododecan, Gd-Komplex}-10-[N-2H, 2H, 4H, 4H, 5H, 5H-3-oxa-perfluortridecanoyl]-1,4,7,10-tetraazacyclododecan eingesetzt wird.

22. Verwendung nach einem der Ansprüche 1 bis 7,
**dadurch gekennzeichnet, dass**
als perfluoralkylhaltige Metallkomplexe die Verbindungen der allgemeinen Formel Ib 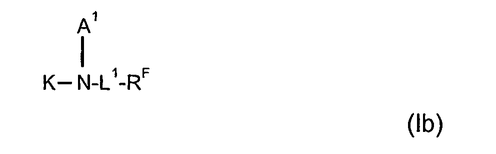 worin
K einen Komplexbildner oder einen Metallkomplex der allgemeinen Formel IIb 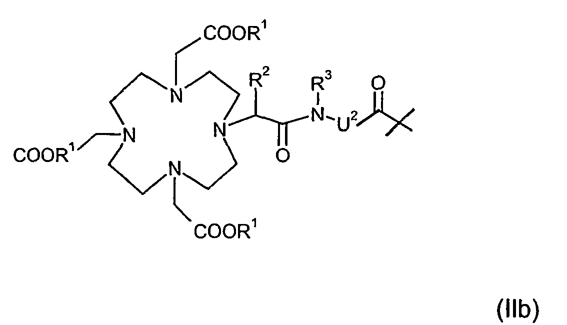 wobei
R¹ für ein Wasserstoffatom oder ein Metallionenäquivalent der Ordnungszahlen 23-29, 42-46 oder 58-70,
R² und R³ für ein Wasserstoffatom, eine C₁-C₇-Alkylgruppe, eine Benzylgruppe, eine Phenylgruppe, -CH₂OH oder-CH₂-OCH₃,
U² für den Rest L¹, wobei aber L¹ und U² unabhängig voneinander gleich oder verschieden sein können,
steht,
bedeutet,
A¹ ein Wasserstoffatom, eine geradkettige oder verzweigte C₁-C₃₀-Alkylgruppe, die gegebenenfalls unterbrochen ist durch 1-15 Sauerstoffatome, und/oder gegebenenfalls substituiert ist mit 1-10 Hydroxygruppen, 1-2 COOH-Gruppen, einer Phenylgruppe, einer Benzylgruppe und/oder 1-5 - OR^{g}-Gruppen, mit R^{g} in der Bedeutung eines Wasserstoffatoms oder eines C₁-C₇-Alkylrestes, oder -L¹-R^{F} bedeutet,
L¹ eine geradkettige oder verzweigte C₁-C₃₀-Alkylengruppe, die gegebenenfalls unterbrochen ist durch 1-10 Sauerstoffatome, 1-5-NH-CO-Gruppen, 1-5 -CO-NH- Gruppen, durch eine gegebenenfalls durch eine COOH-Gruppe substituierte Phenylengruppe, 1-3 Schwefelatome, 1-2 -N(B¹)-SO₂- Gruppen, und/oder 1-2 -SO₂-N(B¹)- Gruppen mit B¹ in der Bedeutung von A¹ und/oder gegebenenfalls substituiert ist mit dem Rest R^{F},
bedeutet und
R^{F} einen geradkettigen oder verzweigten perfluorierten Alkylrest der Formel CₙF₂ₙE,
wobei n für die Zahlen 4-30 steht und
E für ein endständiges Fluoratom, Chloratom, Bromatom, lodatom oder ein Wasserstoffatom steht,
bedeutet,
und gegebenenfalls vorhandene Säuregruppen gegebenenfalls als Salze organischer und/oder anorganischer Basen oder Aminosäuren oder Aminosäureamide vorliegen können,
eingesetzt werden.

23. Verwendung nach Anspruch 22,
**dadurch gekennzeichnet, dass**
die Verbindungen der allgemeinen Formel Ib eingesetzt werden, in der R², R³ und R^{g} unabhängig voneinander Wasserstoff oder eine C₁-C₄-Alkylgruppe bedeuten.

24. Verwendung nach Anspruch 22,
**dadurch gekennzeichnet, dass**
die Verbindungen der allgemeinen Formel lb eingesetzt werden, in der A¹ Wasserstoff, einen C₁-C₁₅-Alkylrest,
die Reste C₂H₄-O-C-H₃, C₃H₆-O-CH₃,
C₂H₄-O-(C₂H₄-O)ₜ-C₂H₄-OH,
C₂H₄-O-(C₂H₄-O)ₜ-C₂H₄-OCH₃,
C₂H₄OH, C₃H₆OH, C₄H₈OH, C₅H₁₀OH, C₆H₁₂OH, C₇H₁₄OH,
CH(OH)CH₂OH,
CH(OH)CH(OH)CH₂OH, CH₂[CH(OH)]ᵤ¹CH₂OH,
CH[CH₂(OH)]CH(OH)CH₂OH,
C₂H₄CH(OH)CH₂OH,
(CH₂)ₛCOOH,
C₂H₄-O-(C₂H₄-O)ₜ-CH₂COOH oder
C₂H₄-O-(C₂H₄-O)ₜ-C₂H₄-CₙF₂ₙE bedeutet,
wobei
s für die ganzen Zahlen 1 bis 15,
t für die ganzen Zahlen 0 bis 13,
u¹ für die ganzen Zahlen 1 bis 10,
n für die ganzen Zahlen 4 bis 20 steht, und
E für ein Wasserstoff-, Fluor-, Chlor-, Brom- oder lodatom
sowie, falls möglich, ihre verzweigten Isomeren.

25. Verwendung nach Anspruch 22,
**dadurch gekennzeichnet, dass**
die Verbindungen der allgemeinen Formel Ib eingesetzt werden, in der A¹
Wasserstoff, C₁-C₁₀-Alkyl,
C₂H₄-O-CH₃, C₃H₆-O-CH₃,
C₂H₄-O-(C₂H₄-O)ₓ-C₂H₄-OH, C₂H₄-O-(C₂H₄-O)ₓ-C₂H₄-OCH₃,
C₂H₄OH, C₃H₆OH,
CH₂[CH(OH)]_{y}CH₂OH,
CH[CH₂(OH)]CH(OH)CH₂OH,
(CH₂)_{w}COOH,
C₂H₄-O-(C₂H₄-O)ₓ-CH₂COOH,
C₂H₄-O-(C₂H₄-O)ₓ-C₂H₄-CₙF₂ₙE bedeutet,
wobei
x für die ganzen Zahlen 0 bis 5,
y für die ganzen Zahlen 1 bis 6,
w für die ganzen Zahlen 1 bis 10,
n für die ganzen Zahlen 4 bis 15 und
E für ein Fluoratom steht, sowie, falls möglich, ihre verzweigten Isomeren.

26. Verwendung nach Anspruch 22,
**dadurch gekennzeichnet, dass**
die Verbindungen der allgemeinen Formel Ib eingesetzt werden, in der L¹
α-(CH₂)ₛ-β
α-CH₂-CH₂-(O-CH₂-CH₂-)_{y}-β
α-CH₂-(O-CH₂-CH₂-)_{y}-β,
α-CH₂-NH-CO-β
α-CH₂-CH₂-NH-SO₂-β
α-CH₂-NH-CO-CH₂-N(CH₂COOH)-SO₂-β
α-CH₂-NH-CO-CH₂-N(C₂H₅)-SO₂-β
α-CH₂-NH-CO-CH₂-N(C₁₀H₂₁)-SO₂-β
α-CH₂-NH-CO-CH₂-N(C₆H₁₃)-SO₂-β
α-CH₂-NH-CO-(CH₂)₁₀-N(C₂H₅)-SO₂-β
α-CH₂-NH-CO-CH₂-N(-CH₂-C₆H₅)-SO₂-β
α-CH₂-NH-CO-CH₂-N(-CH₂-CH₂-OH)SO₂-β
α-CH₂-NHCO-(CH₂)₁₀-S-CH₂CH₂-β
α-CH₂NHCOCH₂-O-CH₂CH₂-β
α-CH₂-CH₂NHCOCH₂-O-CH₂CH₂-β
α-CH₂-(CH₂-CH₂\-O)ₜ-(CH₂)₃NHCO-CH₂-O-CH₂CH₂-β
α-CH₂NHCO(CH₂)₁₀-O-CH₂CH₂-β
α-CH₂CH₂NHCO(CH₂)₁₀-O-CH₂CH₂-β
α-CH₂-C₆H₄-O-CH₂CH₂-β wobei die Phenylengruppe 1,4 oder 1,3 verknüpft ist
α-CH₂-O-CH₂-C(CH₂-OCH₂CH₂-C₆F₁₃)₂-CH₂-OCH₂-CH₂-β
α-CH₂-NHCOCH₂CH₂CON-CH₂CH₂NHCOCH₂N(C₂H₅)SO₂C₈F₁₇β
α-CH₂-CH₂NHCOCH₂N(C₂H₅)-SO₂-β
α-CH₂-O-CH₂-CH(OC₁₀H₂₁)-CH₂-O-CH₂CH₂-β
α-(CH₂NHCO)₄-CH₂O-CH₂CH₂-β
α-(CH₂NHCO)₃-CH₂O-CH₂CH₂-β
α-CH₂-OCH₂C(CH₂OH)₂-CH₂-O-CH₂CH₂-β 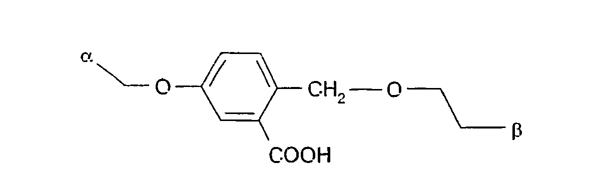 α -CH₂NHCOCH₂N(C₆H₅)-SO₂-β
α-NHCO-CH₂-CH₂-β
α-NHCO-CH₂-O-CH₂CH₂-β
α-NH-CO-β
α-NH-CO-CH₂-N(CH₂COOH)-SO₂-β
α-NH-CO-CH₂-N(C₂H₅)-SO₂-β
α-NH-CO-CH₂-N(C₁₀H₂₁)-SO₂-β
α-NH-CO-CH₂-N(C₆H₁₃)-SO₂-β
α-NH-CO-(CH₂)₁₀-N(C₂H₅)SO₂-β
α-NH-CO-CH₂-N(-CH₂-C₆H₅)-SO₂-β
α-NH-CO-CH₂-N(-CH₂-CH₂-OH)SO₂-β
α-NH-CO-CH₂-β
α-CH₂-O-C₆H₄-O-CH₂-CH₂-β
α-CH₂-C₆H₄-O-CH₂-CH₂-β
α-N(C₂H₅)-SO₂-β
α-N(C₆H₅)-SO₂-β
α-N(C₁₀H₂₁)-SO₂-β
α-N(C₆H₁₃)-SO₂-β
α-N(C₂H₄OH)-SO₂-β
α-N(CH₂COOH)-SO₂-β
α-N(CH₂C₆H₅)-SO₂-β
α-N-[CH(CH₂OH)₂]-SO₂-β
α-N-[CH(CH₂OH)CH(OH)(CH₂OH)]-SO₂-β bedeutet,
wobei
s für die ganzen Zahlen 1 bis 15 und
y für die ganzen Zahlen 1 bis 6 steht.

27. Verwendung nach Anspruch 22,
**dadurch gekennzeichnet, dass**
die Verbindungen der allgemeinen Formel Ib eingesetzt werden, in der L¹
α-CH₂-O-CH₂CH₂-β,
α-CH₂-CH₂-(O-CH₂-CH₂-)_{y}-β,
α-CH₂-(O-CH₂-CH₂-)_{y}-β,
α-CH₂-CH₂-NH-SO₂-β, Bsp. 10
α-CH₂NHCOCH₂-O-CH₂CH₂-β,
α-CH₂-CH₂NHCOCH₂-O-CH₂CH₂-β,
α-CH₂-(CH₂-CH₂-O)_{y}-(CH₂)₃NHCO-CH₂-O-CH₂CH₂-β,
α-CH₂NHCO(CH₂)₁₀-O-CH₂CH₂-β,
α-CH₂CH₂NHCO(CH₂)₁₀-O-CH₂CH₂-β,
α-CH₂-O-CH₂-CH(OC₁₀H₂₁)-CH₂-O-CH₂CH₂-β,
α-CH₂-O-C₆H₄-O-CH₂-CH₂-β oder
α-CH₂-C₆H₄-O-CH₂-CH₂-β bedeutet,
wobei
y für die ganzen Zahlen 1 bis 6 steht.

28. Verwendung nach Anspruch 22,
**dadurch gekennzeichnet, dass**
die Verbindungen der allgemeinen Formel Ib eingesetzt werden, in der R^{F} einen geradkettigen oder verzweigten perfluorierten Alkylrest der Formel CₙF₂ₙE bedeutet, wobei n für die Zahlen 4 bis 15 steht und E für ein endständiges Fluoratom steht.

29. Verwendung nach einem der Ansprüche 22 bis 28,
**dadurch gekennzeichnet, dass**
die folgenden Verbindungen eingesetzt werden:
- 1,4,7-Tris(carboxylatomethyl)-10-(3-aza-4-oxo-hexan-5-yl)-säure-N-(2,3-dihydroxypropyl)-N-(1H, 1H, 2H, 2H, 4H, 4H, 5H, 5H-3-oxa)-perfluortridecyl)-amid]-1,4,7,10-tetraazacyclododecan, Gadoliniumkomplex
- 1,4,7-Tris(carboxylatomethyl)-10-{(3-aza-4-oxo-hexan-5-yl)säure-N-(3,6,9, 12,15-pentaoxa)-hexadecyl)-(1H, 1H, 2H, 2H, 4H, 4H, 5H, 5H-3-oxa)-perfluortridecyl]-amid}-1,4,7,10-tetraazacyclododecan, Gadoliniumkomplex
- 1,4,7-Tris(carboxylatomethyl)-10-{(3-aza-4-oxo-hexan-5-yl)-säure-N-5-hydroxy-3-oxa-pentyl)-N-(1H, 1H, 2H, 2H, 4H, 4H, 5H, 5H-3-oxa)-perfluortridecyl]-amid}-1,4,7,10-tetraazacyclododecan, Gadoliniumkomplex
- 1,4,7-Tris(carboxylatomethyl)-10-{(3-aza-4-oxo-hexan-5-yl)-säure-[N-3,6,9,15-tetraoxa-12-aza-15-oxo-C₁₇-C₂₆-hepta-decafluor)hexacosyl]-amid}-1,4,7,10-tetraazacyclododecan, Gadoliniumkomplex
- 1,4,7-Tris(carboxylatomethyl)-10-[(3-aza-4-oxo-hexan-5-yl]-säure-N-(2-methoxyethyl)-N-(1H, 1H, 2H, 2H, 4H, 4H, 5H, 5H-3-oxa)-perfluortridecyl]-amid}-1,4,7,10-tetraazacyclododecan, Gadoliniumkomplex.

30. Verwendung nach einem der Ansprüche 1 bis 7,
**dadurch gekennzeichnet, dass**
als perfluoralkylhaltige Metallkomplexe die Verbindungen mit Zuckerresten der allgemeinen Formel lc 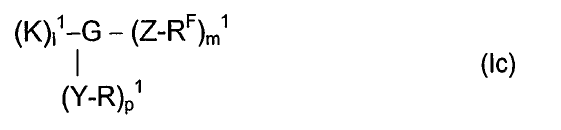 in der
R einen über die 1-OH- oder 1-SH-Position gebundenen Mono- oder Oligosaccharidrest darstellt,
R^{F} eine perfluorierte, geradkettige oder verzweigte Kohlenstoffkette mit der Formel -CₙF₂ₙE ist, in der E ein endständiges Fluor-, Chlor-, Brom-, Jod- oder Wasserstoffatom darstellt und n für die Zahlen 4-30 steht,
K für einen Metallkomplex der allgemeinen Formel IIc steht, 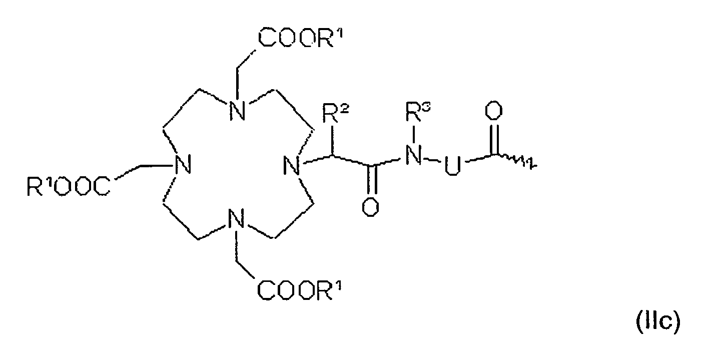
in der
R¹ ein Wasserstoffatom oder ein Metallionenäquivalent der Ordnungszahlen 23-29, 42-46 oder 58-70 bedeutet,
mit der Maßgabe, dass mindestens zwei R¹ für Metallionenäquivalente stehen
R² und R³ unabhängig voneinander Wasserstoff, C₁-C₇-Alkyl, Benzyl, Phenyl,-CH₂OH oder -CH₂OCH₃ darstellen und
U -C₆H₄-O-CH₂-ω-, -(CH₂)₁₋₅-ω, eine Phenylengruppe, -CH₂-NHCO-CH₂-CH(CH₂COOH)-C₆H₄-ω-, -C₆H₄-(OCH₂CH₂)₀₋₁-N(CH₂COOH)-CH₂-ω oder eine gegebenenfalls durch ein oder mehrere Sauerstoffatome, 1 bis 3-NHCO-, 1 bis 3 -CONH-gruppen unterbrochene und/oder mit 1 bis 3-(CH₂)₀₋₅COOH-Gruppen substituierte C₁-C₁₂-Alkylen- oder C₇-C₁₂-C₆H₄-O-Gruppe darstellt, wobei ω für die Bindungsstelle an -CO- steht,
oder
der allgemeinen Formel IIIc 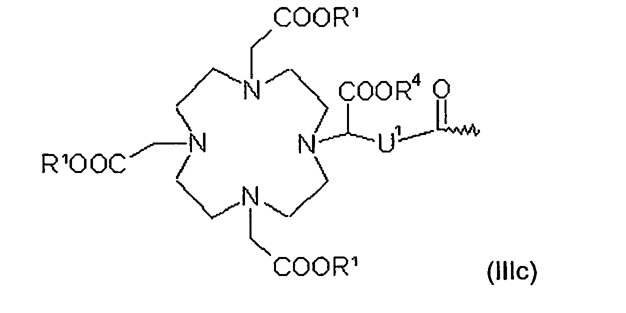 in der R¹ die oben genannte Bedeutung hat, R⁴ Wasserstoff oder ein unter R¹ genanntes Metallionenäquivalent darstellt und U¹ -C₆H₄-O-CH₂-ω- darstellt, wobei ω die Bindungsstelle an -CO- bedeutet
oder der allgemeinen Formel IVc 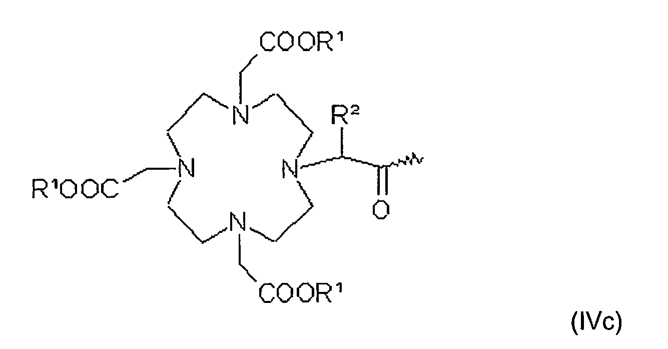 in der R¹ und R² die oben genannte Bedeutung haben
oder der allgemeinen Formel VcA oder VcB 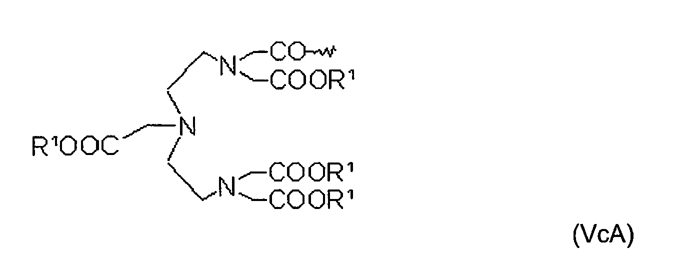 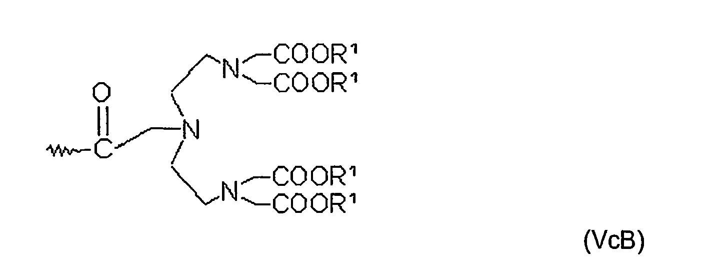 in der R¹ die oben genannte Bedeutung hat,
oder der allgemeinen Formel VIc 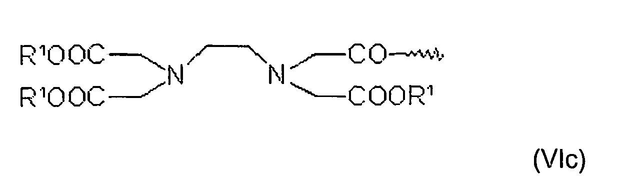 in der R¹ die oben genannte Bedeutung hat,
oder der allgemeinen Formel Vllc 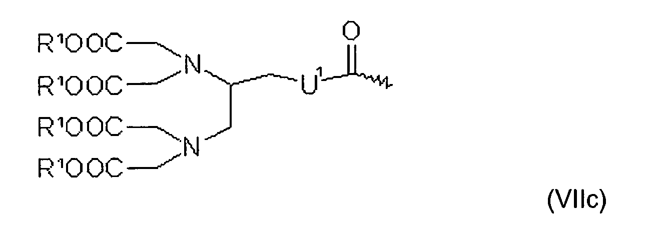 in der R¹ die oben genannte Bedeutung hat und
U¹ -C₆H₄-O-CH₂-ω- darstellt, wobei ω die Bindungsstelle an -CO- bedeutet
oder der allgemeinen Formel VIIIc 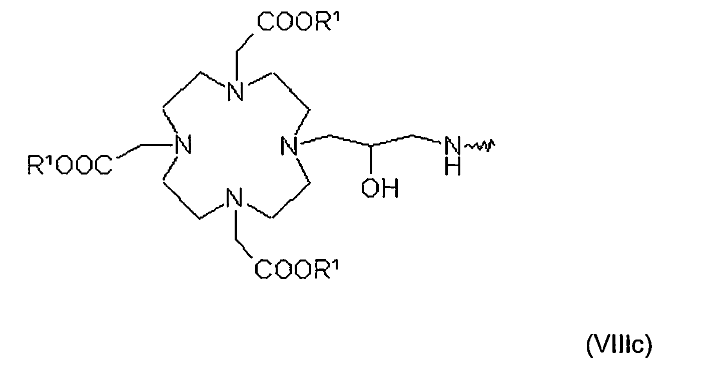 in der R¹ die oben genannte Bedeutung hat,
und im Rest K gegebenenfalls vorhandene freie Säuregruppen gegebenenfalls als Salze organischer und/oder anorganischer Basen oder Aminosäuren oder Aminosäureamide vorliegen können,
G für den Fall, dass K die Metallkomplexe IIc bis Vllc bedeutet, einen mindestens dreifach funktionalisierten Rest ausgewählt aus den nachfolgenden Resten a) bis j) darstellt 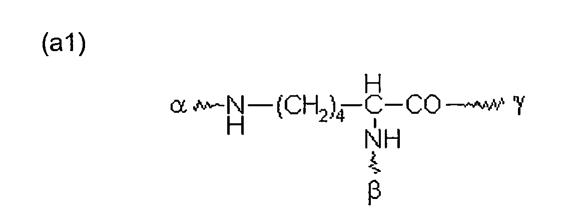 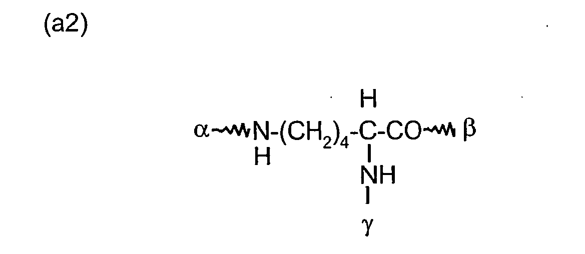 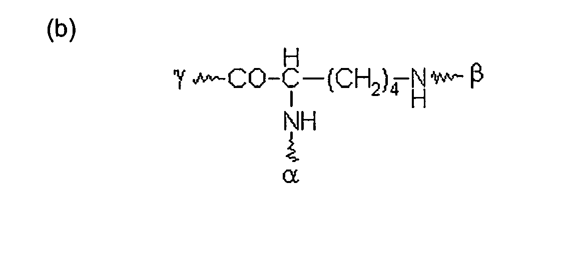 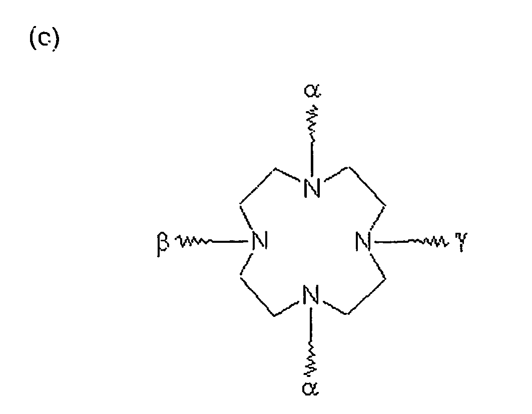 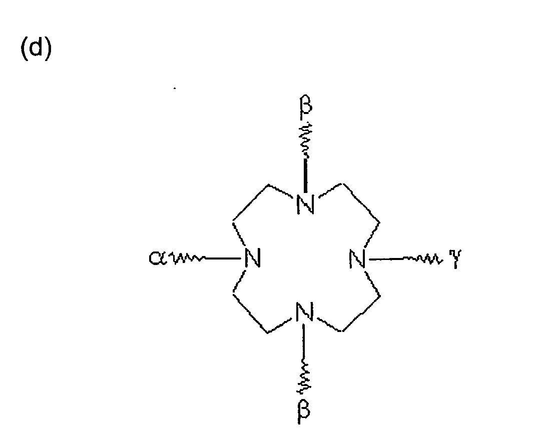 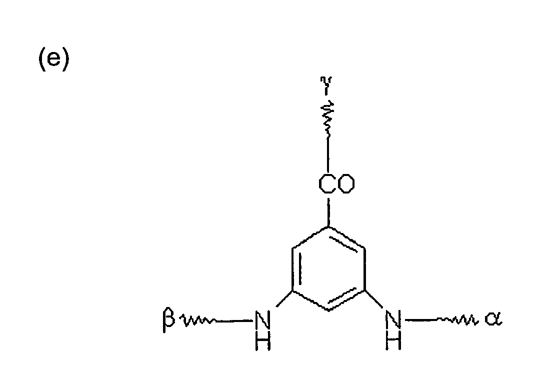 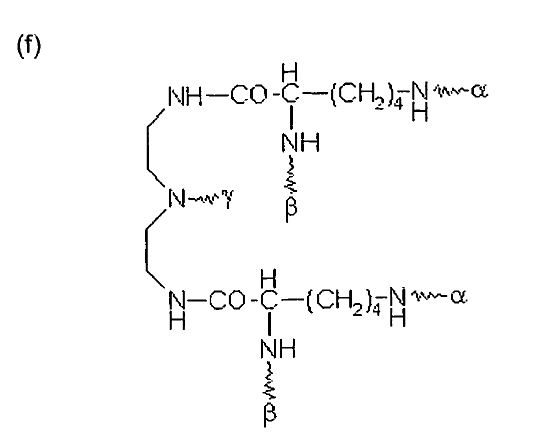 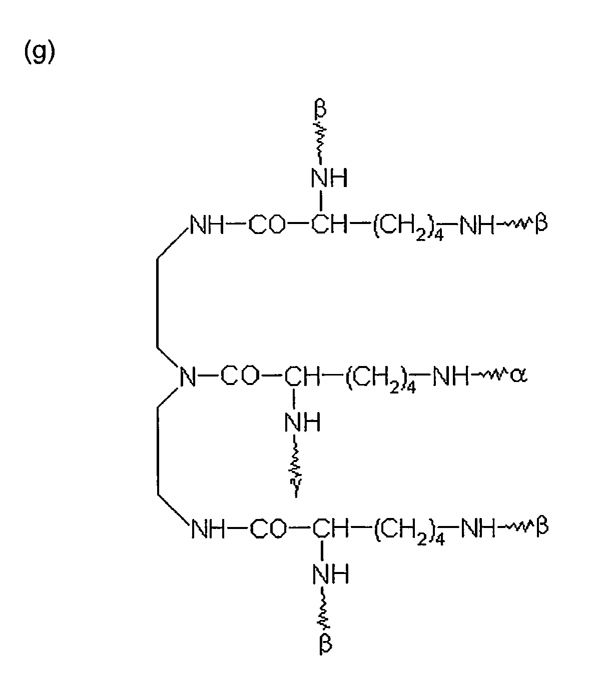 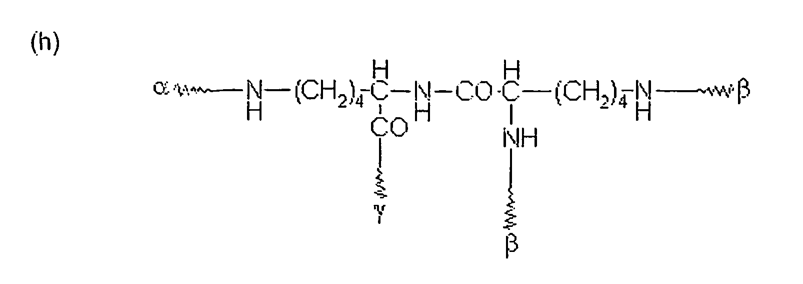 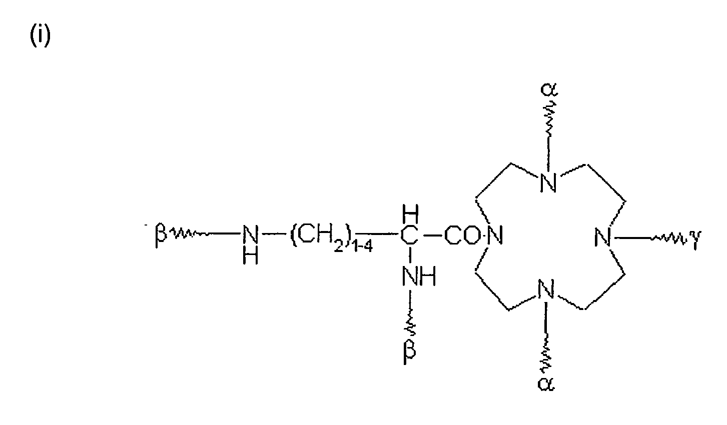 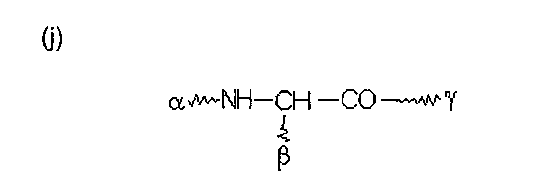 und
G für den Fall, dass K den Metallkomplex Vlllc bedeutet, einen mindestens dreifach funktionalisierten Rest ausgewählt aus k) oder l) darstellt, 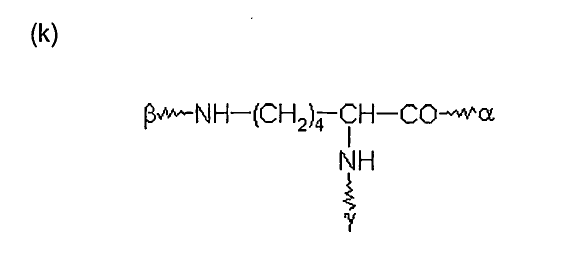 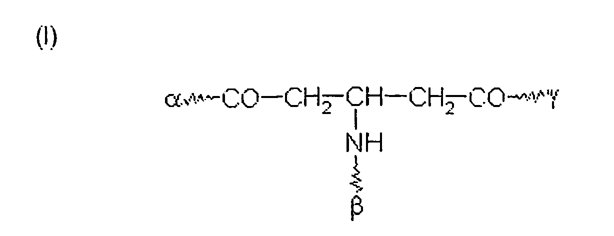 wobei α die Bindungsstelle von G an den Komplex K bedeutet, β die Bindungsstelle von G zum Rest Y ist und γ die Bindungsstelle von G zum Rest Z darstellt
Y -CH₂-, δ-(CH₂)₍₁₋₅₎CO-β, β-(CH₂)₍₁₋₅₎CO-δ, δ-CH₂-CHOH-CO-β oder δ-CH(CHOH-CH₂OH)-CHOH-CHOH-CO-β bedeutet, wobei δ die Bindungsstelle zum Zuckerrest R darstellt und β die Bindungsstelle zum Rest G ist
Z für 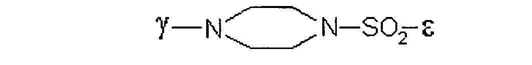 γ -COCH₂-N(C₂H₅)-SO₂-ε,
γ-COCH₂-O-(CH₂)₂-SO₂-ε, 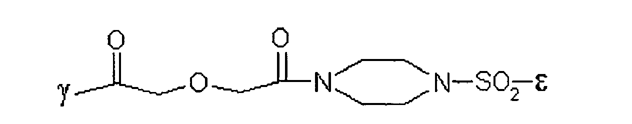 oder
γ - NHCH₂CH₂-O-CH₂CH₂-ε
steht, wobei γ die Bindungsstelle von Z zum Rest G darstellt und ε die Bindungsstelle von Z an den perfluorierten Rest R^{F} bedeutet
und
l¹, m¹ unabhängig voneinander die ganzen Zahlen 1 oder 2 bedeuten und
p¹ die ganzen Zahlen 1 bis 4 bedeutet,
eingesetzt werden.

31. Verwendung nach Anspruch 30,
**dadurch gekennzeichnet, dass**
die Verbindungen der allgemeinen Formel Ic eingesetzt werden, in der R einen Monosaccharidrest mit 5 bis 6 C-Atomen oder dessen Desoxy-Verbindung darstellt, vorzugsweise Glucose, Mannose oder Galactose.

32. Verwendung nach Anspruch 30,
**dadurch gekennzeichnet, dass**
die Verbindungen der allgemeinen Formel Ic eingesetzt werden, in der R² und R³ unabhängig voneinander Wasserstoff oder C₁-C₄-Alkyl bedeuten und/oder E in der Formel -CₙF₂ₙE ein Fluoratom bedeutet.

33. Verwendung nach Anspruch 30,
**dadurch gekennzeichnet, dass**
die Verbindungen der allgemeinen Formel Ic eingesetzt werden, in der G den Lysinrest (a) oder (b) darstellt.

34. Verwendung nach Anspruch 30,
**dadurch gekennzeichnet, dass**
die Verbindungen der allgemeinen Formel lc eingesetzt werden, in der Z 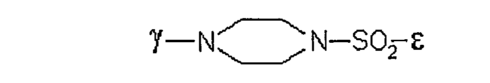 bedeutet, wobei γ die Bindungsstelle von Z zum Rest G darstellt und ε die Bindungsstelle von Z an den perfluorierten Rest R^{F} bedeutet und/oder Y δ-CH₂CO-β bedeutet, wobei δ die Bindungsstelle zum Zuckerrest R darstellt und β die Bindungsstelle zum Rest G darstellt.

35. Verwendung nach Anspruch 30,
**dadurch gekennzeichnet, dass**
die Verbindungen der allgemeinen Formel Ic eingesetzt werden, in der U im Metallkomplex K -CH₂- oder -C₆H₄-O-CH₂-ω darstellt, wobei ω für die Bindungsstelle an -CO- steht.

36. Verwendung nach Anspruch 30,
**dadurch gekennzeichnet, dass**
der Gadolinium-Komplex von 6-N-[1,4,7-Tris(carboxylatomethyl)- 1,4,7,10-tetraazacyclododecan-10-N-(pentanoyl-3-aza-4-oxo-5-methyl-5-yl)]-2-N-[1-O-α-D-carbonylmethyl-mannopyranose]-L-lysin-[1-(4-perfluoroctylsulfonyl)-piperazin]-amid eingesetzt wird.

37. Verwendung nach einem der Ansprüche 1 bis 7,
**dadurch gekennzeichnet, dass**
als perfluoralkylhaltige Metallkomplexe die Verbindungen mit polaren Resten der allgemeinen Formel Id 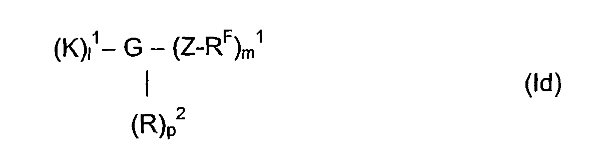 in der
R^{F} eine perfluorierte, geradkettige oder verzweigte Kohlenstoffkette mit der Formel -CₙF₂ₙE ist, in der E ein endständiges Fluor-, Chlor-, Brom-, Jod-oder Wasserstoffatom darstellt und n für die Zahlen 4-30 steht,
K für einen Metallkomplex der allgemeinen Formel IId steht, 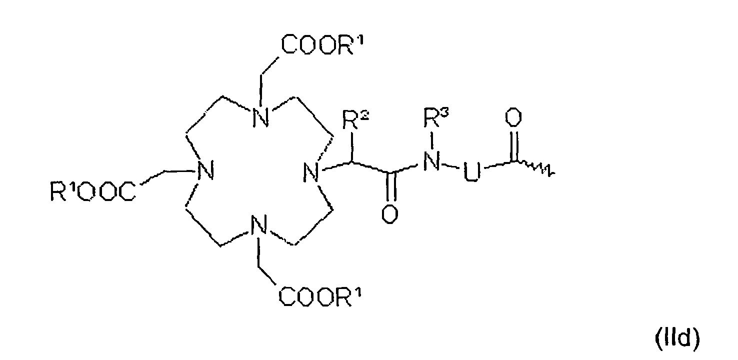 in der
R¹ ein Wasserstoffatom oder ein Metallionenäquivalent der Ordnungszahlen 23-29, 42-46 oder 58-70 bedeutet,
mit der Maßgabe, dass mindestens zwei R¹ für Metall-ionenäquivalente stehen
R² und R³ unabhängig voneinander Wasserstoff, C₁-C₇-Alkyl, Benzyl, Phenyl,-CH₂OH oder -CH₂OCH₃ darstellen und
U -C₆H₄-O-CH₂-ω-, -(CH₂)₁₋₅-ω, eine Phenylengruppe, -CH₂-NHCO-CH₂-CH(CH₂COOH)-C₆H₄-ω-, -C₆H₄-(OCH₂CH₂)₀₋₁-N(CH₂COOH)-CH₂-ω oder eine gegebenenfalls durch ein oder mehrere Sauerstoffatome, 1 bis 3-NHCO-, 1 bis 3 -CONH-gruppen unterbrochene und/oder mit 1 bis 3
-(CH₂)₀₋₅COOH-Gruppen substituierte C₁-C₁₂-Alkylen- oder C₇-C₁₂-C₆H₄-0-Gruppe darstellt, wobei ω für die Bindungsstelle an -CO-steht,
oder
der allgemeinen Formel IIId 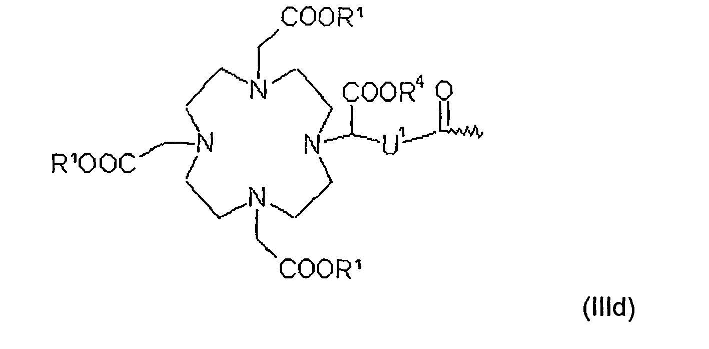 in der R¹ die oben genannte Bedeutung hat, R⁴ Wasserstoff oder ein unter R¹ genanntes Metattionenäquivatent darstellt und U¹ -C₆H₄-O-CH₂-ω- darstellt, wobei ω die Bindungsstelle an -CO- bedeutet
oder der allgemeinen Formel IVd 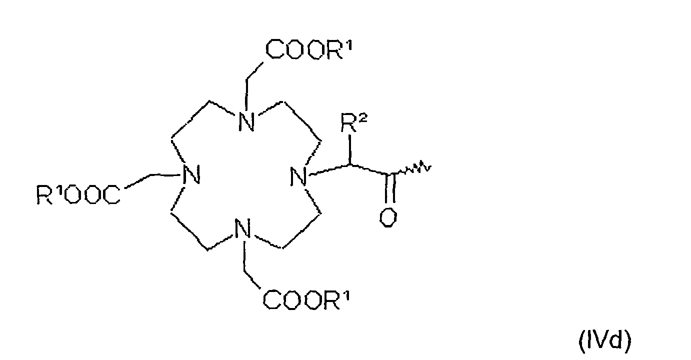 in der R¹ und R² die oben genannte Bedeutung haben
oder der allgemeinen Formel VdA oder VdB 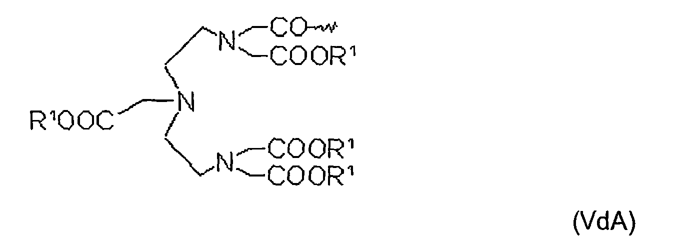 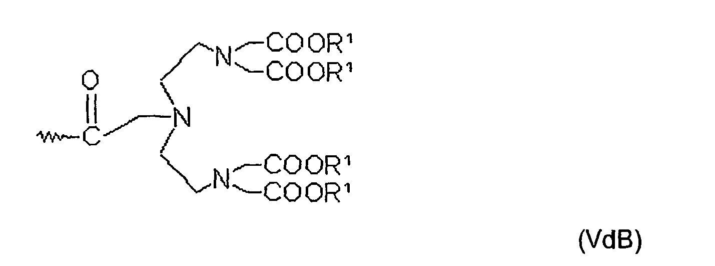 in der R¹ die oben genannte Bedeutung hat,
oder der allgemeinen Formel VId 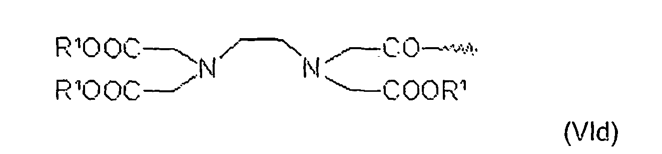 in der R¹ die oben genannte Bedeutung hat,
oder der allgemeinen Formel Vlld 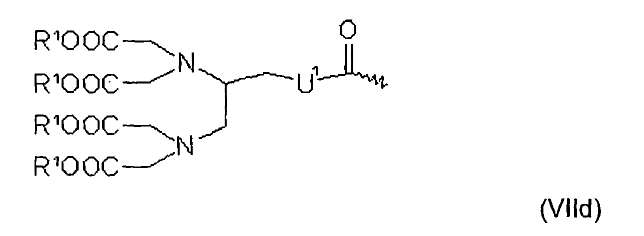 in der R¹ die oben genannte Bedeutung hat und
U¹ -C₆H₄-O-CH₂-ω- darstellt, wobei ω die Bindungsstelle an -CO- bedeutet
und im Rest K gegebenenfalls vorhandene freie Säuregruppen gegebenenfalls als Salze organischer und/oder anorganischer Basen oder Aminosäuren oder Aminosäureamide vorliegen können,
G einen mindestens dreifach funktionalisierten Rest ausgewählt aus den nachfolgenden Resten a) bis i) darstellt 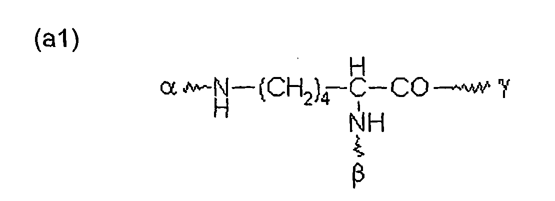 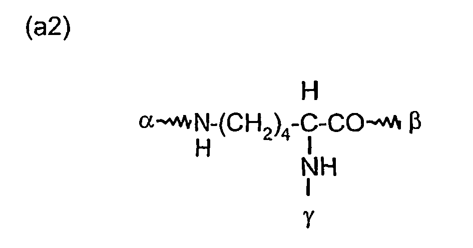 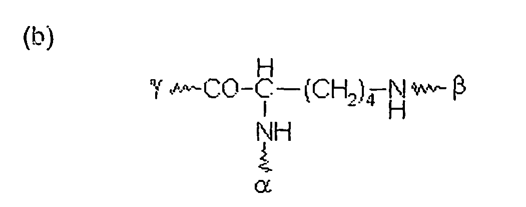 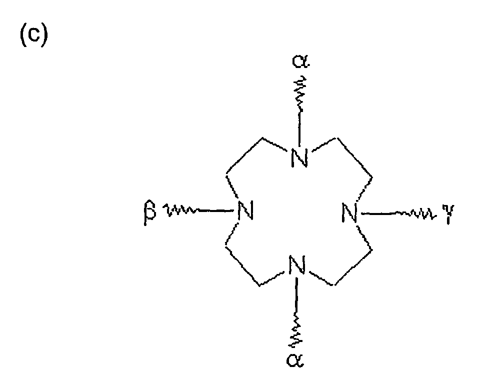 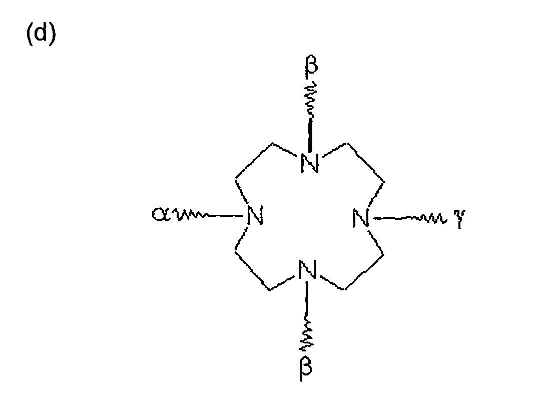 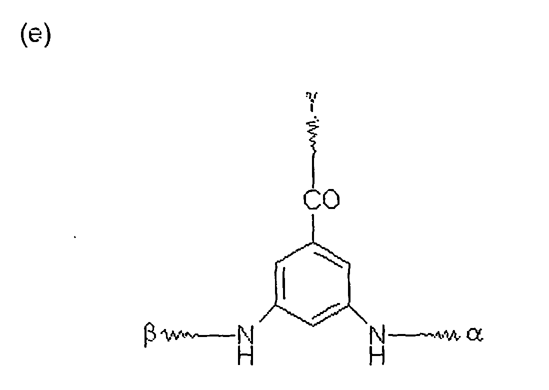 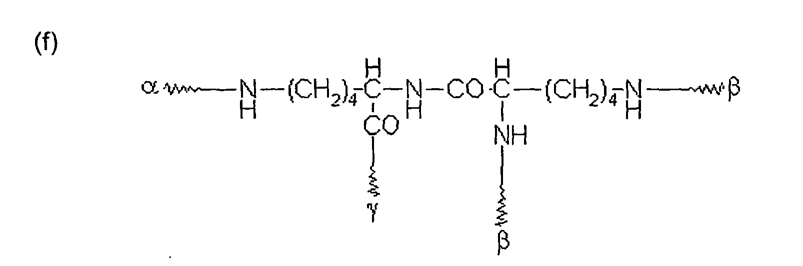 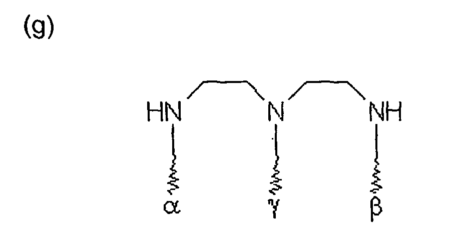 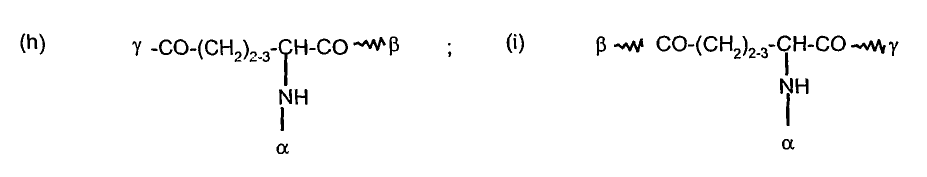
wobei α die Bindungsstelle von G an den Komplex K bedeutet, β die Bindungsstelle von G zum Rest R ist und γ die Bindungsstelle von G zum Rest Z darstellt
Z für 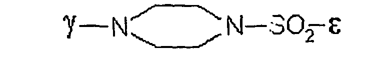 γ -C(O)CH₂O(CH₂)₂-ε,
steht, wobei γ die Bindungsstelle von Z zum Rest G darstellt und ε die Bindungsstelle von Z an den perfluorierten Rest R_{f} bedeutet
R einen polaren Rest ausgewählt aus den Komplexen K der allgemeinen Formeln IId bis VIId darstellt, wobei R¹ hier ein Wasserstoffatom oder ein Metallionenäquivalent der Ordnungszahlen 20, 23-29, 42-46 oder 58-70 bedeutet,
und die Reste R², R³, R⁴, U und U¹ die oben angegebene Bedeutung aufweisen
oder
den Folsäurerest
oder
eine über -CO- , SO₂- oder eine direkte Bindung an den Rest G gebundene Kohlenstoffkette mit 2-30 C-Atomen bedeutet, geradlinig oder verzweigt, gesättigt oder ungesättigt,
gegebenenfalls unterbrochen durch 1-10 Sauerstoffatome, 1-5 - NHCO-Gruppen, 1-5 -CONH-Gruppen, 1-2 Schwefelatome, 1-5 -NH-Gruppen oder 1-2 Phenylengruppen, die gegebenenfalls mit 1-2 OH-Gruppen, 1-2 NH₂-Gruppen, 1-2 -COOH-Gruppen, oder 1-2 -SO₃H-Gruppen substituiert sein können
oder
gegebenenfalls substituiert mit 1-8 OH-Gruppen, 1-5 -COOH-Gruppen, 1-2 SO₃H-Gruppen, 1-5 NH₂-Gruppen, 1-5 C₁-C₄-Alkoxygruppen,
und
l¹, m¹, p² unabhängig voneinander die ganzen Zahlen 1 oder 2 bedeuten.
eingesetzt werden.

38. Verwendung nach Anspruch 37,
**dadurch gekennzeichnet, dass**
die Verbindungen der allgemeinen Formel ld eingesetzt werden, in der K für einen Metallkomplex der allgemeinen Formel lld, llld, VdB oder Vlld steht.

39. Verwendung nach Anspruch 37,
**dadurch gekennzeichnet, dass**
die Verbindungen der allgemeinen Formel Id eingesetzt werden, in der der polare Rest R die Bedeutung des Komplexes K hat, vorzugsweise der Komplexe K der allgemeinen Formeln IId, IIId, VdA oder Vlld.

40. Verwendung nach Anspruch 37,
**dadurch gekennzeichnet, dass**
die Verbindungen der allgemeinen Formel Id eingesetzt werden, in der der polare Rest R folgende Bedeutungen hat:
-C(O)CH₂CH₂SO₃H
-C(O)CH₂OCH₂CH₂OCH₂CH₂OH
-C(O)CH₂OCH₂CH₂OH
-C(O)CH₂OCH₂CH(OH)CH₂OH
-C(O)CH₂NH-C(O)CH₂COOH
-C(O)CH₂CH(OH)CH₂OH
-C(O)CH₂OCH₂COOH
-SO₂CH₂CH₂COOH
-C(O)-C₆H₃-(m-COOH)₂
-C(O)CH₂O(CH₂)₂-C₆H₃-(m-COOH)₂
-C(O)CH₂O-C₆H₄-m-SO₃H
-C(O)CH₂NHC(O)CH₂NHC(O)CH₂OCH₂COOH
-C(O)CH₂OCH₂CH₂OCH₂COOH
-C(O)CH₂OCH₂CH(OH)CH₂O-CH₂CH₂OH
-C(O)CH₂OCH₂CH(OH)CH₂OCH₂-CH(OH)-CH₂OH
-C(O)CH₂SO₃H
-C(O)CH₂CH₂COOH
-C(O)CH(OH)CH(OH)CH₂OH
-C(O)CH₂O[(CH₂)₂O]₁₋₉-CH₃
-C(O)CH₂O[(CH₂)₂O]₁₋₉-H
-C(O)CH₂OCH(CH₂OH)₂
-C(O)CH₂OCH(CH₂OCH₂COOH)₂
-C(O)-C₆H₃-(m-OCH₂COOH)₂
-CO-CH₂O-(CH₂)₂O(CH₂)₂O-(CH₂)₂O(CH₂)₂OCH₃
vorzugsweise -C(O)CH₂O[(CH₂)₂O]₄-CH₃.

41. Verwendung nach Anspruch 37,
**dadurch gekennzeichnet, dass**
die Verbindungen der allgemeinen Formel Id eingesetzt werden, in der der polare Rest R der Folsäurerest ist.

42. Verwendung nach Anspruch 37,
**dadurch gekennzeichnet, dass**
die Verbindungen der allgemeinen Formel Id eingesetzt werden, in der G den Lysinrest (a) oder (b) darstellt.

43. Verwendung nach Anspruch 37,
**dadurch gekennzeichnet, dass**
die Verbindungen der allgemeinen Formel Id eingesetzt werden, in der U im Metallkomplex K die Gruppe -CH₂- oder -C₆H₄-O-CH₂-ω darstellt, wobei ω für die Bindungsstelle an -CO- steht.

44. Verwendung nach einem der Ansprüche 37-43,
**dadurch gekennzeichnet, dass**
der Gadolinium-Komplex von 2,6-N,N'-Bis[1,4,7-tris(carboxylatomethyl)-1,4,7,10-tetraazacyclododecan-10-(pentanoyl-3-aza-4-oxo-5-methyl-5-yl)]-lysin-[1-(4-perfluoroctylsulfonyl-piperazin]-amid eingesetzt wird.

45. Verwendung nach einem der Ansprüche 1-7,
**dadurch gekennzeichnet, dass**
als perfluoralkylhaltige Metallkomplexe galenische Formulierungen eingesetzt werden, die paramagnetische perfluoralkylhaltige Metallkomplexe der allgemeinen Formeln I, Ia, Ib, Ic und/oder Id und diamagnetische perfluoralkylhaltige Substanzen beinhalten, vorzugsweise gelöst in einem wässrigen Lösungsmittel.

46. Verwendung nach Anspruch 45,
**dadurch gekennzeichnet, dass**
als diamagnetische perfluoralkyhaltige Substanzen solche der allgemeinen Formel XX verwendet werden:
R^{F}-L²-B² (XX)
worin R^{F} einen geradkettigen oder verzweigten Perfluoralkylrest mit 4 bis 30 Kohlenstoffatomen darstellt, L² für einen Linker und B² für eine hydrophile Gruppe steht.

47. Verwendung nach Anspruch 46,
**dadurch gekennzeichnet, dass**
der Linker L² eine direkte Bindung, eine -SO₂-Gruppe oder eine geradkettige oder verzweigte Kohlenstoffkette mit bis zu 20 Kohlenstoffatomen ist, welche mit einer oder mehreren
-OH, -COO⁻, -SO₃-Gruppen substituiert sein kann und/oder gegebenenfalls eine oder mehrere -O-, -S-, -CO-, -CONH-, -NHCO-, - CONR⁹-, -NR⁹CO-, -SO₂-, -PO₄⁻-, -NH-, -NR⁹-Gruppen, einen Arylring oder ein Piperazin enthält, wobei R⁹ für einen C₁- bis C₂₀-Alkylrest steht, welcher wiederum ein oder mehrere O-Atome enthalten kann und/oder mit -COO⁻ oder SO₃-Gruppen substituiert sein kann.

48. Verwendung nach Anspruch 46,
**dadurch gekennzeichnet, dass**
die hydrophile Gruppe B² ein Mono- oder Disaccharid, eine oder mehrere benachbarte -COO⁻ oder -SO₃⁻-Gruppen, eine Dicarbonsäure, eine Isophthalsäure, eine Picolinsäure, eine Benzolsulfonsäure, eine Tetrahydropyrandicarbonsäure, eine 2,6-Pyridindicarbonsäure, ein quartäres Ammoniumion, eine Aminopolycarbonsäure, eine Aminodipolyethylenglycolsulfonsäure, eine Aminopolyethylenglycolgruppe, eine SO₂-(CH₂)₂-OH-Gruppe, eine Polyhydroxyalkylkette mit mindestens zwei Hydroxylgruppen oder eine oder mehrere Polyethylenglycolketten mit mindestens zwei Glycoleinheiten ist, wobei die Polyethylenglycolketten durch eine -OH oder -OCH₃-Gruppe terminiert sind.

49. Verwendung nach Anspruch 45,
**dadurch gekennzeichnet, dass**
als diamagnetische perfluoralkylhaltige Substanzen Konjugate aus α-, β-, oder γ-Cyclodextrin und Verbindungen der allgemeinen Formel XXII eingesetzt werden:
A²-L³-R^{F} (XXII)
worin A² für ein Adamantan-, Biphenyl- oder Anthracenmolekül, L³ für einen Linker und R^{F} für einen geradkettigen oder verzweigten Perfluoralkylrest mit 4 bis 30 Kohlenstoffatomen steht; und wobei der Linker L³ eine geradkettige Kohlenwasserstoffkette mit 1 bis 20 Kohlenstoffatomen ist, welche durch ein oder mehrere Sauerstoffatome, ein oder mehrere CO-, SO₂-, CONH-, NHCO-, CONR¹⁰-, NR¹⁰CO-, NH-, NR¹⁰-Gruppen oder ein Piperazin unterbrochen sein kann, wobei R¹⁰ ein C₁-C₅-Alkylrest ist.

50. Verwendung nach Anspruch 45,
**dadurch gekennzeichnet, dass**
als diamagnetische perfluoralkylhaltige Substanzen solche der allgemeinen Formel XXI eingesetzt werden:
R^{F}-X¹ (XXI)
worin R^{F} einen geradkettigen oder verzweigten Perfluoralkylrest mit 4 bis 30 Kohlenstoffatomen darstellt und X¹ ein Rest ausgewählt aus der Gruppe der folgenden Reste ist (n ist dabei eine Zahl zwischen 1 und 10):

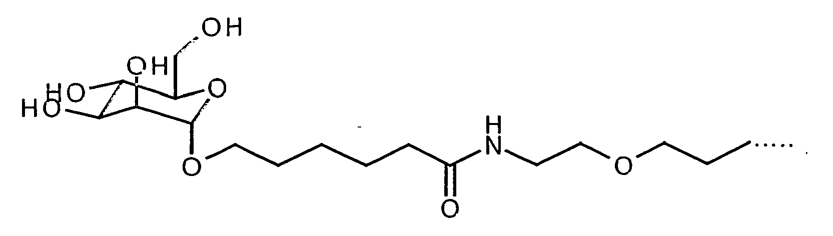

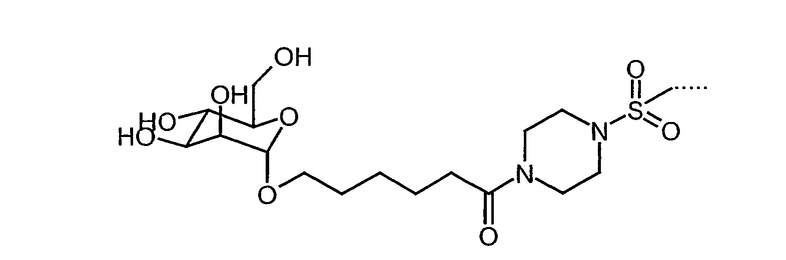

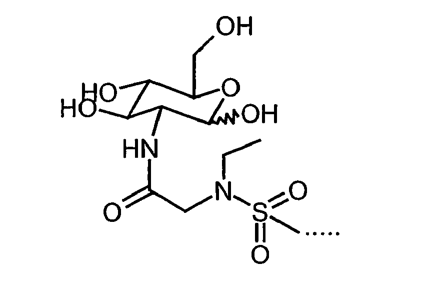

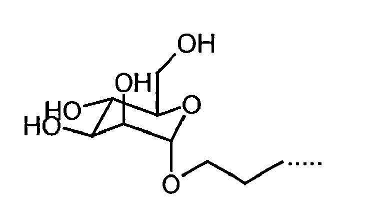

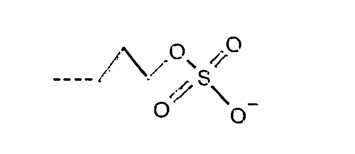

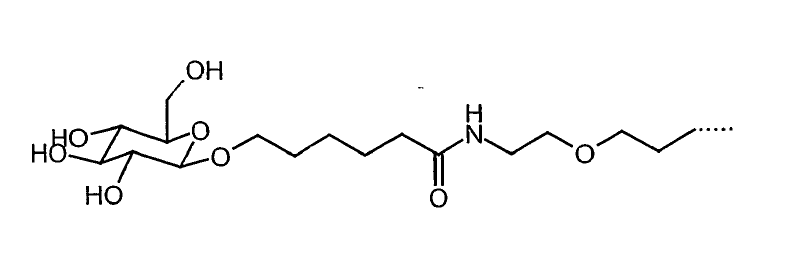

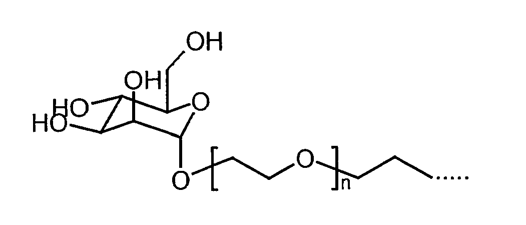

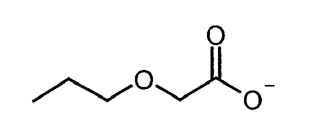

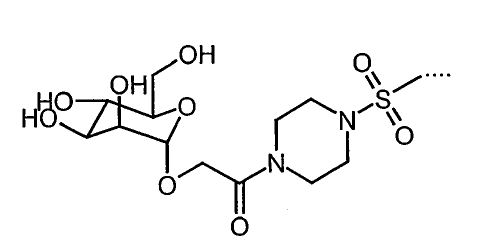

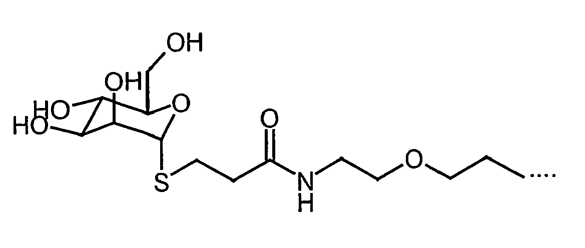

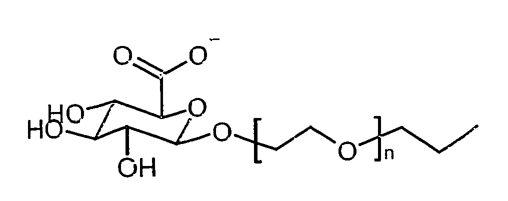

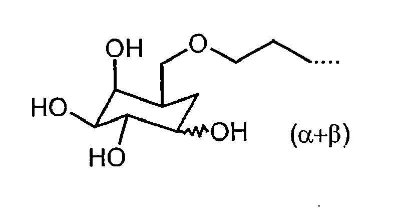

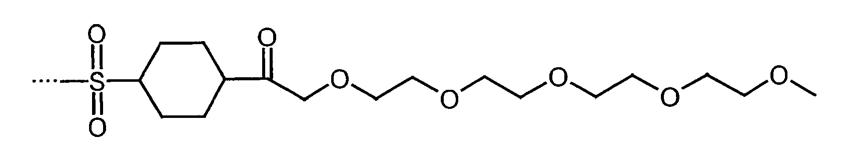

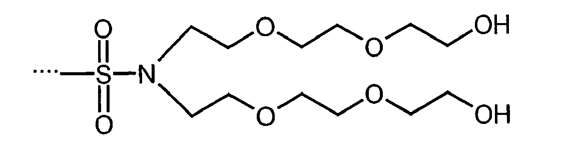

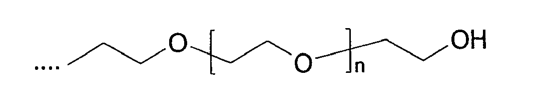

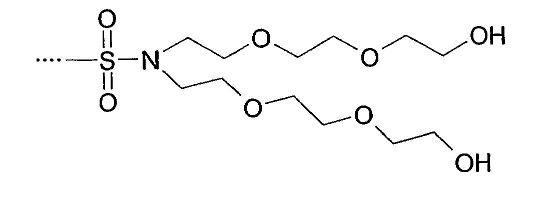

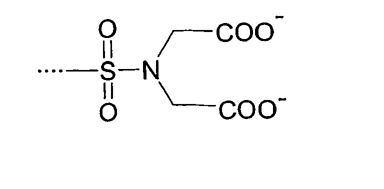

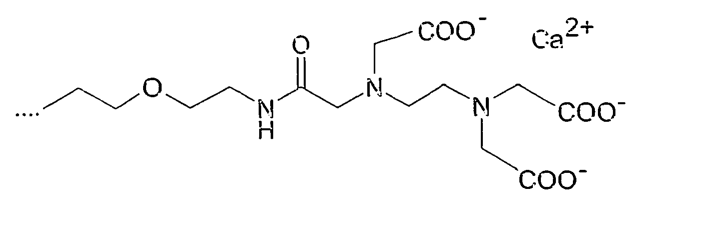

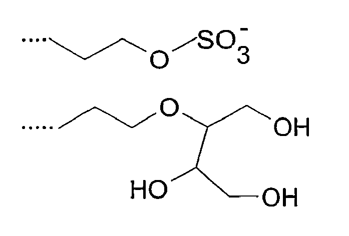

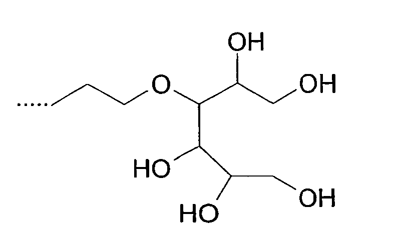

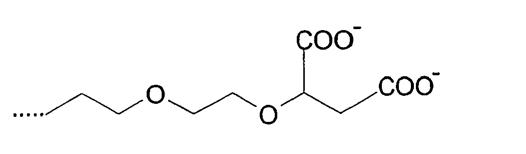

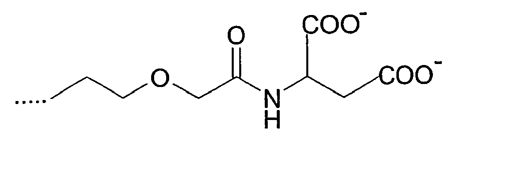

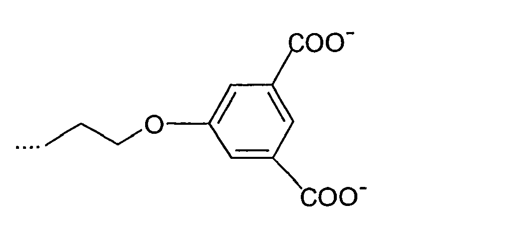

## Claims

1. Use of perfluoroalkyl-containing metal complexes that have a critical micelle formation concentration <10⁻³ mol/l, a hydrodynamic micelle diameter (2 Rh)>1 nm and a proton relaxivity in plasma (R¹) (at 40°C and a field strength of 0.47 tesla) >10 l/mmol·s in the manufacture of a contrast medium in MR imaging for visualization of intravascular thrombi.

2. Use according to Claim 1, **characterized in that** the metal complexes are used as MRI contrast media for visualization of venous thrombi.

3. Use according to Claim 1 or 2, **characterized in that** the metal complexes are used as MRI contrast media for visualization of arterial thrombi.

4. Use according to one of Claims 1 to 3, **characterized**
**in that** the metal complexes are used as MRI contrast media for early determination of a thrombotic occlusive vascular disease.

5. Use according to Claim 1, **characterized in that** metal complexes whose micelle formation concentration is <10⁻⁴ mol/l are used.

6. Use according to Claim 1, **characterized in that** metal complexes whose hydrodynamic micelle diameter is ≥3 nm, preferably >4 nm, are used.

7. Use according to Claim 1, **characterized in that** metal complexes that have a proton relaxivity in plasma of >13 l/mmol·s, preferably >15 l/mmol·s, are used.

8. Use according to one of Claims 1 to 7, **characterized**
**in that** as perfluoroalkyl-containing metal complexes, the compounds of general formula I
R^{F}-L-K I
in which R^{F} is a perfluorinated, straight-chain or branched carbon chain with formula -CₙF₂ₙE, in which E represents a terminal fluorine, chlorine, bromine, iodine or hydrogen atom and n stands for numbers 4-30, L means a direct bond, a methylene group, an -NHCO group, a group 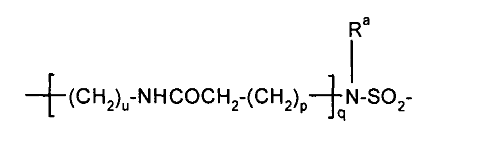 where p means the numbers 0 to 10, and q and n, independently of one another, mean numbers 0 or 1, and R^{a} is a hydrogen atom, a methyl group, a benzyl group, a phenyl group, a -CH₂-OH group, a CH₂OCH₃ group, a -CH₂-CO₂H group or a C₂-C₁₅ chain, which optionally is interrupted by 1 to 3 oxygen atoms, 1 to 2>CO groups or an optionally substituted aryl group and/or is substituted with 1 to 4 hydroxyl groups, 1 to 2 C₁-C₄ alkoxy groups, 1 to 2 carboxy groups, a group -SO₃H-, or is a straight-chain, branched, saturated or unsaturated C₂-C₃₀ carbon chain, which optionally contains 1 to 10 oxygen atoms, 1 to 3 -NR^{a} groups, 1 to 2 sulphur atoms, a piperazine, a -CONR^{a} group, one to six -NR^{a}CO groups, an -SO₂ group, an -NR^{a}-CO₂ group, 1 to 2 CO groups, a group 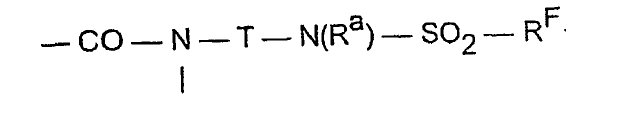 or 1 to 2 optionally substituted aryls and/or is interrupted by these groups and/or is optionally substituted with 1 to 3 -OR^{a} groups, 1 to 2 oxo groups, 1 to 2 -NH-COR^{a} groups, 1 to 2 -CONHR^{a} groups, 1 to 2 -(CH₂)ₚ-CO₂H groups, 1 to 2 groups -(CH₂)ₚ-(O)_{q}-CH₂CH₂-R^{F},
where R^{a}, R^{F} and p and q have the above-indicated meanings, and T means a C₂-C₁₀ chain, which optionally is interrupted by 1 to 2 oxygen atoms or 1 to 2 -NHCO groups, K stands for a complexing agent or metal complex or their salts of organic and/or inorganic bases or amino acids or amino acid amides, specifically for a complexing agent or complex of general formula II 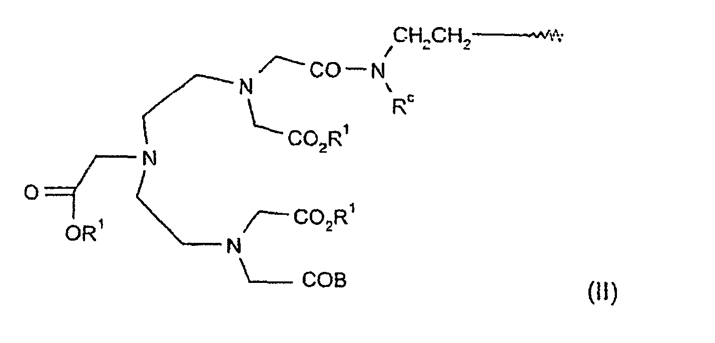 in which R^{c}, R¹ and B are independent of one another, and R^{c} has the meaning of R^{a} or means -(CH₂)ₘ-L-R^{F}, where m is 0, 1 or 2, and L and R^{F} have the above-mentioned meaning, R¹, independently of one another, mean a hydrogen atom or a metal ion equivalent of atomic numbers 22-29, 42-46 or 58-70, B means -OR¹ or 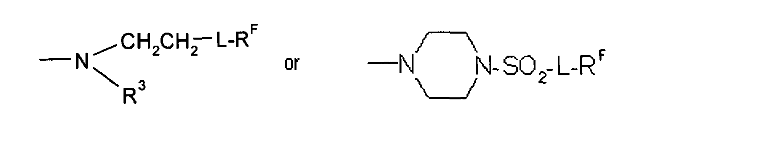 where R¹, L, R^{F} and R^{c} have the above-mentioned meanings, or K stands for a complexing agent or complex of general formula III 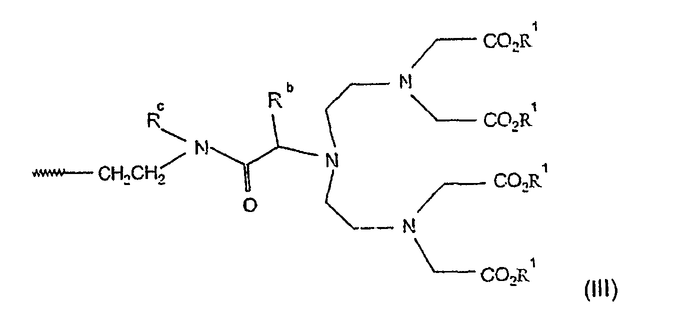 in which R^{c} and R¹ have the above-mentioned meanings, R^{b} has the meaning of R^{a}, and or K stands for a complexing agent or complex of general formula IV 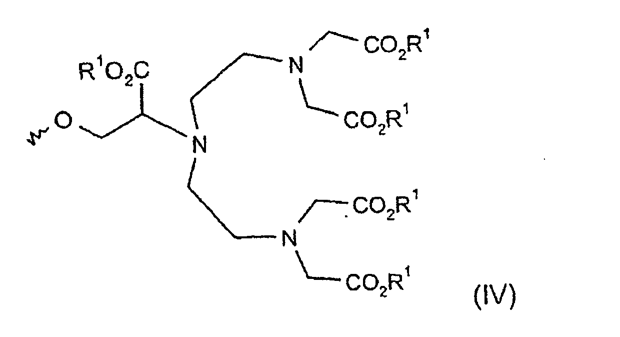 in which R¹ has the above-mentioned meaning or K stands for a complexing agent or complex of general formula V 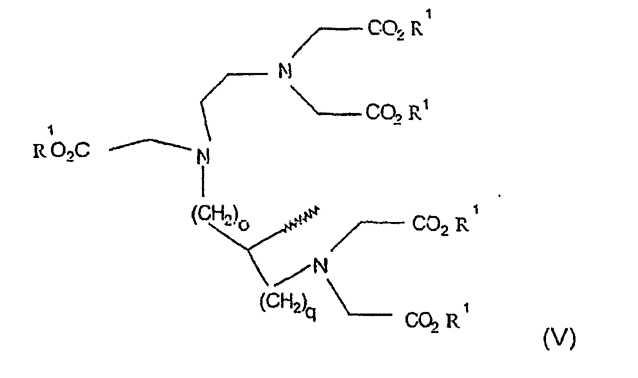 in which R¹ has the above-mentioned meaning, and o and q stand for numbers 0 or 1, and yields the sum o+q=1, or K stands for a complexing agent or complex of general formula VI 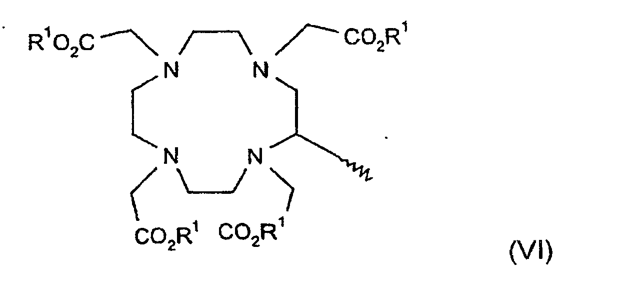 in which R¹ has the above-mentioned meaning or K stands for a complexing agent or complex of general formula VII 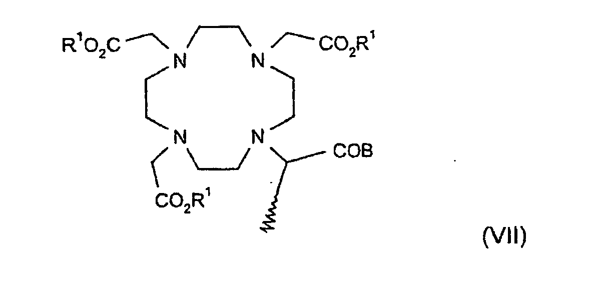 in which R¹ and B have the above-mentioned meanings or K stands for a complexing agent or complex of general formula VIII 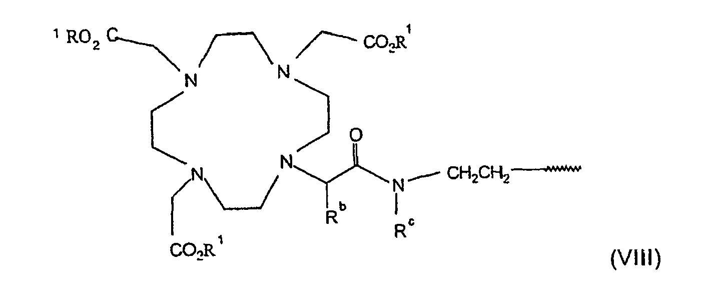 in which R^{c} and R¹ have the above-mentioned meanings, and R^{b} has the above-mentioned meaning of R^{a} or K stands for a complexing agent or complex of general formula IX 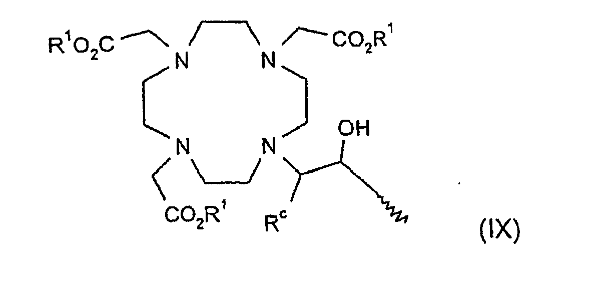 in which R^{c} and R¹ have the above-mentioned meanings, or K stands for a complexing agent or complex of general formula X 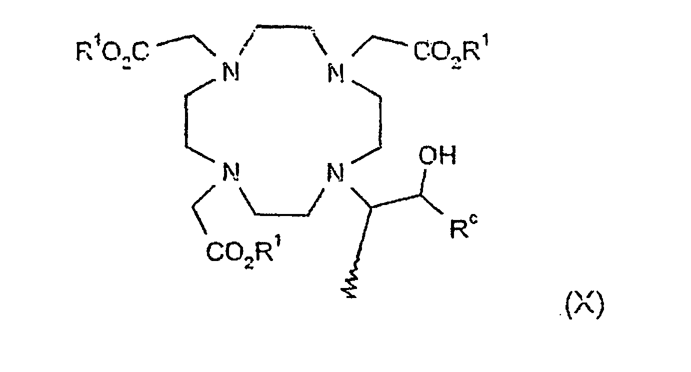 in which R^{c} and R¹ have the above-mentioned meanings, or K stands for a complexing agent or complex of general formula XI 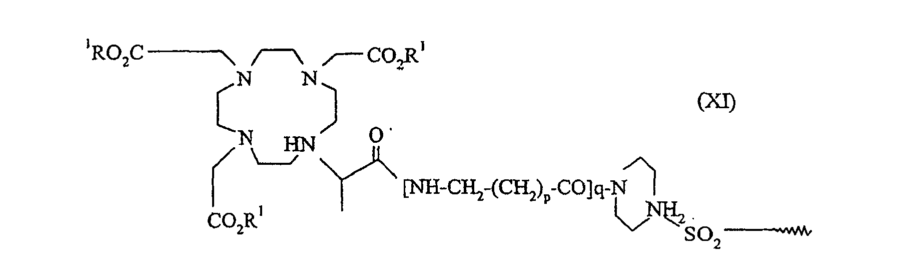 in which R¹, p and q have the above-mentioned meanings, and R^{b} has the meaning of R^{a}, or K stands for a complexing agent or complex of general formula XII 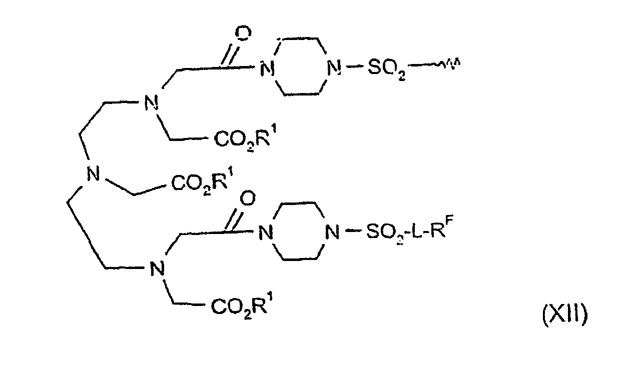 in which L, R^{F} and R¹ have the above-mentioned meanings, or K stands for a complexing agent or complex of general formula XIII 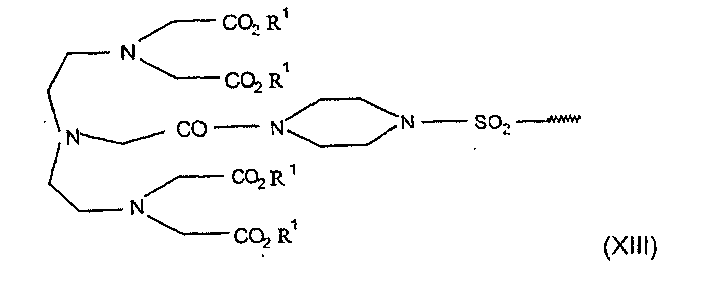 in which R¹ has the above-mentioned meaning, are used.

9. Use according to Claim 8, **characterized in that** the compounds of general formula I, in which L stands for
α-CH₂-β
α-CH₂CH₂-β
α-(CH₂)ₛ-β s = 3 - 15
α-CH₂-O-CH₂CH₂-β
α-CH₂-(O-CH₂-CH₂-)ₜ-β t = 2 - 6
α-CH₂-NH-CO-β
α-CH₂-NH-CO-CH₂-N(CH₂COOH)-SO₂-β
α-CH₂-NH-CO-CH₂-N(C₂H₅)-SO₂-β
α-CH₂-NH-CO-CH₂-N(C₁₀H₂₁)-SO₂-β
α-CH₂-NH-CO-CH₂-N(C₆H₁₃)-SO₂-β
α-CH₂-NH-CO-(CH₂)₁₀-N(C₂H₅)-SO₂-β
α-CH₂-NH-CO-CH₂-N(-CH₂-C₆H₅)-SO₂-β
α-CH₂-NH-CO-CH₂-N(-CH₂-CH₂-OH)SO₂-β
α-CH₂-NHCO-(CH₂)₁₀-S-CH₂CH₂-β
α-CH₂NHCOCH₂-O-CH₂CH₂-β
α-CH₂NHCO(CH₂)₁₀-O-CH₂CH₂-β
α-CH₂-C₆H₄-O-CH₂CH₂-β
α-CH₂-O-CH₂-C(CH₂-OCH₂CH₂-C₆F₁₃)₂-CH₂-OCH₂-CH₂-β 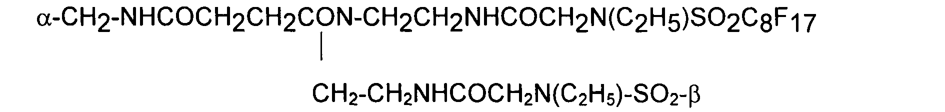 α -CH₂-O-CH₂-CH(OC₁₀H₂₁)-CH₂-O-CH₂CH₂-β
α-(CH₂NHCO)₄-CH₂O-CH₂CH₂-β
α-(CH₂NHCO)₃-CH₂O-CH₂CH₂-β
α-CH₂-OCH₂C(CH₂OH)₂-CH₂-O-CH₂CH₂-β 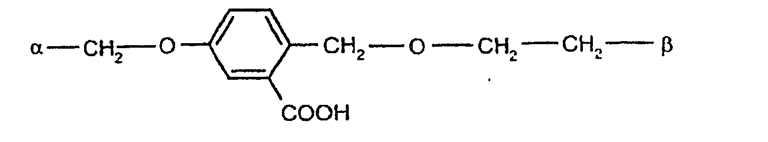 α -CH₂NHCOCH₂N(C₆H₅)-SO₂-β
α-NHCO-CH₂-CH₂-β
α-NHCO-CH₂-O-CH₂CH₂-β
α-NH-CO-β
α-NH-CO-CH₂-N(CH₂COOH)-SO₂-β
α-NH-CO-CH₂-N(C₂H₅)-SO₂-β
α-NH-CO-CH₂-N(C₁₀H₂₁)-SO₂-β
α-NH-CO-CH₂-N(C₆H₁₃)-SO₂-β
α-NH-CO-(CH₂)₁₀-N(C₂H₅)-SO₂-β
α-NH-CO-CH₂-N(-CH₂-C₆H₅)-SO₂-β
α-NH-CO-CH₂-N(-CH₂-CH₂-OH)SO₂-β
α-NH-CO-CH₂-β
α-CH₂-O-C₆H₄-O-CH₂-CH₂-β
α-CH₂-C₆H₄-O-CH₂-CH₂-β
α-N(C₂H₅)-SO₂-β
α-N(C₆H₅)-SO₂-β
α-N(C₁₀H₂₁)-SO₂-β
α-N(C₆H₁₃)-SO₂-β
α-N(C₂H₄OH)-SO₂-β
α-N(CH₂COOH)-SO₂-β
α-N(CH₂C₆H₅)-SO₂-β
α-N-[CH(CH₂OH)₂]-SO₂-β
α-N-[CH(CH₂OH)CH(CH₂OH)]-SO₂-β
and in which α represents the binding site to the complexing agent or metal complex K, and β represents the binding site to the fluorine radical, are used.

10. Use according to Claim 8 or 9, **characterized in that** the compounds of formula I in which n in formula - CₙF₂ₙE stands for numbers 4-15 and/or E in this formula means a fluorine atom are used.

11. Use according to one of Claims 8 to 10, **characterized in that** the following compounds are used:
- Gadolinium complex of 10-[1-methyl-2-oxo-3-aza-5-oxo-{4-perfluorooctylsulphonyl-piperazin-1-yl}-pentyl]-1,4,7-tris(carboxymethyl-)-1,4,7,10-tetraazacyclododecane,
- Gadolinium complex of 10-[2-hydroxy-4-aza-5-oxo-7-oxa-10,10,11,11,12,12,13,13,14,14,15,15,16,16,17,17-heptadecafluoroheptadecyl]-1,4,7-tris(carboxymethyl)-1,4,7,10-tetraazacyclododecane,
- Gadolinium complex of 10-[2-hydroxy-4-aza-5,9-dioxo-9-{4-perfluorooctyl)-piperazin-1-yl}-nonyl]-1,4,7-tris(carboxymethyl)-1,4,7,10-tetraazacyclododecane,
- Gadolinium complex of 10-[2-hydroxy-4-aza-5-oxo-7-aza-7-(perfluorooctyl-sulphonyl)-nonyl]-1,4,7-tris(carboxymethyl)-1,4,7,10-tetraazacyclododecane,
- Gadolinium complex of 10-[2-hydroxy-4-oxa-1H,1H,2H,-3H,3H,5H,5H,6H,6H-perfluorotetradecyl]-1,4,7-tris(carboxymethyl)-1,4,7,10-tetraazacyclododecane,
- Gadolinium complex of 10-[2-hydroxy-4-aza-5-oxo-7-oxa-10,10,11,11,12,12,13,13,14,14,15,15,16,16,17,17,18,18,1-9,19-henicosafluoro-nonadecyl]-1,4,7-tris(carboxymethyl)-1,4,7,10-tetraazacyclododecane,
- Gadolinium complex of 10-[2-hydroxy-4-aza-5-oxo-11-aza-11-(perfluorooctylsulphonyl)-tridecyl]-1,4,7-tris(carboxymethyl)-1,4,7,10-tetraazacyclododecane,
- Gadolinium complex of 10-[2-hydroxy-4-aza-5-oxo-7-aza-7-(perfluorooctylsulphonyl)-8-phenyl-octyl]-1-4-7-tris(carboxymethyl)-1,4,7,10-tetraazacyclododecane.

12. Use according to one of Claims 1-7, **characterized in that** as perfluoroalkyl-containing metal complexes, the compounds of general formula Ia
A-R^{F} (Ia)
in which
• A is a moiety that contains 2 to 6 metal complexes, which are bonded directly or via a linker to a nitrogen atom of an annular skeleton chain,
and
• R^{F} is a perfluorinated, straight-chain or branched carbon chain with formula -CₙF₂ₙE, in which E represents a terminal fluorine, chlorine, bromine, iodine or hydrogen atom, and n stands for numbers 4-30, where the moiety A has the following structure: 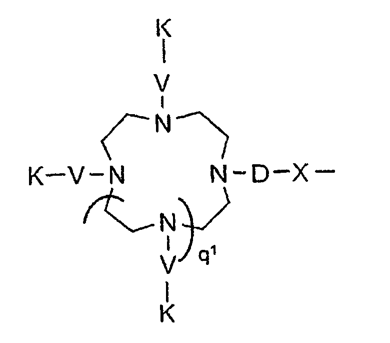
where
• q¹ is a number 0, 1, 2 or 3,
• K stands for a complexing agent or metal complex or their salts of organic and/or inorganic bases or amino acids or amino acid amides,
• X is a direct bond to the perfluoroalkyl group, a phenylene group or a C₁-C₁₀-alkylene chain, which optionally contains 1-15 oxygen atoms, 1-5 sulphur atoms, 1-10 carbonyl groups, 1-10 (NR^{d}) groups, 1-2 NR^{d}SO₂ groups, 1-10 CONR^{d} groups, 1 piperidine group, 1-3 SO₂ groups and 1-2 phenylene groups or optionally is substituted by 1-3 radicals R^{F}, in which R^{d} stands for a hydrogen atom, a phenyl group, benzyl group or a C₁-C₁₅ alkyl group, which optionally contains 1-2 NHCO groups, 1-2 CO groups, or 1-5 oxygen atoms and optionally is substituted by 1-5 hydroxy, 1-5 methoxy, 1-3 carboxy, or 1-3 R^{F} radicals,
• V is a direct bond or a chain of general formula IIa or IIIa: 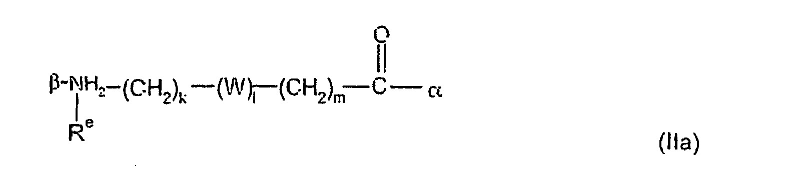 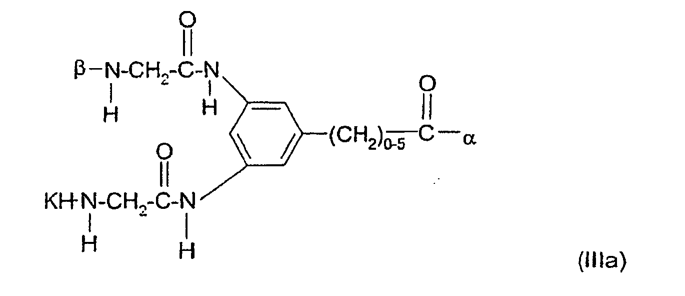
in which
■ R^{e} is a hydrogen atom, a phenyl group, a benzyl group or a C₁-C₇-alkyl group, which optionally is substituted with a carboxy group, a methoxy group or a hydroxy group,
■ W is a direct bond, a polyglycol ether group with up to 5 glycol units, or a molecule part of general formula IVa
-CH(R^{h}) - (IVa)
in which R^{h} is a C₁-C₇ carboxylic acid, a phenyl group, a benzyl group or a -(CH₂)₁₋₅-NH-K group,
■ α represents the binding to the nitrogen atom of the skeleton chain, β represents the binding to complexing agents or metal complex K,
■ and in which variables k and m stand for natural numbers between 0 and 10, and 1 stands for 0 or 1 and where
O D is a CO or SO₂ group, are used.

13. Use according to Claim 12, **characterized in that** the compounds of general formula Ia in which q is the number 1 are used.

14. Use according to Claim 12, **characterized in that** the compounds of general formula Ia are used, in which molecule part X is an alkylene chain, which contains 1-10 CH₂CH₂O groups or 1-5 COCH₂NH groups, a direct bond or one of the following structures
γ-CH₂-O-(CH₂)₂-δ, 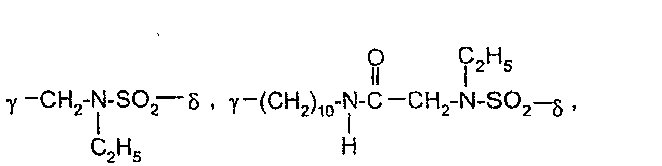 γ -(CH₂)₁₀-O-(CH₂)₂-δ , 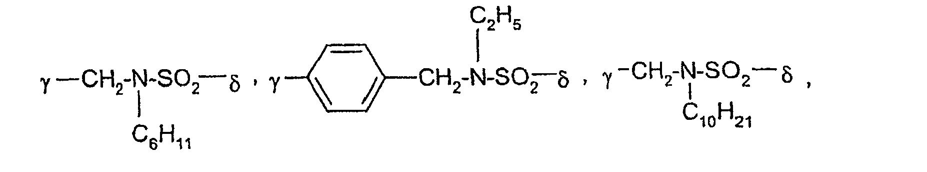 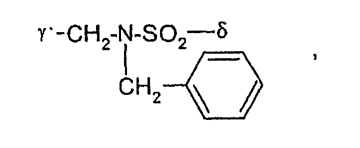 where γ binds to D, and δ binds to R^{F}.

15. Use according to Claim 12, **characterized in that** the compounds of general formula Ia, in which V is a molecule part with one of the following structures 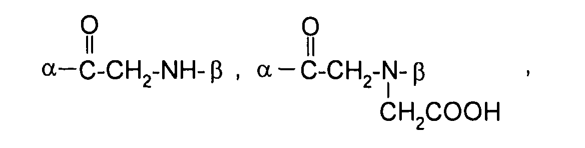 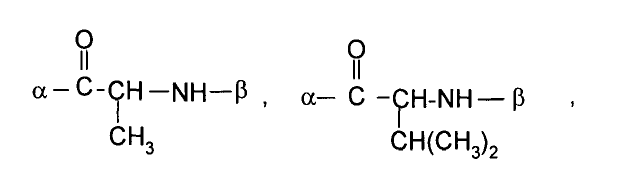 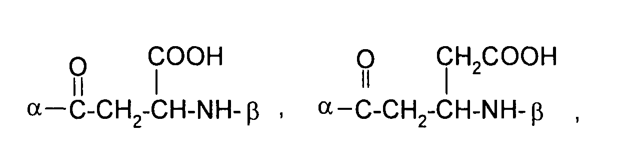 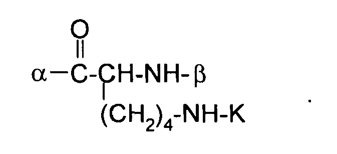 are used.

16. Use according to Claim 12, **characterized in that** the compounds of general formula Ia, in which K represents a complex of general formula Va, VIa, VIIa or VIIIa, 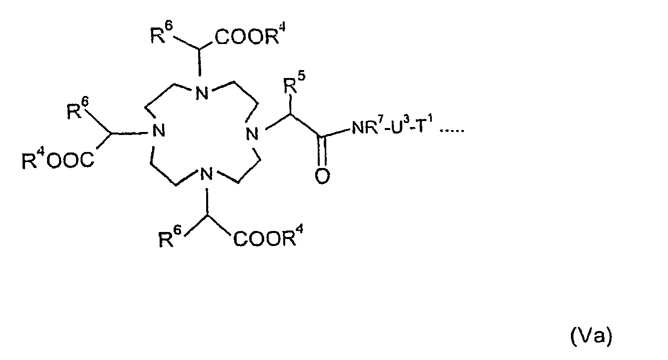 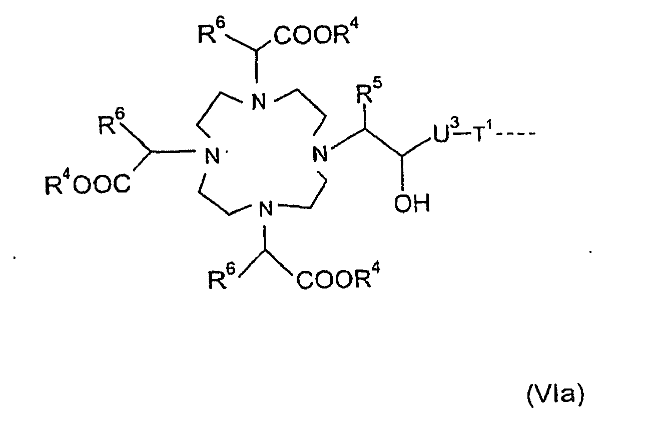 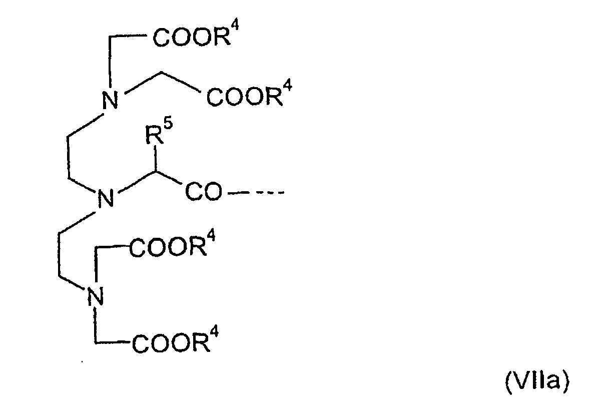 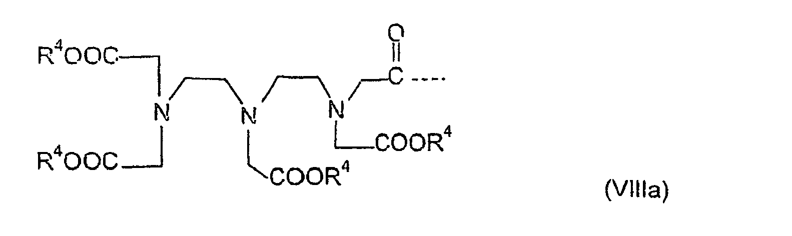 where
• R⁴, independently of one another, are a hydrogen atom or a metal ion equivalent of the elements of atomic numbers 23-29, 42-46 or 58-70,
• R⁵ is a hydrogen atom or a straight-chain, branched, saturated or unsaturated C₁-C₃₀ alkyl chain, which optionally is substituted by 1-5 hydroxy, 1-3 carboxy or 1 phenyl group(s) and/or optionally is interrupted by 1-10 oxygen atoms, 1 phenylene group or 1 phenylenoxy group,
• R⁶ is a hydrogen atom, a straight-chain or branched C₁-C₇ alkyl radical, a phenyl radical or benzyl radical,
• R⁷ is a hydrogen atom, a methyl group or ethyl group, which optionally is substituted by a hydroxy group or carboxy group,
• U³ is a straight-chain, branched, saturated or unsaturated C₁-C₂₀ alkylene group optionally containing 1-5 imino groups, 1-3 phenylene groups, 1-3 phenylenoxy groups, 1-3 phenylenimino groups, 1-5 amide groups, 1-2 hydrazide groups, 1-5 carbonyl groups, 1-5 ethylenoxy groups, 1 urea group, 1 thiourea group, 1-2 carboxyalkylimino groups, 1-2 ester groups, 1-10 oxygen atoms, 1-5 sulphur atoms and/or 1-5 nitrogen atoms, and/or optionally substituted by 1-5 hydroxy groups, 1-2 mercapto groups, 1-5 oxo groups, 1-5 thioxo groups, 1-3 carboxy groups, 1-5 carboxyalkyl groups, 1-5 ester groups and/or 1-3 amino groups, where the optionally contained phenylene groups can be substituted by 1-2 carboxy groups, 1-2 sulphone groups or 1-2 hydroxy groups
• T¹ stands for a -CO-β, -NHCO-β or -NHCS-β group, where β represents the binding site to V.

17. Use according to Claim 16, **characterized in that** the C₁-C₂₀-alkylene chain that stands for U³ contains the groups -CH₂NHCO-, -NHCOCH₂-, -NHCOCH₂OC₆H₄-, -N(CH₂CO₂H)-, -CH₂OCH2-, -NHCOCH₂C₆H₄-, -NHCSNHC₆H₄-, -CH₂OC₆H₄-, -CH₂CH₂O- and/or is substituted by the groups -COOH and -CH₂COOH.

18. Use according to Claim 16, **characterized in that** U³ stands for a -CH₂, -CH₂CH₂, -CH₂CH₂CH₂, -C₆H₄, -C₆H₁₀, -CH₂C₆H₄, -CH₂NHCOCH₂CH(CH₂CO₂H)-C₆H₄, -CH₂NHCOCH₂OCH₂, or -CH₂NHCOCH₂C₆H₄ group.

19. Use according to Claim 12, **characterized in that** the compounds of general formula Ia are used in which K has one of the following structures:

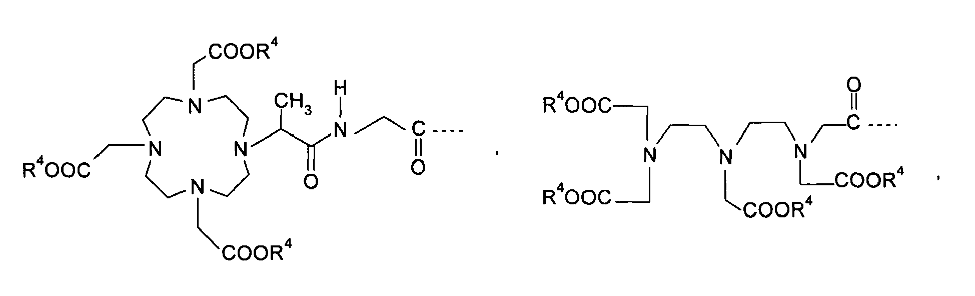

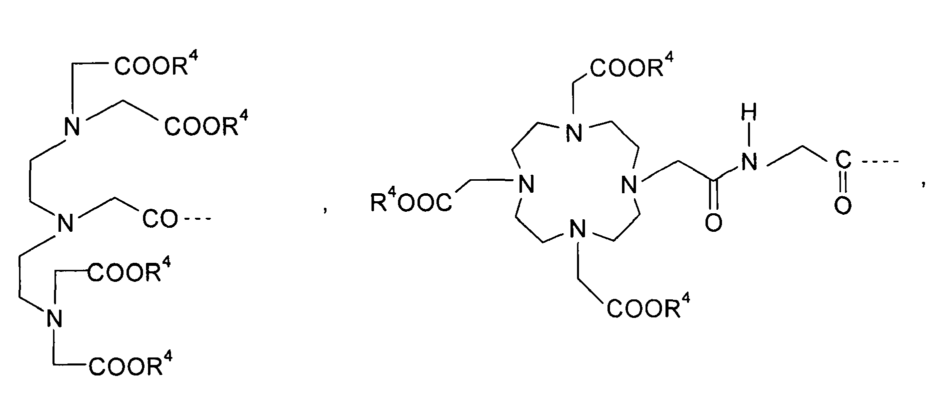

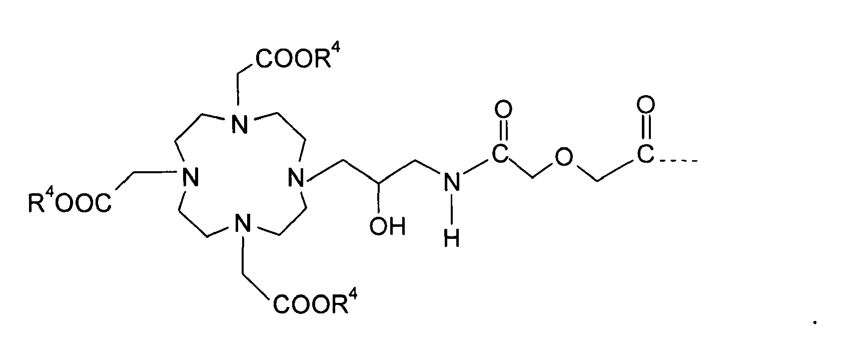

20. Use according to one of Claims 12 to 19, **characterized in that** the compounds of general formula Ia in which the perfluoroalkyl chain R^{F} -C₆F₁₃, -C₈F₁₇, -C₁₀F₂₁ or -C₁₂F₂₅ are used.

21. Use according to one of Claims 12 to 20, **characterized in that** the gadolinium complex of 1,4,7-tris{1,4,7-tris(N-(carboxylatomethyl)-10-[N-1-methyl-3,6-diaza-2,5,8-trioxooctane-1,8-diyl)]-1,4,7,10-tetraazacyclododecane, Gd complex}-10-[N-2H,2H,4H,4H,5H,5H-3-oxa-perfluorotridecanoyl]-1,4,7,10-tetraazacyclododecane is used.

22. Use according to one of Claims 1 to 7, **characterized in that** as perfluoroalkyl-containing metal complexes, the compounds of general formula Ib 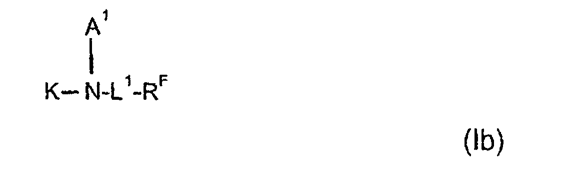 in which K means a complexing agent or a metal complex of general formula IIb 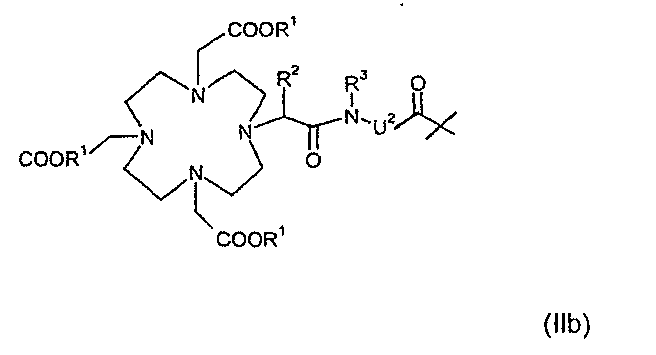 where R¹ stands for a hydrogen atom or a metal ion equivalent of atomic numbers 23-29, 42-46 or 58-70, R² and R³ stand for a hydrogen atom, a C₁-C₇-alkyl group, a benzyl group, a phenyl group, -CH₂OH or -CH₂-OCH₃, U² stands for radical L¹, where L¹ and U², independently of one another, can be the same or different, however, A¹ means a hydrogen atom, a straight-chain or branched C₁-C₃₀ alkyl group, which optionally is interrupted by 1-15 oxygen atoms, and/or optionally is substituted with 1-10 hydroxy groups, 1-2 COOH groups, a phenyl group, a benzyl group and/or 1-5 -OR⁹ groups, with R⁹ in the meaning of a hydrogen atom or a C₁-C₇ alkyl radical, or -L¹-R^{F}, L¹ means a straight-chain or branched C₁-C₃₀-alkylene group, which optionally is interrupted by 1-10 oxygen atoms, 1-5 -NH-CO groups, 1-5 -CO-NH groups, by a phenylene group optionally substituted by a COOH group, 1-3 sulphur atoms, 1-2 -N(B¹)-SO₂ groups and/or 1-2 -SO₂-N(B¹) groups with B¹ in the meaning of A¹, and/or optionally is substituted with radical R^{F}, and R^{F} means a straight-chain or branched perfluorinated alkyl radical of formula CₙF₂ₙE, where n stands for numbers 4-30, and E stands for a terminal fluorine atom, chlorine atom, bromine atom, iodine atom or a hydrogen atom, and optionally present acid groups' optionally can be present as salts of organic and/or inorganic bases or amino acids or amino acid amides, are used.

23. Use according to Claim 22, **characterized in that** the compounds of general formula Ib, in which R², R³ and R⁹, independently of one another, mean hydrogen or a C₁-C₄ alkyl group, are used.

24. Use according to Claim 22, **characterized in that** the compounds of general formula Ib, in which A¹ means hydrogen, a C₁-C₁₅-alkyl radical, the radicals C₂H₄-O-CH₃, C₃H₆-O-CH₃,
C₂H₄-O-(C₂H₄-O)ₜ-C₂H₄-OH,
C₂H₄-O-(C₂H₄-O)ₜ-C₂H₄-OCH₃,
C₂H₄OH, C₃H₆OH, C₄H₈OH, C₅H₁₀OH, C₆H₁₂OH, C₇H₁₄OH,
CH(OH)CH₂OH,
CH(OH)CH(OH)CH₂OH, CH₂[CH(OH)]ᵤ¹CH₂OH,
CH[CH₂(OH)]CH(OH)CH₂OH,
C₂H₄CH(OH)CH₂OH,
(CH₂)ₛCOOH,
C₂H₄-O-(C₂H₄-O)ₜ-CH₂COOH or
C₂H₄-O-(C₂H₄-O)ₜ-C₂H₄-CₙF₂ₙE or where s stands for integers 1 to 15, t stands for integers 0 to 13, u¹ stands for integers 1 to 10, n stands for integers 4 to 20, and E stands for hydrogen, fluorine, chlorine, bromine or iodine atoms, and if necessary, their branched isomers, are used.

25. Use according to Claim 22, **characterized in that** the compounds of general formula Ib, in which A¹ means hydrogen, C₁-C₁₀-alkyl,
C₂H₄-O-CH₃, C₃H₆-O-CH₃,
C₂H₄-O- (C₂H₄-O)ₓ-C₂H₄-OH, C₂H₄-O- (C₂H₄-O)ₓ-C₂H₄-OCH₃,
C₂H₄OH, C₃H₆OH,
CH₂[CH(OH)]_{y}CH₂OH,
CH[CH₂(OH)]CH(OH)CH₂OH,
(CH₂)_{w}COOH,
C₂H₄-O-(C₂H₄-O)ₓ-CH₂COOH,
C₂H₄-O-(C₂H₄O)ₓ-C₂H₄-CₙF₂ₙE where x stands for integers 0 to 5, y stands for integers 1 to 6, w stands for integers 1 to 10, n stands for integers 4 to 15, and E stands for a fluorine atom, and, if necessary, their branched isomers, are used.

26. Use according to Claim 22, **characterized in that** the compounds of general formula Ib, in which L¹ means
α-(CH₂)ₛ-β
α-CH₂-CH₂-(O-CH₂-CH₂-)_{y}-β
α-CH₂-(O-CH₂-CH₂-)_{y}-β,
α-CH₂-NH-CO-β
α-CH₂-CH₂-NH-SO₂-β
α-CH₂-NH-CO-CH₂-N(CH₂COOH)-SO₂-β
α-CH₂-NH-CO-CH₂-N(C₂H₅)-SO₂-β
α-CH₂-NH-CO-CH₂-N(C₁₀H₂₁)-SO₂-β
α-CH₂-NH-CO-CH₂-N(C₆H₁₃)-SO₂-β
α-CH₂-NH-CO-(CH₂)₁₀-N(C₂H₅)-SO₂-β
α-CH₂-NH-CO-CH₂-N(-CH₂-C₆H₅)-SO₂-β
α-CH₂-NH-CO-CH₂-N(-CH₂-CH₂-OH)SO₂-β
α-CH₂-NHCO-(CH₂)₁₀-S-CH₂CH₂-β
α-CH₂NHCOCH₂-O-CH₂CH₂-β
α-CH₂-CH₂NHCOCH₂-O-CH₂CH₂-β
α-CH₂-(CH₂-CH₂-O)ₜ-(CH₂)₃NHCO-CH₂-O-CH₂CH₂-β
α-CH₂NHCO(CH₂)₁₀-O-CH₂CH₂-β
α-CH₂CH₂NHCO(CH₂)₁₀-O-CH₂CH₂-β
α-CH₂-C₆H₄-O-CH₂CH₂-β
where phenylene group 1,4 or 1,3 is linked
α-CH₂-O-CH₂-C(CH₂-OCH₂CH₂-C₆F₁₃)₂-CH₂-OCH₂-CH₂-β
α-CH₂-NHCOCH₂CH₂CON-
CH₂CH₂NHCOCH₂N(C₂H₅)SO₂CO₂C₈F₁₇β
α-CH₂-CH₂NHCOCH₂N(C₂H₅)-SO₂-β
α-CH₂-O-CH₂-CH(OC₁₀H₂₁)-CH₂-O-CH₂CH₂-β
α-(CH₂NHCO)₄-CH₂O-CH₂CH₂-β
α-(CH₂NHCO)₃-CH₂O-CH₂CH₂-β
α-CH₂-OCH₂C(CH₂OH)₂-CH₂-O-CH₂CH₂-β 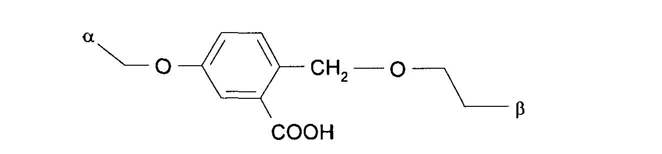 α -CH₂NHCOCH₂N(C₆H₅)-SO₂-β
α-NHCO-CH₂-CH₂-β
α-NHCO-CH₂-O-CH₂CH₂-β
α-NH-CO-β
α-NH-CO-CH₂-N(CH₂COOH)-SO₂-β
α-NH-CO-CH₂-N(C₂H₅)-SO₂-β
α-NH-CO-CH₂-N(C₁₀H₂₁)-SO₂-β
α-NH-CO-CH₂-N(C₆H₁₃)-SO₂-β
α-NH-CO-(CH₂)₁₀-N(C₂H₅)-SO₂-β
α-NH-CO-CH₂-N(-CH₂-C₆H₅)-SO₂-β
α-NH-CO-CH₂-N(-CH₂-CH₂-OH)SO₂-β
α-NH-CO-CH₂-β
α-CH₂-O-C₆H₄-O-CH₂-CH₂-β
α-CH₂-C₆H₄-O-CH₂-CH₂-β
α-N(C₂H₅)-SO₂-β
α-N(C₆H₅)-SO₂-β
α-N(C₁₀H₂₁)-SO₂-β
α-N(C₆H₁₃)-SO₂-β
α-N(C₂H₄OH)-SO₂-β
α-N(CH₂COOH)-SO₂-β
α-N(CH₂C₆H₅)-SO₂-β
α-N-[CH(CH₂OH)₂]-SO₂-β
α-N-[CH(CH₂OH)CH(OH)(CH₂OH)]-SO₂-β
where s stands for integers 1 to 15 and y stands for integers 1 to 6, are used.

27. Use according to Claim 22, **characterized in that** the compounds of general formula Ib, in which L¹ means
α-CH₂-O-CH₂CH₂-β,
α-CH₂-CH₂-(O-CH₂-CH₂-)_{y}-β,
α-CH₂-(O-CH₂-CH₂-)_{y}-β,
α-CH₂-CH₂-NH-SO₂-β, Ex. 10
α-CH₂NHCOCH₂-O-CH₂CH₂-β,
α-CH₂-CH₂NHCOCH₂-O-CH₂CH₂-β,
α-CH₂-(CH₂-CH₂-O)_{y}-(CH₂)₃NHCO-CH₂-O-CH₂CH₂-β,
α-CH₂NHCO(CH₂)₁₀-O-CH₂CH₂-β,
α-CH₂CH₂NHCO(CH₂)₁₀-O-CH₂CH₂-β,
α-CH₂-O-CH₂-CH(OC₁₀H₂₁)-CH₂-O-CH₂CH₂-β,
α-CH₂-O-C₆H₄-O-CH₂-CH₂-β or
α-CH₂-C₆H₄-O-CH₂-CH₂-β
or where y stands for integers 1 to 6, are used.

28. Use according to Claim 22, **characterized in that** the compounds of general formula Ib, in which R^{F} means a straight-chain or branched perfluorinated alkyl radical of formula CₙF₂ₙE, where n stands for numbers 4 to 15 and E stands for a terminal fluorine atom, are used.

29. Use according to one of Claims 22 to 28, **characterized in that** the following compounds are used:
- 1,4,7-Tris(carboxylatomethyl)-10-(3-aza-4-oxo-hexan-5--ylic)-acid-(2,3-dihydroxypropyl)-N-(1H, 1H,2H,2H,4H,4H,5H,5H-3-oxa)-perfluorotridecyl)-amide]-1,4,7,10-tetraazacyclododecane, gadolinium complex
- 1,4,7-Tris(carboxylatomethyl)-10-((3-aza-4-oxo-hexan-5-ylic)acid-N-(3,6,9,12,15-pentaoxa)-hexadecyl)-(1H,1H,2H,2H,4H,4H,5H,5H-3-oxa)-perfluorotridecyl]-amide}-1,4,7,10-tetraazacyclododecane, gadolinium complex
- 1,4,7-Tris(carboxylatomethyl)-10-{(3-aza-4-oxo-hexan-5-ylic)-acid-N-5-hydroxy-3-oxa-pentyl)-N-(1H,1H,2H,2H,4H,4H,5H,5H-3-oxa)-perfluorotridecyl]-amide}-1,4,7,10-tetraazacyclododecane, gadolinium complex
- 1,4,7-Tris(carboxylatomethyl)-10-{(3-aza-4-oxo-hexan-5-ylic)-acid-[N-3,6,9,15-tetraoxa-12-aza-15-oxo-C₁₇-C₂₆-hepta-decafluoro)hexacosyl]amide}-1,4,7,10-tetraazacyclododecane, gadolinium complex
- 1,4,7-Tris(carboxylatomethyl)-10-[(3-aza-4-oxo-hexan-5-ylic]-acid-N-(2-methoxyethyl)-N-(1H,1H,2H,2H,4H,4H,5H,5H-3-oxa)-perfluorotridecyl]-amide}-1,4,7,10-tetraazacyclododecane, gadolinium complex.

30. Use according to one of Claims 1 to 7, **characterized in that** as perfluoroalkyl-containing metal complexes, the compounds with sugar radicals of general formula Ic 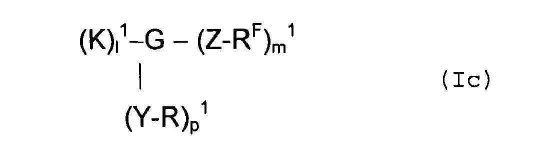 in which R represents a mono- or oligosaccharide radical bonded by the 1-OH- or 1-SH-position, R^{F} is a perfluorinated, straight-chain or branched carbon chain with the formula -CₙF₂ₙE, in which E represents a terminal fluorine, chlorine, bromine, iodine or hydrogen atom, and n stands for numbers 4-30, K stands for a metal complex of general formula IIc, 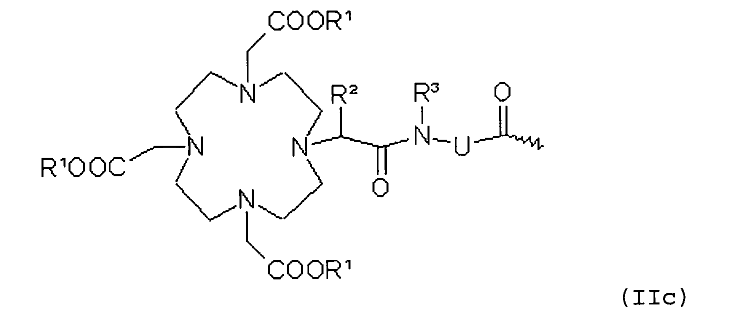 in which R¹ means a hydrogen atom or a metal ion equivalent of atomic numbers 23-29, 42-46 or 58-70, provided that at least two R¹ stand for metal ion equivalents, R² and R³ independently of one another, represent hydrogen, C₁-C₇-alkyl, benzyl, phenyl, -CH₂OH or -CH₂OCH₃, and U represents -C₆H₄-O-CH₂-ω-, -(CH₂)₁₋₅-ω-, a phenylene group, -CH₂-NHCO-CH₂-CH(CH₂COOH)-C₆H₄-ω-, -C₆H₄-(OCH₂CH₂)₀₋₁-N(CH₂COOH)-CH₂-ω, or a C₁-C₁₂-alkylene group or C₇-C₁₂-C₆H₄-O group optionally interrupted by one or more oxygen atoms, 1 to 3 -NHCO groups or 1 to 3 -CONH groups and/or substituted with 1 to 3 -(CH₂)₀₋₅COOH groups, where ω stands for the binding site to -CO-, or of general formula IIIc 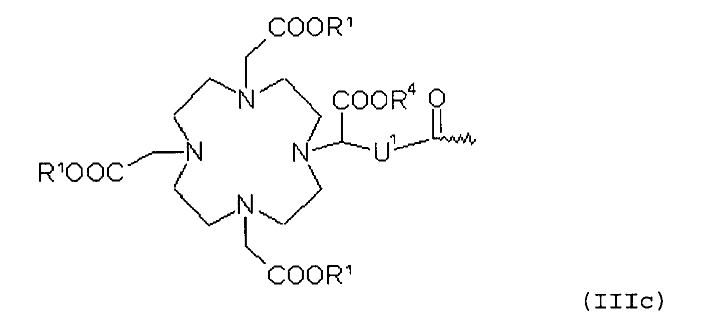 in which R¹ has the above-mentioned meaning, R⁴ represents hydrogen or a metal ion equivalent mentioned under R¹, and U¹ represents -C₆H₄-O-CH₂-ω-, where ω means the binding site to -CO-, or of general formula IVc 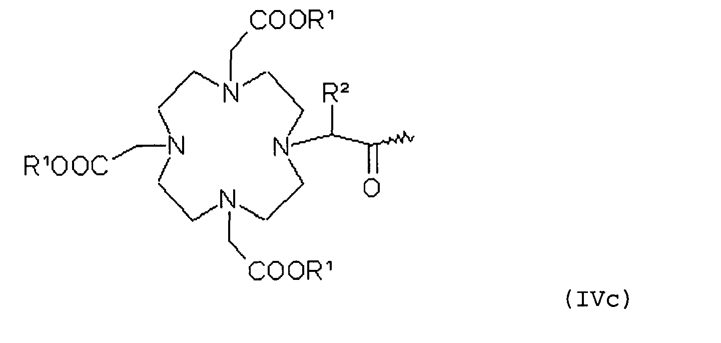 in which R¹ and R² have the above-mentioned meaning or of general formula VcA or VcB 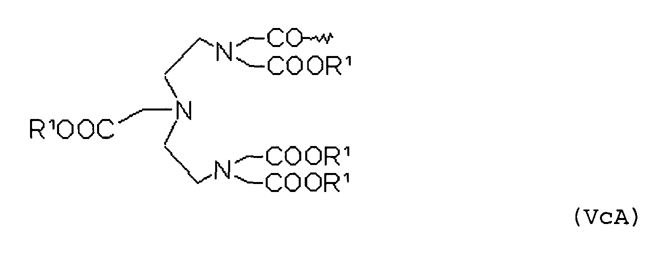 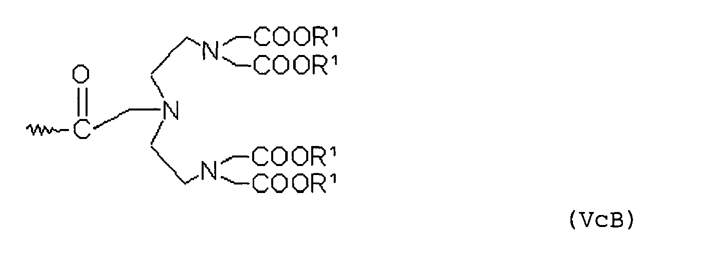 in which R¹ has the above-mentioned meaning, or of general formula VIc 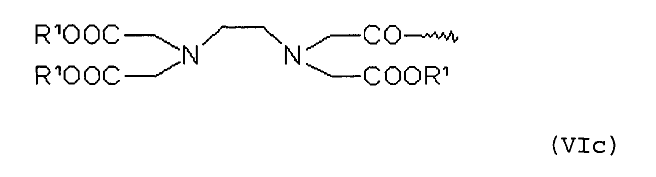 in which R¹ has the above-mentioned meaning, or of general formula VIIc 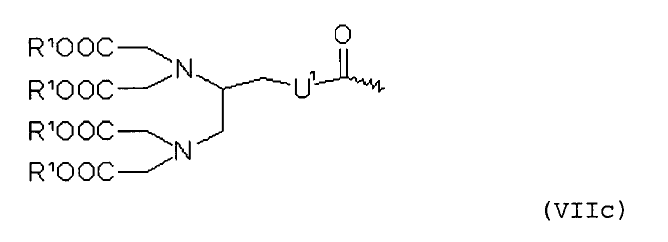 in which R¹ has the above-mentioned meaning, and U¹ represents -C₆H₄-O-CH₂-ω-, where ω means the binding site to -CO- or of general formula VIIIc 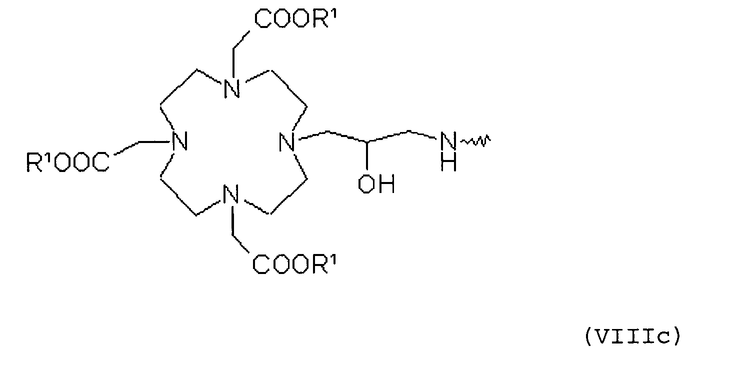 in which R¹ has the above-mentioned meaning, and in radical K, optionally present free acid groups optionally can be present as salts of organic and/or inorganic bases or amino acids or amino acid amides, G for the case that K means metal complexes IIc to VIIc represents a radical that is functionalized in at least three places and is selected from the following radicals a) to j) 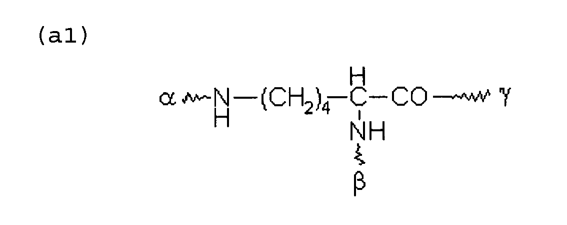 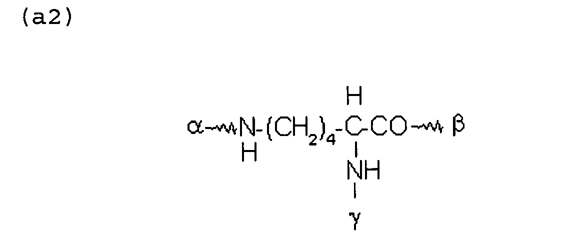 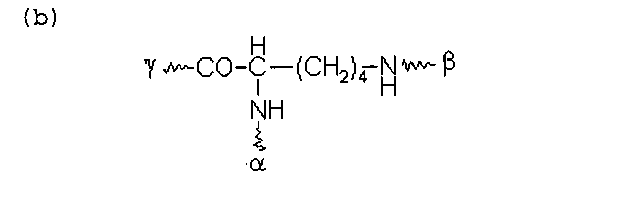 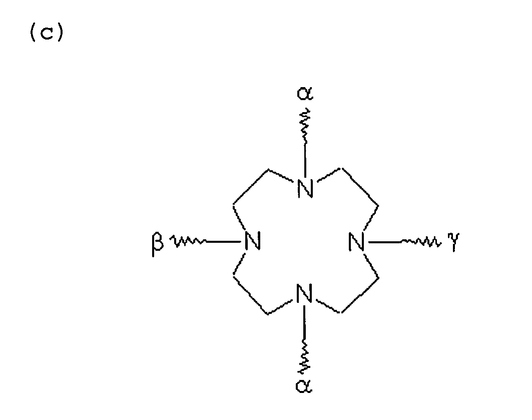 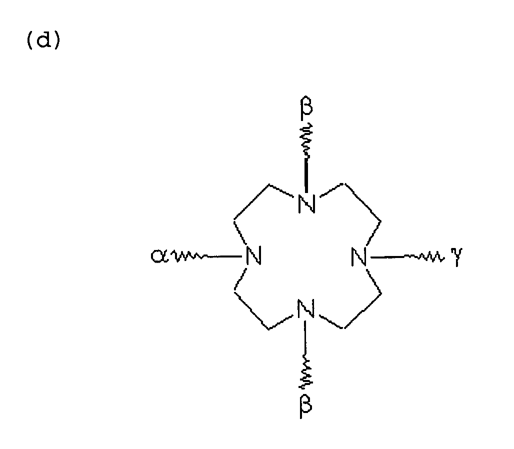 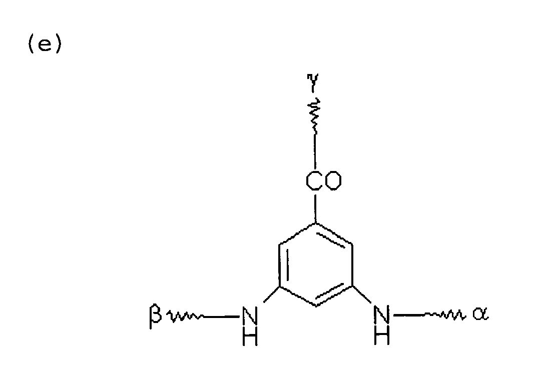 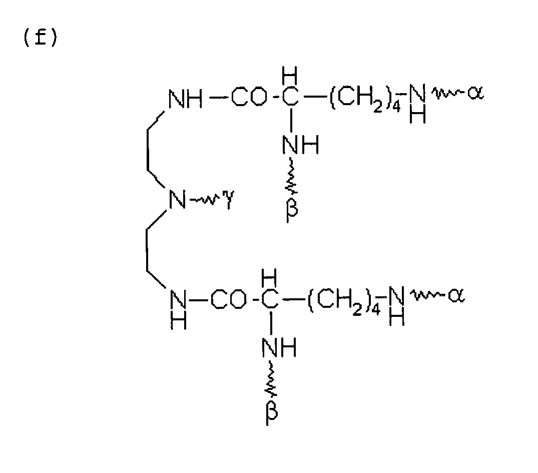 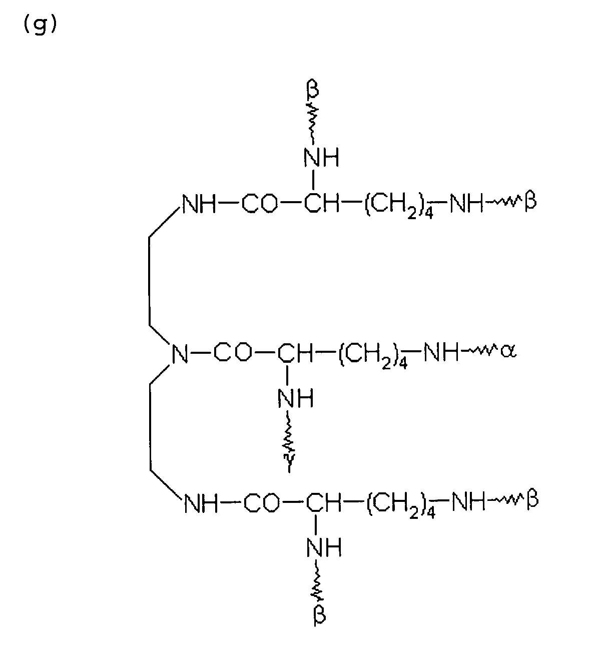 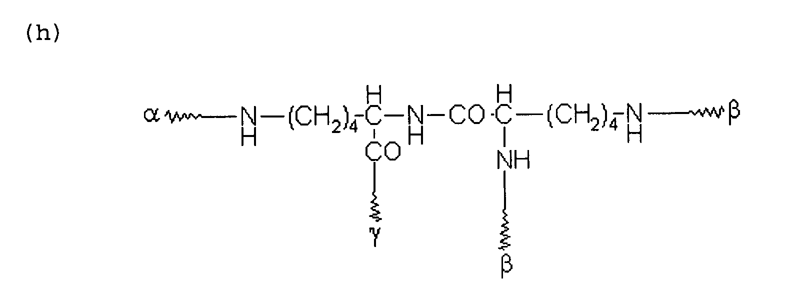 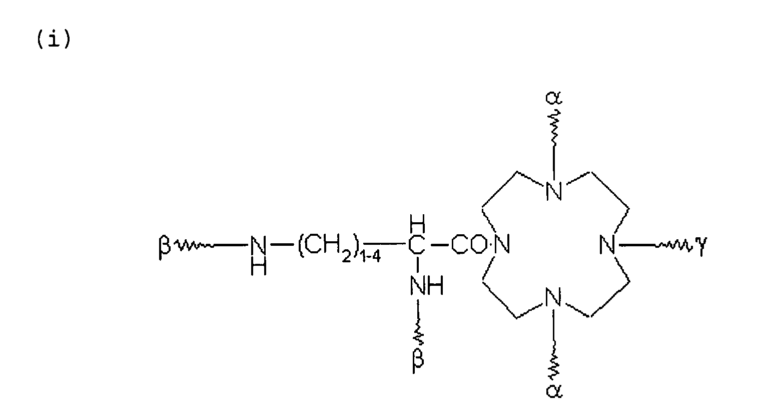 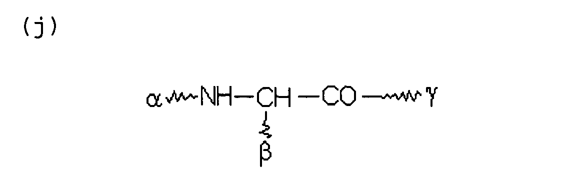 and G for the case that K means metal complex VIIIc represents a radical that is functionalized in at least three places and is selected from k) or l), 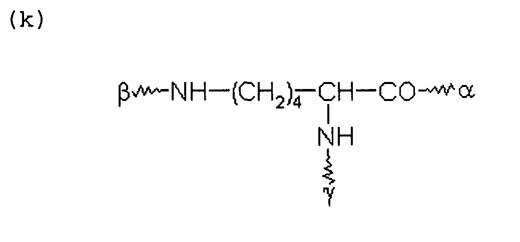 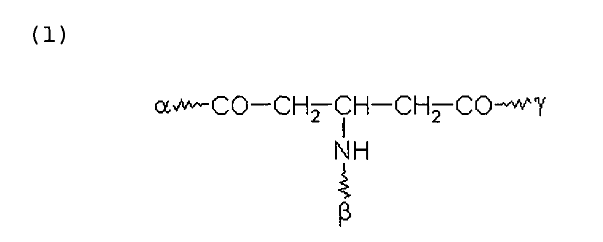 where α means the binding site of G to complex K, β is the binding site of G to radical Y, and γ represents the binding site of G to radical Z, Y means -CH₂-, δ-(CH₂)₍₁₋₅₎CO-β, β-(CH₂)₍₁₋₅₎CO-δ, δ-CH₂-CHOH-CO-β oder δ-CH(CHOH-CH₂OH)-CHOH-CHOH-CO-β where δ represents the binding site to sugar radical R and β is the binding site to radical G, Z stands for 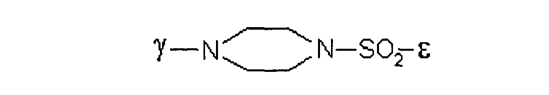 γ -COCH₂-N(C₂H₅)-SO₂-ε,
γ-COCH₂-O-(CH₂)₂-SO₂-ε, 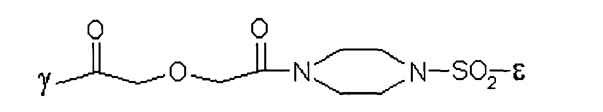 or
γ - NHCH₂CH₂-O-CH₂CH₂ - ε
where γ represents the binding site of Z to radical G, and ε means the binding site of Z to perfluorinated radical R^{F} and l¹, m¹, independently of one another, mean integers 1 or 2, and p¹ means integers 1 to 4, are used.

31. Use according to Claim 30, **characterized in that** the compounds of general formula Ic, in which R represents a monosaccharide radical with 5 to 6 C atoms or its deoxy compound, preferably glucose, mannose or galactose, are used.

32. Use according to Claim 30, **characterized in that** the compounds of general formula Ic, in which R² and R³, independently of one another, mean hydrogen or C₁-C₄ alkyl and/or E in formula -CₙF₂ₙE means a fluorine atom, are used.

33. Use according to Claim 30, **characterized in that** the compounds of general formula Ic, in which G represents lysine radical (a) or (b), are used.

34. Use according to Claim 30, **characterized in that** the compounds of general formula Ic are used, in which Z means 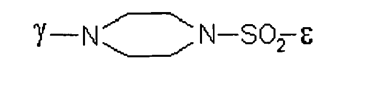 where γ represents the binding site of Z to radical G, and ε means the binding site of Z to perfluorinated radical R^{F}, and/or Y means δ-CH₂CO-β, where δ represents the binding site to sugar radical R and β represents the binding site to radical G.

35. Use according to Claim 30, **characterized in that** the compounds of general formula Ic are used, in which U in metal complex K represents -CH₂- or -C₆H₄-O-CH₂-ω, where ω stands for the binding site to -CO-.

36. Use according to Claim 30, **characterized in that** the gadolinium complex of 6-N-[1,4,7-tris(carboxylataomethyl)-1,4,7,10-tetraazacyclododecane-10-N-(pentanoyl-3-aza-4-oxo-5-methyl-5-yl)]-2-N-[1-O-α-D-carbonylmethyl-mannopyranose]-L-lysine-(1-(4-perfluorooctylsulphonyl)-piperazine]-amide is used.

37. Use according to one of Claims 1 to 7, **characterized in that** as perfluoroalkyl-containing metal complexes, the compounds with polar radicals of general formula Id 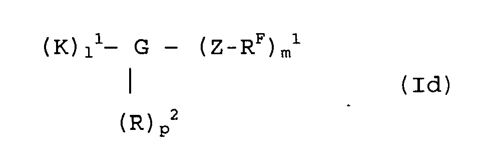 in which R^{F} is a perfluorinated, straight-chain or branched carbon chain with formula -CₙF₂ₙE, in which E represents a terminal fluorine, chlorine, bromine, iodine or hydrogen atom, and n stands for numbers 4-30, K stands for a metal complex of general formula IId, 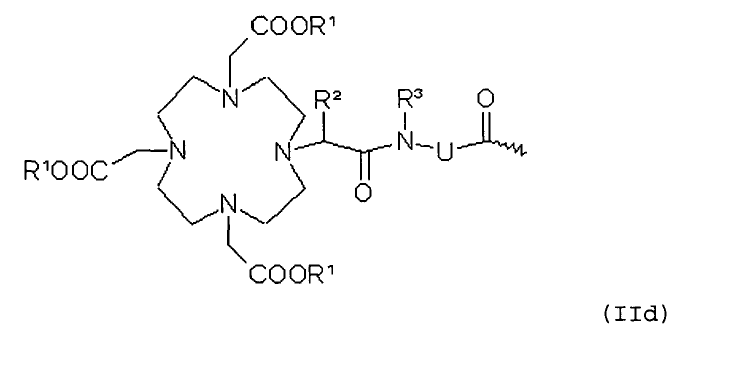 in which R¹ means a hydrogen atom or a metal ion equivalent of atomic numbers 23-29, 42-46 or 58-70, provided that at least two R¹ stand for metal ion equivalents, R² and R³, independently of one another, represent hydrogen, C₁-C₇ alkyl, benzyl, phenyl, -CH₂OH or -CH₂OCH₃, and U represents -C₆H₄-O-CH₂-ω-, -(CH₂)₁₋₅-ω, a phenylene group, -CH₂-NHCO-CH₂-CH(CH₂COOH)-C₆H₄-ω-, -C₆H₄-(OCH₂CH₂)₀₋₁-N(CH₂COOH)-CH₂-ω, or a C₁-C₁₂ alkylene group or C₇-C₁₂-C₆H₄-O group optionally interrupted by one or more oxygen atoms, 1 to 3 -NHCO groups, 1 to 3 -CONH groups and/or substituted with 1 to 3 -(CH₂)₀₋₅COOH groups, where ω stands for the binding site to -CO-, or of general formula IIId 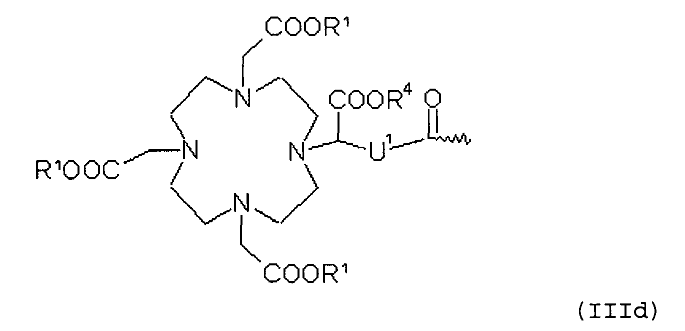 in which R¹ has the above-mentioned meaning, R⁴ represents hydrogen or a metal ion equivalent mentioned under R¹, and U¹ represents -C₆H₄-O-CH₂-ω-, where ω means the binding site to -CO-, or of general formula IVd 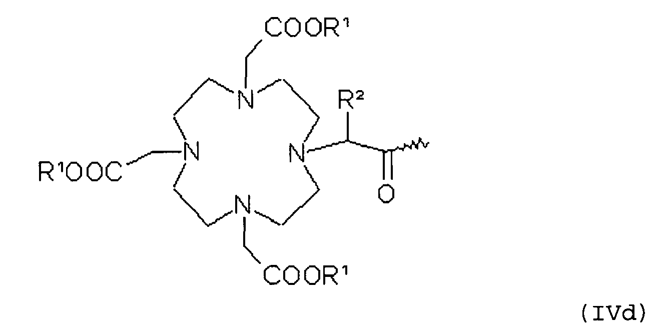 in which R¹ and R² have the above-mentioned meaning, or of general formula VdA or VdB 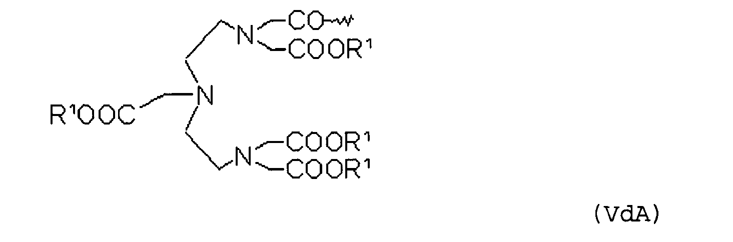 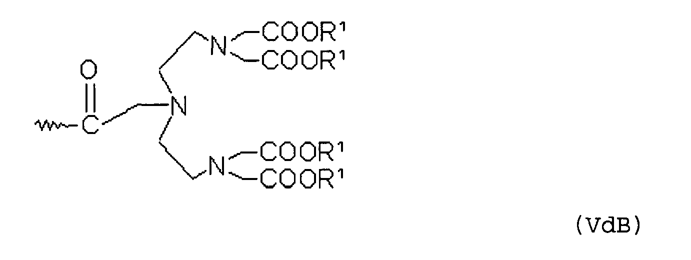 in which R¹ has the above-mentioned meaning, or of general formula VId 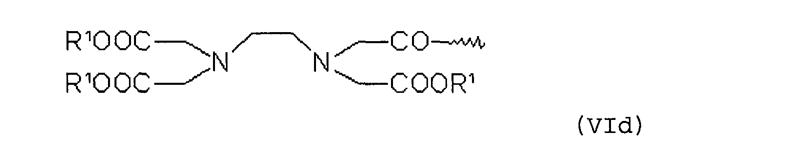 in which R¹ has the above-mentioned meaning, or of general formula VIId 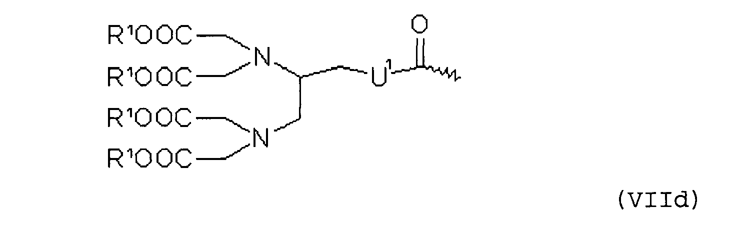 in which R¹ has the above-mentioned meaning, and U¹ represents -C₆H₄-O-CH₂-ω-, where ω means the binding site to -CO-, and in radical K, optionally present free acid groups optionally can be present as salts of organic and/or inorganic bases or amino acids or amino acid amides, G represents a radical that is functionalized in at least three places and is selected from the following radicals a) to i) 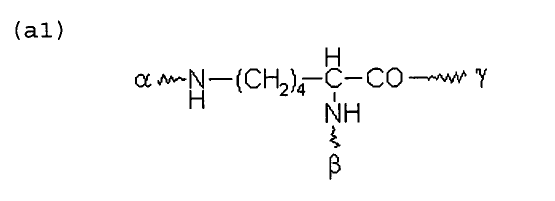 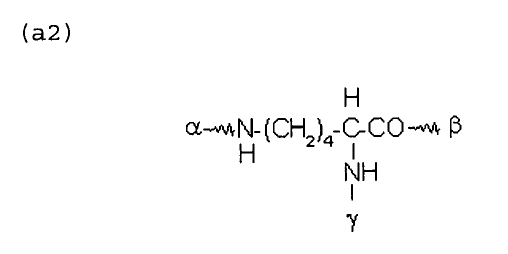 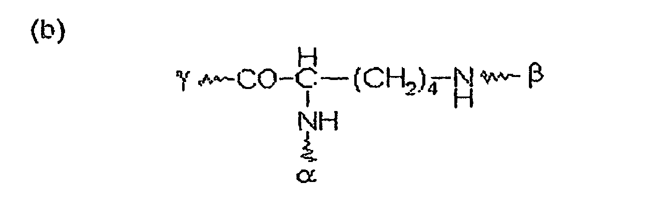 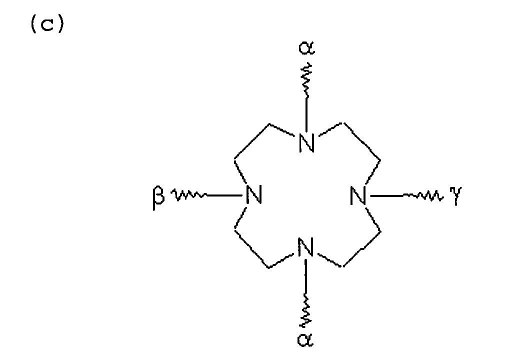 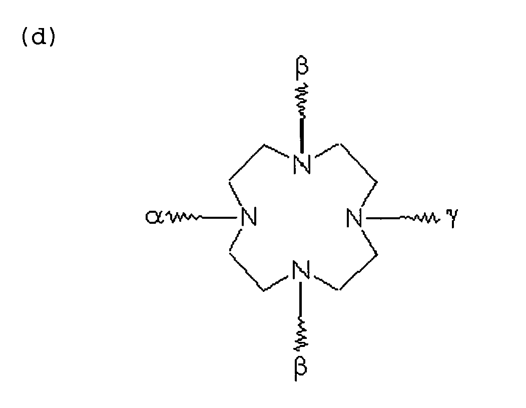 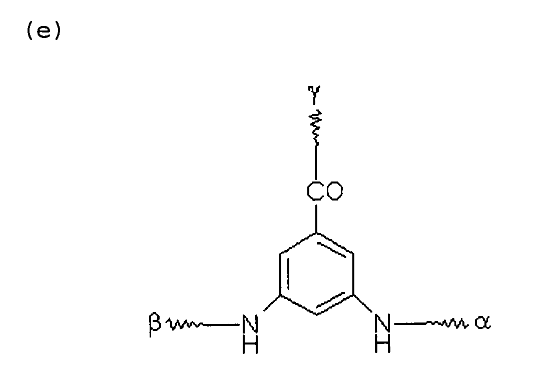 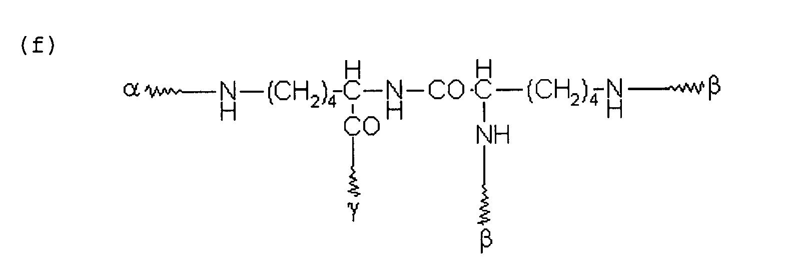 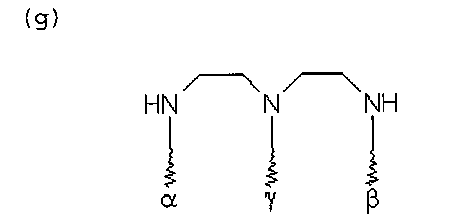 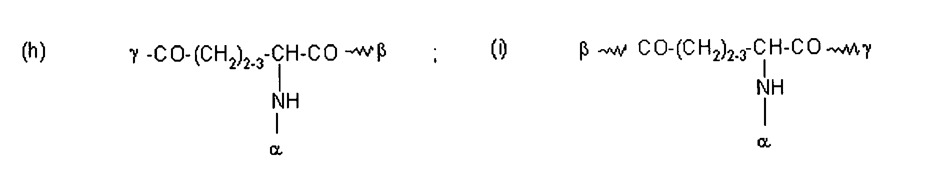 where α means the binding site of G to complex K, β is the binding site of G to radical R, and γ represents the binding site of G to radical Z, Z stands for 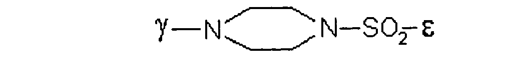 γ -C(O)CH₂O(CH₂)₂-ε,
where γ represents the binding site of Z to radical G and ε means the binding site of Z to perfluorinated radical R_{f}, R represents a polar radical that is selected from complexes K of general formulas IId to VIId, where R¹ here means a hydrogen atom or a metal ion equivalent of atomic numbers 20, 23-29, 42-46 or 58-70, and radicals R², R³, R⁴, U and U¹ have the above-indicated meaning, or means the folic acid radical or means a carbon chain with 2-30 C atoms that is bonded to radical G via -CO- or SO₂- or a direct bond, and is straight or branched, saturated or unsaturated, optionally interrupted by 1-10 oxygen atoms, 1-5 -NHCO groups, 1-5 -CONH groups, 1-2 sulphur atoms, 1-5 -NH groups or 1-2 phenylene groups, which optionally can be substituted with 1-2 OH groups, 1-2 NH₂ groups, 1-2 - COOH groups, or 1-2 -SO₃H groups, or optionally substituted with 1-8 OH groups, 1-5 -COOH groups, 1-2 SO₃H groups, 1-5 NH₂ groups, or 1-5 C₁-C₄ alkoxy groups, and l¹, m¹, p², independently of one another, mean integers 1 or 2, are used.

38. Use according to Claim 37, **characterized in that** the compounds of general formula Id, in which K stands for a metal complex of general formula IId, IIId, VdB or VIId, are used.

39. Use according to Claim 37, **characterized in that** the compounds of general formula Id, in which polar radical R has the meaning of complex K, preferably complex K of general formulas IId, IIId, VdA or VIId, are used.

40. Use according to Claim 37, **characterized in that** the compounds of general formula Id, in which polar radical R has the following meanings:
- C(O)CH₂CH₂SO₃H
- C(O)CH₂OCH₂CH₂OCH₂CH₂OH
- C(O)CH₂OCH₂CH₂OH
- C(O)CH₂OCH₂CH(OH)CH₂OH
- C(O)CH₂NH-C(O)CH₂COOH
- C (O)CH₂CH(OH) CH₂OH
- C(O)CH₂OCH₂COOH
- SO₂CH₂CH₂COOH
- C(O)-C₆H₃- (m-COOH)₂
- C(O)CH₂O(CH₂)₂-C₆H₃-(m-COOH)₂
- C(O)CH₂O-C₆H₄-m-SO₃H
- C(O)CH₂NHC(O)CH₂NHC(O)CH₂OCH₂COOH
- C(O)CH₂OCH₂CH₂OCH₂COOH
- C(O)CH₂OCH₂CH(OH) CH₂O-CH₂CH₂OH
- C(O)CH₂OCH₂CH(OH) CH₂OCH₂-CH(OH)-CH₂OH
- C(O)CH₂SO₃H
- C(O)CH₂CH₂COOH
- C(O)CH(OH)CH(OH)CH₂OH
- C(O)CH₂O[(CH₂)₂O]₁₋₉-CH₃
- C(O)CH₂O[(CH₂)₂O]₁₋₉-H
- C(O)CH₂OCH(CH₂OH)₂
- C(O)CH₂OCH(CH₂OCH₂COOH)₂
- C(O)-C₆H₃-(m-OCH₂COOH)₂
- CO-CH₂O-(CH₂)₂O(CH₂)₂O-(CH₂)₂O(CH₂)₂OCH₃
preferably -C(O)CH₂O[(CH₂)₂O]₄-CH₃ are used.

41. Use according to Claim 37, **characterized in that** the compounds of general formula Id, in which polar radical R is the folic acid radical, are used.

42. Use according to Claim 37, **characterized in that** the compounds of general formula Id, in which G represents lysine radical (a) or (b), are used.

43. Use according to Claim 37, **characterized in that** the compounds of general formula Id, in which U represents group -CH₂- or -C₆H₄-O-CH₂-ω in metal complex K, where ω stands for the binding site to -CO-, are used.

44. Use according to one of Claims 37-43, **characterized in that** the gadolinium complex of 2,6-N,N'-bis[1,4,7-tris(carboxylatomethyl)-1,4,7,10-tetraazacyclododecane-10-(pentanoyl-3-aza-4-oxo-5-methyl-5-yl)]-lysine-[1-(4-perfluorooctylsulfonyl-piperazine]-amide is used.

45. Use according to one of Claims 1-7, **characterized in that** as perfluoroalkyl-containing metal complexes, galenical formulations that contain paramagnetic, perfluoroalkyl-containing metal complexes of general formulas I, Ia, Ib, Ic and/or Id and diamagnetic perfluoroalkyl-containing substances, preferably dissolved in an aqueous solvent, are used.

46. Use according to Claim 45, **characterized in that** as diamagnetic perfluoroalkyl-containing substances, those of general formula XX
R^{F}-L²-B² (XX)
in which R^{F} represents a straight-chain or branched perfluoroalkyl radical with 4 to 30 carbon atoms, L² stands for a linker and B² stands for a hydrophilic group, are used.

47. Use according to Claim 46, **characterized in that** linker L² is a direct bond, an -SO₂ group, or a straight-chain or branched carbon chain with up to 20 carbon atoms, which can be substituted with one or more -OH, -COO⁻, or -SO₃ groups and/or optionally contains one or more -O-, -S-, -CO-, -CONH-, -NHCO-, -CONR⁹-, -NR⁹CO-, -SO₂-, -PO₄⁻-, -NH or -NR⁹ groups, an aryl ring or a piperazine, where R⁹ stands for a C₁- to C₂₀-alkyl radical, which in turn can contain one or more O atoms, and/or can be substituted with -COO⁻ or SO₃ groups.

48. Use according to Claim 46, **characterized in that** hydrophilic group B² is a mono- or disaccharide, one or more adjacent -COO⁻ or -SO₃⁻ groups, a dicarboxylic acid, an isophthalic acid, a picolinic acid, a benzenesulphonic acid, a tetrahydropyrandicarboxylic acid, a 2,6-pyridinedicarboxylic acid, a quaternary ammonium ion, an aminopolycarboxylic acid, an aminodipolyethylene glycolsulphonic acid, an aminopolyethylene glycol group, an SO₂-(CH₂)₂-OH group, a polyhydroxyalkyl chain with at least two hydroxyl groups or one or more polyethylene glycol chains with at least two glycol units, where the polyethylene glycol chains are terminated by an -OH or -OCH₃ group.

49. Use according to Claim 45, **characterized in that** as diamagnetic perfluoroalkyl-containing substances conjugates that consist of α-, β- or γ-cyclodextrin and compounds of general formula XXII
A²-L³-R^{F} (XXII)
in which A² stands for an adamantane, biphenyl or anthracene molecule, L³ stands for a linker, and R^{F} stands for a straight-chain or branched perfluoroalkyl radical with 4 to 30 carbon atoms, and where linker L³ is a straight-chain hydrocarbon chain with 1 to 20 carbon atoms, which can be interrupted by one or more oxygen atoms, one or more CO-, SO₂-, CONH-, NHCO-, CONR¹⁰-, NR¹⁰CO-, NH- or NR¹⁰ groups or a piperazine, where R¹⁰ is a C₁-C₅ alkyl radical, are used.

50. Use according to Claim 45, **characterized in that** as diamagnetic perfluoroalkyl-containing substances, those of general formula XXI:
R^{F}-X¹ (XXI)
in which R^{F} represents a straight-chain or branched perfluoroalkyl radical with 4 to 30 carbon atoms, and X¹ is a radical that is selected from the group of the following radicals (n in this case is a number between 1 and 10), are used:

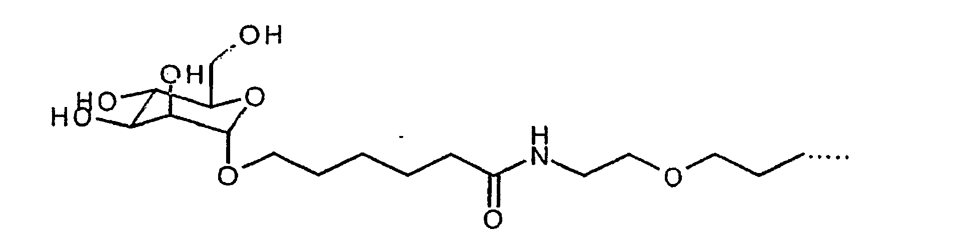

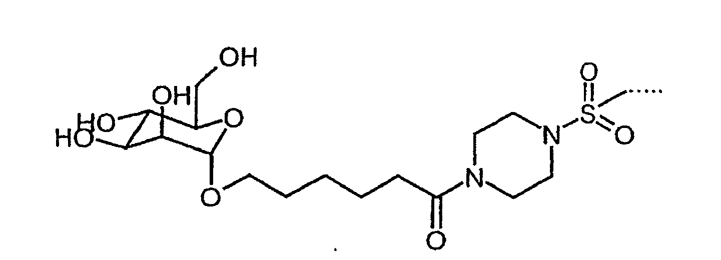

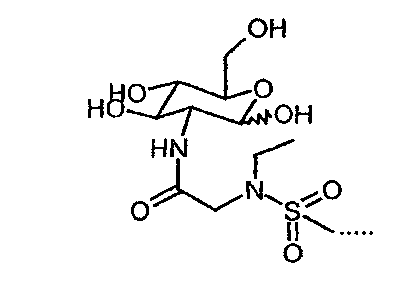

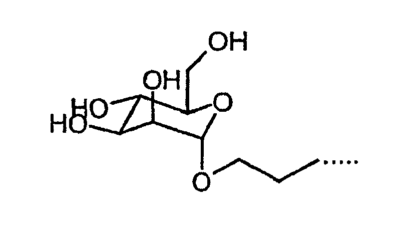

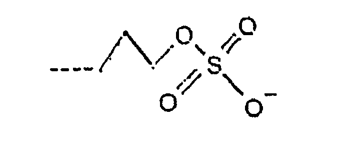

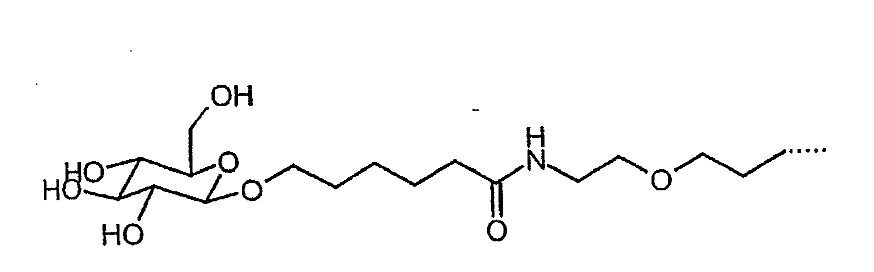

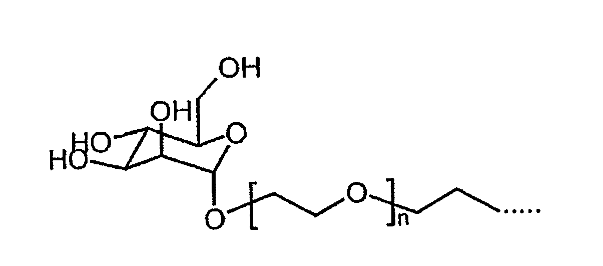

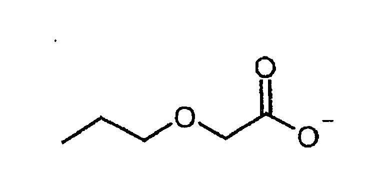

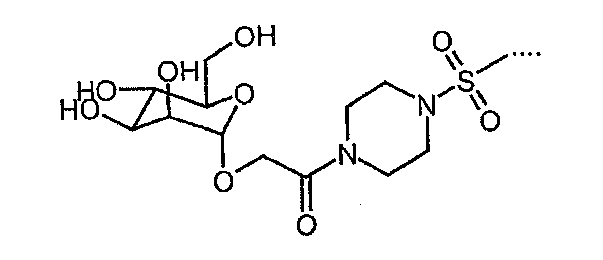

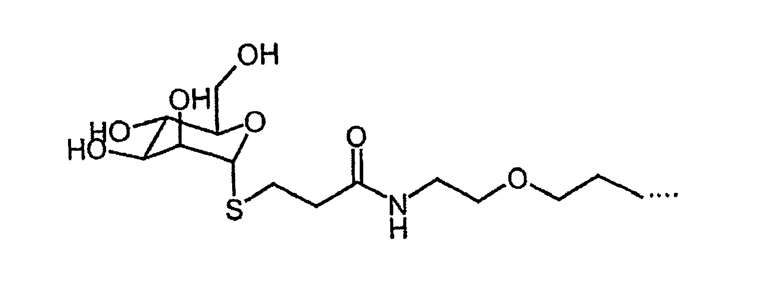

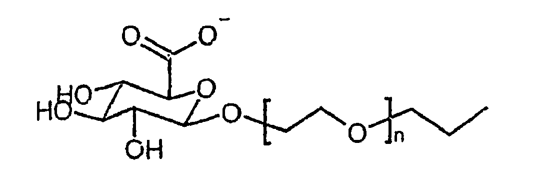

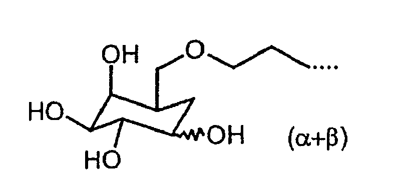

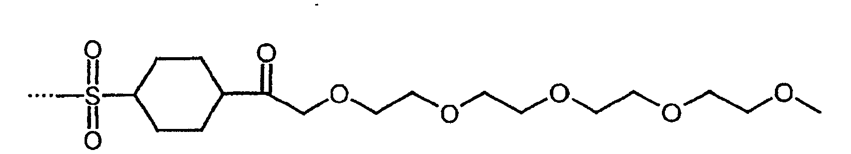

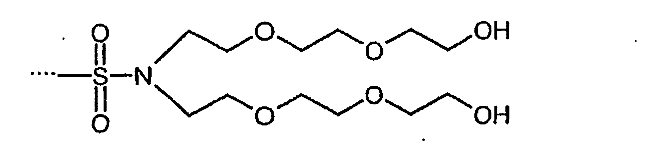

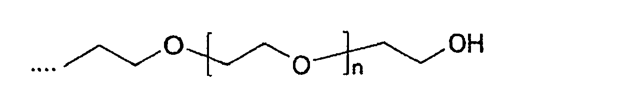

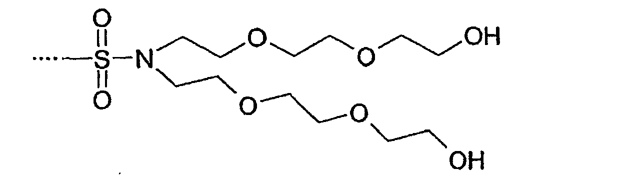

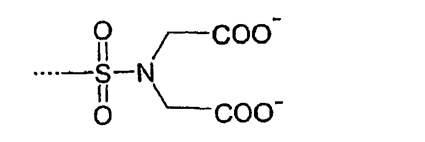

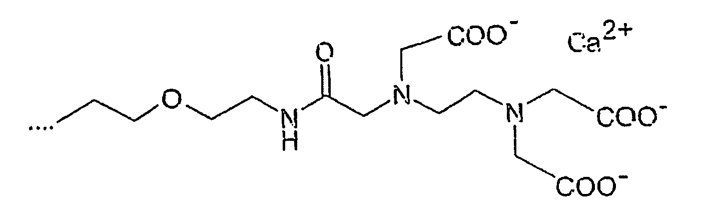

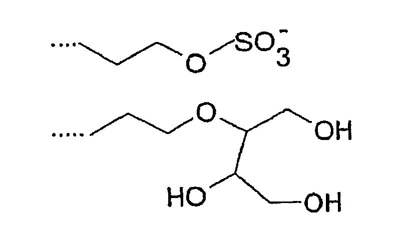

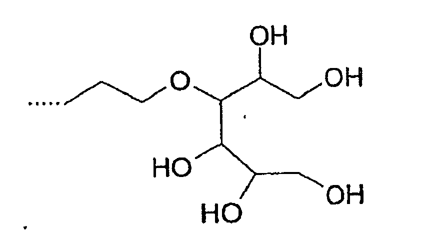

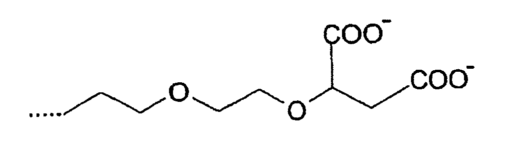

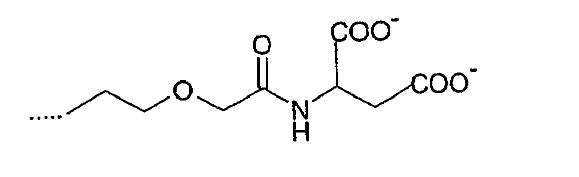

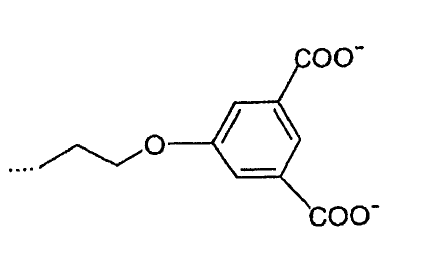

## Revendications

1. Utilisation de complexes métalliques perfluoralkylés, qui présentent une concentration critique pour la formation des micelles inférieure à 10⁻³ mol/l, un diamètre hydrodynamique des micelles (2 Rh) > 1 nm et une relaxivité des protons dans le plasma (R¹) à 40°C pour une intensité de champ de 0,47 teslas supérieure à 10 1/mmol.s, pour préparer un produit de contraste en IRM dans le but de représenter des thrombus intravasculaires.

2. Utilisation selon la revendication 1, **caractérisée en ce que** les complexes métalliques sont utilisés en tant que produits de contraste en IRM pour représenter des thrombus veineux.

3. Utilisation selon la revendication 1 ou 2, **caractérisée en ce que** les complexes métalliques sont utilisés en tant que produits de contraste en IRM pour représenter des thrombus artériels.

4. Utilisation selon l'une des revendications 1 à 3, **caractérisée en ce que** les complexes métalliques sont utilisés en tant que produits de contraste en IRM pour la détection précoce d'une oblitération thrombotique.

5. Utilisation selon la revendication 1, **caractérisée en ce qu'**on utilise des complexes métalliques dont la concentration pour la formation des micelles est inférieure à 10⁻⁴ mol/l.

6. Utilisation selon la revendication 1, **caractérisée en ce qu'**on utilise des complexes métalliques dont le diamètre hydrodynamique des micelles est ≥ 3 nm, de préférence > 4 nm.

7. Utilisation selon la revendication 1, **caractérisée en ce qu'**on utilise des complexes métalliques qui présentent une relaxivité des protons dans le plasma supérieure à 13 l/mmol.s, de préférence supérieure à 15 1/mmol.s.

8. Utilisation selon l'une des revendications 1 à 7, **caractérisée en ce qu'**on utilise en tant que complexes métalliques perfluoralkylés les composés de formule générale I
R^{F}-L-K I
dans laquelle
R^{F} est une chaîne carbonée perfluorée, droite ou ramifiée, de formule -CₙF₂ₙE, dans laquelle E est un atome de fluor, de chlore, de brome, d'iode ou d'hydrogène terminal, et n est un nombre de 4 à 30,
L est une liaison directe, un groupe méthylène, un groupe -NHCO-, un groupe 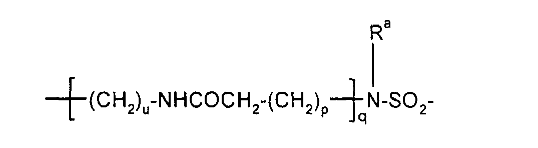 où p est un nombre de 0 à 10, q et u représentent chacun indépendamment de l'autre le nombre 0 ou 1, et
R^{a} est un atome d'hydrogène, un groupe méthyle, un groupe benzyle, un groupe phényle, un groupe -CH₂-OH, un groupe -CH₂OCH₃, un groupe -CH₂-CO₂H, ou une chaîne en C₂-C₁₅, qui est éventuellement interrompue par 1 à 3 atomes d'oxygène, 1 à 2 groupes >CO ou un groupe aryle éventuellement substitué, et/ou est substituée par 1 à 4 groupes hydroxyle, 1 à 2 groupes alcoxy en C₁ à C₄, 1 à 2 groupes carboxy, un groupe -SO₃H,
ou
une chaîne carbonée en C₂ à C₃₀ droite, ramifiée, saturée ou insaturée, qui contient éventuellement 1 à 10 atomes d'oxygène, 1 à 3 groupes -NR^{a}, 1 à 2 atomes de soufre, une pipérazine, un groupe -CONR^{a}, 1 à 6 groupes -NR^{a}CO, un groupe -SO₂, un groupe -NR^{a}-CO₂, 1 à 2 groupes -CO, un groupe 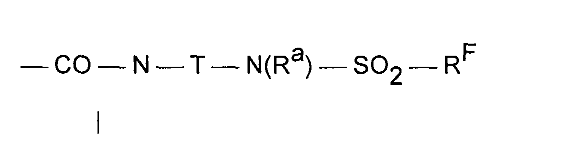 ou
1 à 2 groupes aryle éventuellement substitués, et/ou est interrompue par ces groupes, et/ou est éventuellement substituée par 1 à 3 groupes -OR^{a}, 1 à 2 groupes oxo, 1 à 2 groupes -NH-COR^{a}, 1 à 2 groupes -CONHR^{a}, 1 à 2 groupes -(CH₂)ₚ-CO₂H, 1 à 2 groupes -(CH₂)ₚ-(O)_{q}-CH₂CH₂-R^{F}, où R^{a} et R^{F} et p et q ont les significations données ci-dessus, et T représente une chaîne en C₂ à C₁₀, qui est éventuellement interrompue par 1 à 2 atomes d'oxygène ou 1 à 2 groupes -NHCO,
K est un complexant ou un complexe métallique, ou leurs sels de bases organiques et/ou inorganiques ou d'acides aminés ou des amides d'acides aminés, et plus précisément un complexant ou un complexe de formule générale II 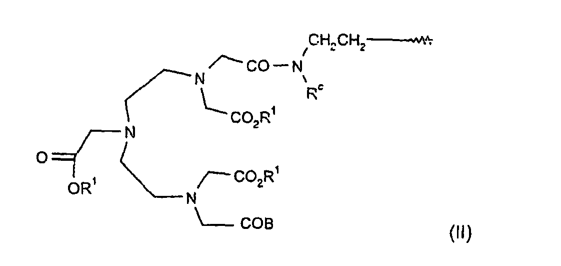
dans laquelle R^{c}, R¹ et B sont indépendants les uns des autres, et
R^{c} a les significations de R^{a} ou représente -(CH₂)ₘ-L-R^{F}, où m vaut 0, 1 ou 2, et L et R^{F} ont les significations données ci-dessus,
les radicaux R¹ représentent chacun indépendamment des autres un atome d'hydrogène ou un équivalent d'un ion métallique ayant les numéros atomiques 22-29, 42-46 ou 58-70,
B est -OR¹ ou 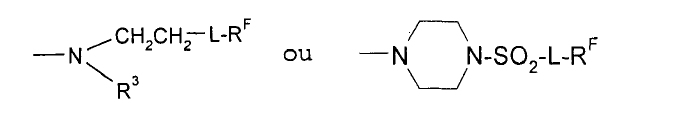
où R¹, L, R^{F} et R^{c} ont les significations données ci-dessus,
ou
un complexant ou un complexe de formule générale III 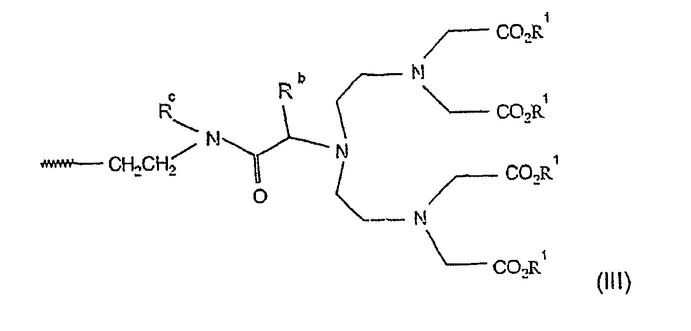 dans laquelle R^{c} et R¹ ont les significations données ci-dessus, et R^{b} a les significations de R^{a},
ou
un complexant ou un complexe de formule générale IV 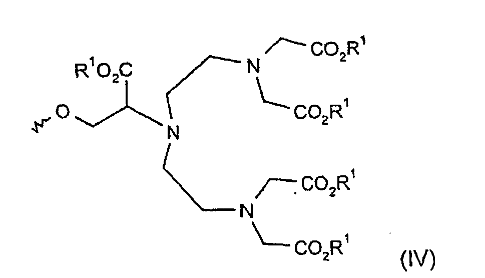 dans laquelle R¹ a les significations données ci-dessus,
ou
un complexant ou un complexe de formule générale V 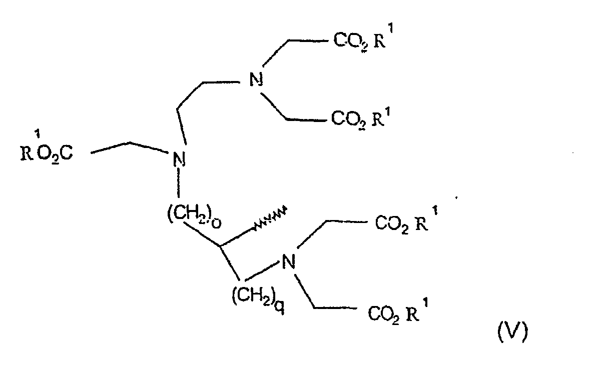 dans laquelle R¹ a les significations données ci-dessus et o et q représentent les nombres 0 ou 1, et la somme o + q vaut 1,
ou
un complexant ou un complexe de formule générale VI 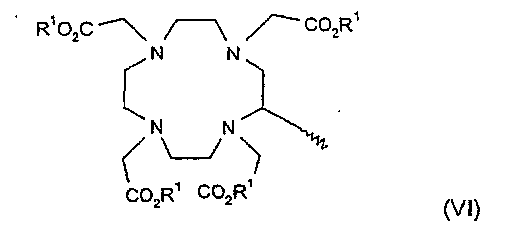 dans laquelle R¹ a les significations données ci-dessus,
ou
un complexant ou un complexe de formule générale VII 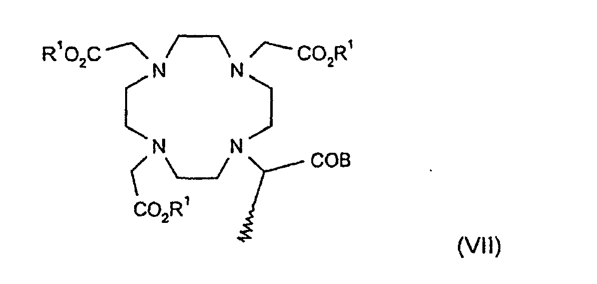 dans laquelle R¹ et B ont les significations données ci-dessus,
ou
un complexant ou un complexe de formule générale VIII 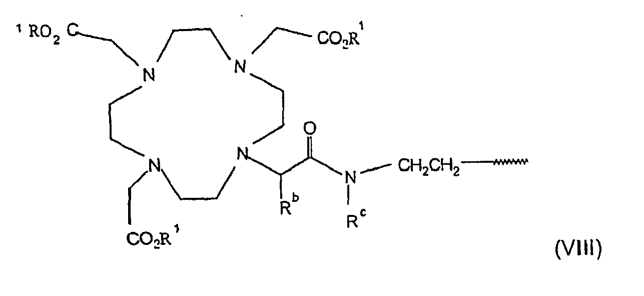 dans laquelle R^{c} et R¹ ont les significations données ci-dessus, et R^{b} a les significations données ci-dessus pour R^{a},
ou
un complexant ou un complexe de formule générale IX 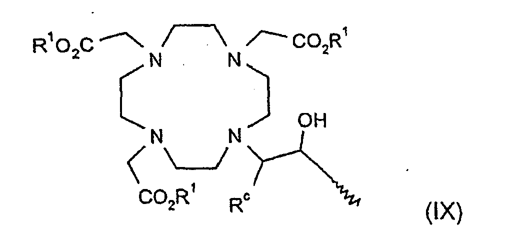 dans laquelle R^{c} et R¹ ont les significations données ci-dessus,
ou
un complexant ou un complexe de formule générale X 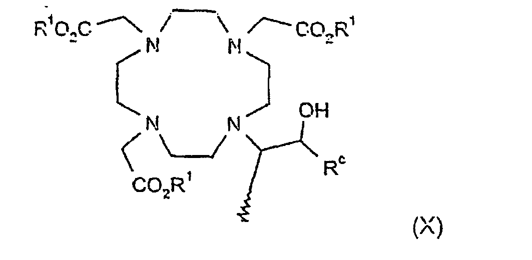 dans laquelle R^{c} et R¹ ont les significations données ci-dessus,
ou
un complexant ou un complexe de formule générale XI 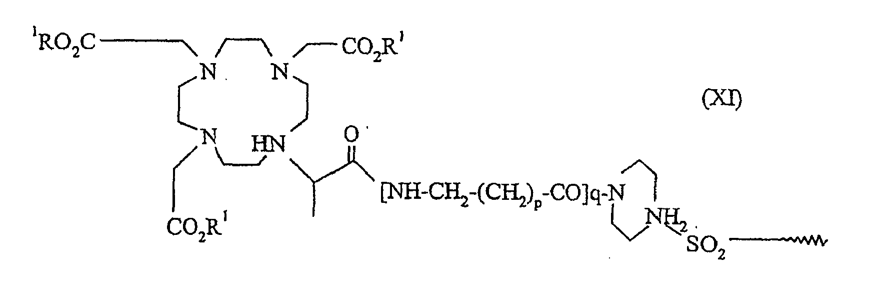 dans laquelle R¹, p et q ont les significations données ci-dessus, et R^{b} a les significations de R^{a},
ou
un complexant ou un complexe de formule générale XII 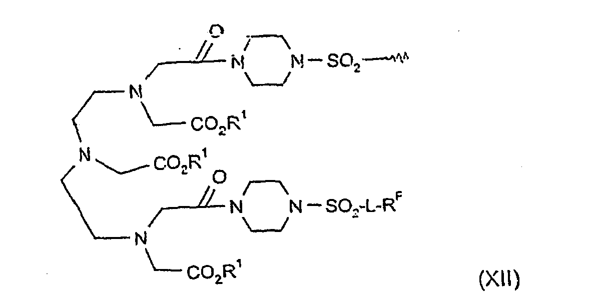 dans laquelle L, R^{F} et R¹ ont les significations données ci-dessus,
ou
un complexant ou un complexe de formule générale XIII 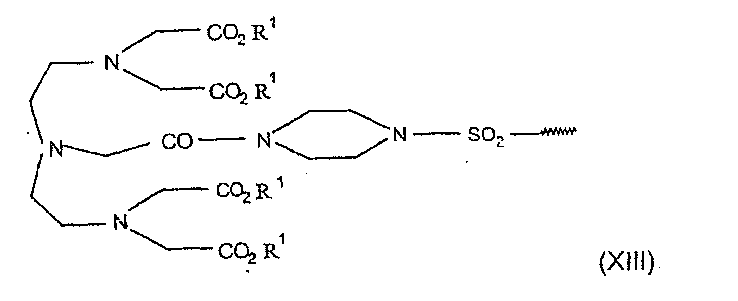 dans laquelle R¹ a les significations données ci-dessus.

9. Utilisation selon la revendication 8,
**caractérisée en ce qu'**on utilise les composés de formule générale I dans laquelle L représente
α-CH₂-β
α-CH₂CH₂-β
α-(CH₂)ₛ-β s = 3-15
α-CH₂-O-CH₂CH₂-β
α-CH₂-(O-CH₂-CH₂-)ₜ-β t = 2-6
α-CH₂-NH-CO-β
α-CH₂-NH-CO-CH₂-N(CH₂COOH)-SO₂-β
α-CH₂-NH-CO-CH₂-N(C₂H₅)-SO₂-β
α-CH₂-NH-CO-CH₂-N(C₁₀H₂₁)-SO₂-β
α-CH₂-NH-CO-CH₂-N(C₆H₁₃)-SO₂-β
α-CH₂-NH-CO-(CH₂)₁₀-N(C₂H₅)-SO₂-β
α-CH₂-NH-CO-CH₂-N(-CH₂-C₆H₅)-SO₂-β
α-CH₂-NH-CO-CH₂-N(-CH₂-CH₂-OH)SO₂-β
α-CH₂-NHCO-(CH₂)₁₀-S-CH₂CH₂-β
α-CH₂NHCOCH₂-O-CH₂CH₂-β
α-CH₂NHCO(CH₂)₁₀-O-CH₂CH₂-β
α-CH₂-C₆H₄-O-CH₂CH₂-β
α-CH₂-O-CH₂-C(CH₂-OCH₂CH₂-C₆F₁₃)₂-CH₂-OCH₂-CH₂-β 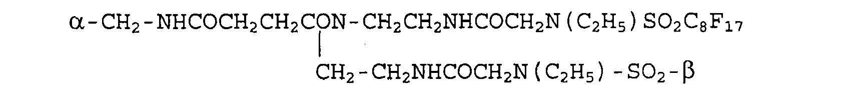 α -CH₂-O-CH₂-CH(OC₁₀H₂₁)-CH₂-O-CH₂CH₂-β
α-(CH₂NHCO)₄-CH₂O-CH₂CH₂-β
α-(CH₂NHCO)₃-CH₂O-CH₂CH₂-β
α-CH₂-OCH₂C(CH₂OH)₂-CH₂-O-CH₂CH₂-β 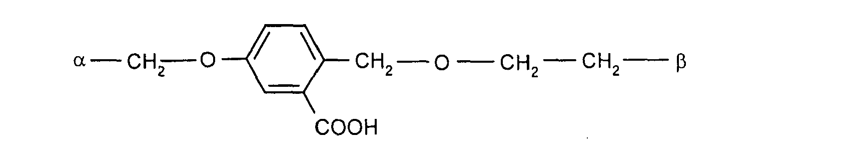 α -CH₂NHCOCH₂N(C₆H₅)-SO₂-β
α-NHCO-CH₂-CH₂-β
α-NHCO-CH₂-O-CH₂CH₂-β
α-NH-CO-β
α-NH-CO-CH₂-N(CH₂COOH)-SO₂-β
α-NH-CO-CH₂-N(C₂H₅)-SO₂-β
α-NH-CO-CH₂-N(C₁₀H₂₁)-SO₂-β
α-NH-CO-CH₂-N(C₆H₁₃)-SO₂-β
α-NH-CO-(CH₂)₁₀-N(C₂H₅)-SO₂-β
α-NH-CO-CH₂-N(-CH₂-C₆H₅)-SO₂-β
α-NH-CO-CH₂-N(-CH₂-CH₂-OH)SO₂-β
α-NH-CO-CH₂-β
α-CH₂-O-C₆H₄-O-CH₂-CH₂-β
α-CH₂-C₆H₄-O-CH₂-CH₂-β
α-N(C₂H₅)-SO₂-β
α-N(C₆H₅)-SO₂-β
α-N(C₁₀H₂₁)-SO₂-β
α-N(C₆H₁₃)-SO₂-β
α-N(C₂H₄OH)-SO₂-β
α-N(CH₂COOH)-SO₂-β
α-N(CH₂C₆H₅)-SO₂-β
α-N[CH (CH₂OH)₂]-SO₂-β
α-N-[CH(CH₂OH)CH(CH₂OH)]-SO₂-β
et où α représente le point de liaison au complexant ou au complexe métallique K, et β le point de liaison au radical fluoro.

10. Utilisation selon la revendication 8 ou 9, **caractérisée en ce qu'**on utilise les composés de formule I dans laquelle n, dans la formule -CₙF₂ₙE, est un nombre de 4 à 15, et/ou E, dans cette formule, est un atome de fluor.

11. Utilisation selon l'une des revendications 8 à 10, **caractérisée en ce qu'**on utilise les composés suivants :
- complexe de gadolinium du 10-[1-méthyl-2-oxo-3-aza-5-oxo-{4-perfluorooctylsulfonyl-pipérazin-1-yl}-pentyl]-1,4,7-tris(carboxyméthyl)-1,4,7,10-tétraazacyclododécane,
- complexe de gadolinium du 10-[2-hydroxy-4-aza-5-oxo-7-oxa-10,10,11,11,12,12,13,13,14,14,15,15,16,16,17,17-heptadécafluoroheptadécyl]-1,4,7-tris(carboxyméthyl)-1,4,7,10-tétraazacyclododécane,
- complexe de gadolinium du 10-[2-hydroxy-4-aza-5,9-dioxo-9-{4-pefluoroctyl)-pipérazin-1-yl}-nonyl]-1,4,7-tris(carboxyméthyl)-1,4,7,10-tétraazacyclododécane,
- complexe de gadolinium du 10-[2-hydroxy-4-aza-5-oxo-7-aza-7-(perfluoroctylsulfonyl)-nonyl]-1,4,7-tris(carboxyméthyl)-1,4,7,10-tétraazacyclododécane,
- complexe de gadolinium du 10-[2-hydroxy-4-oxa-1H,1H,2H,3H,3H,5H,5H,6H,6H-perfluoro-tétradécyl]-1,4,7-tris(carboxyméthyl)-1,4,7,10-tétraazacyclododécane,
- complexe de gadolinium du 10-[2-hydroxy-4-aza-5-oxo-7-oxa-10,10,11,11,12,12,13,13,14,14,15,15,16,16,17,17,18,18, 19,19-hénicosafluoro-nonadécyl]-1,4,7-tris(carboxyméthyl)-1,4,7,10-tétraazacyclododécane,
- complexe de gadolinium du 10-[2-hydroxy-4-aza-5-oxo-11-aza-11-(perfluoroctylsulfonyl)-tridécyl]-1,4,7-tris(carboxyméthyl)-1,4,7,10-tétraazacyclododécane,
- complexe de gadolinium du 10-[2-hydroxy-4-aza-5-oxo-7-aza-7-(perfluoroctylsulfonyl)-8-phényl-octyl]-1,4,7-tris(carboxyméthyl)-1,4,7,10-tétraazacyclododécane.

12. Utilisation selon l'une des revendications 1 à 7, **caractérisée en ce qu'**on utilise en tant que complexes métalliques perfluoralkylés les composés de formule générale Ia
A-R^{F} (Ia)
dans laquelle
• A est une partie de molécule qui contient 2 à 6 complexes métalliques, qui sont liés directement, ou par l'intermédiaire d'un élément de liaison, à un atome d'azote d'un squelette cyclique,
et
• R^{F} est une chaîne carbonée perfluorée droite, ou ramifiée de formule -CₙF₂ₙE, dans laquelle
E représente un atome de fluor, de chlore, de brome, d'iode ou d'hydrogène terminal,
et n est un nombre de 4 à 30,
où la partie de molécule A présente la structure suivante : 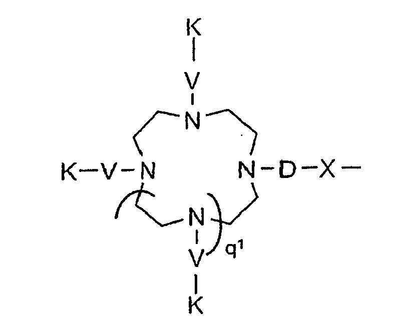 dans laquelle
• q¹ est un nombre valant 0, 1, 2 ou 3,
• K est un complexant ou un complexe métallique, ou leurs sels de bases organiques et/ou inorganiques ou d'acides aminés, ou des amides d'acides aminés,
• X est une liaison directe au groupe perfluoralkyle, un groupe phénylène ou une chaîne alkylène en C₁ à C₁₀, qui contient éventuellement 1 à 15 atomes d'oxygène, 1 à 5 atomes de soufre, 1 à 10 groupes carbonyle, 1 à 10 groupes (NR^{d})-, 1 à 2 groupes NR^{d}SO₂-, 1 à 10 groupes CONR^{d}-, 1 groupe pipéridine, 1 à 3 groupes SO₂-, 1 à 2 groupes phénylène, ou est éventuellement substitué par 1 à 3 radicaux R^{F}, où R^{d} représente un atome d'hydrogène, un groupe phényle, benzyle, ou un groupe alkyle en C₁ à C₁₅ qui contient éventuellement 1 à 2 groupes NHCO, 1 à 2 groupes CO, 1 à 5 atomes d'oxygène, et est éventuellement substitué par 1 à 5 radicaux hydroxy, 1 à 5 radicaux méthoxy, 1 à 3 radicaux carboxy, 1 à 3 radicaux R^{F},
• V est une liaison directe ou une chaîne de formules générales IIa ou IIIa, 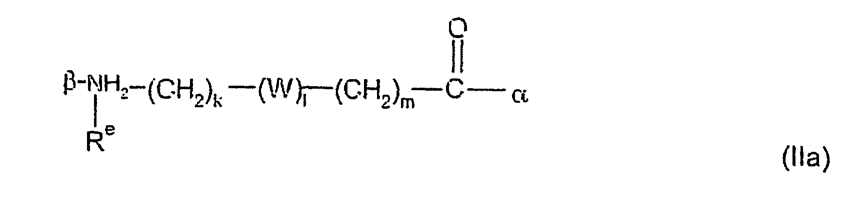 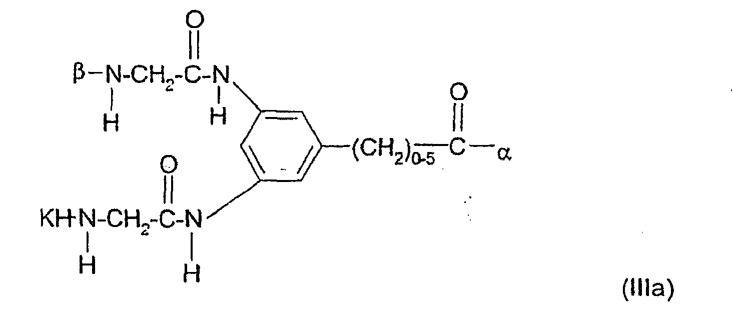
dans lesquelles
■ R^{e} est un atome d'hydrogène, un groupe phényle, un groupe benzyle, ou un groupe alkyle en C₁ à C₇, qui est éventuellement substitué par un groupe carboxy, un groupe méthoxy ou un groupe hydroxy,
■ W est une liaison directe, un groupe polyglycoléther ayant jusqu'à 5 motifs glycol, ou une partie de molécule de formule générale IVa
-CH(R^{h})- (IVa)
dans laquelle R^{h} est un acide carboxylique en C₁ à C₇, un groupe phényle, un groupe benzyle ou un groupe -(CH₂)₁₋₅-NH-K,
■ α représente la liaison à l'atome d'azote du squelette, β représente la liaison au complexant ou au complexe métallique K,
■ et dans lesquelles les variables k et m sont des nombres entiers de 0 à 10, et 1 vaut 0 ou 1,
et où
o D représente un groupe CO ou SO₂.

13. Utilisation selon la revendication 12, **caractérisée en ce qu'**on utilise les composés de formule générale Ia dans laquelle q est le nombre 1.

14. Utilisation selon la revendication 12, **caractérisée en ce qu'**on utilise les composés de formule générale Ia dans laquelle la partie de molécule X est une chaîne alkylène, qui contient 1 à 10 groupes CH₂CH₂O ou 1 à 5 groupes COCH₂NH, une liaison directe, ou l'une des structures suivantes : γ-CH₂-O-(CH₂)₂-δ, 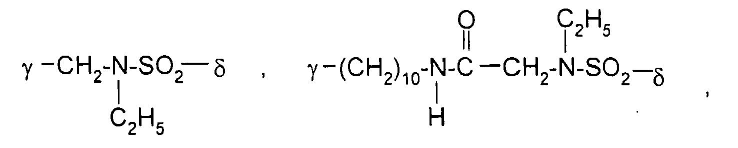 γ -(CH₂)₁₀-O-(CH₂)₂-δ 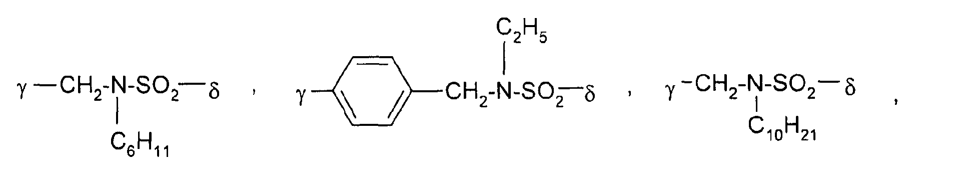 où γ assure la liaison à D et δ à R^{F}.

15. Utilisation selon la revendication 12, **caractérisée en ce qu'**on utilise les composés de formule générale Ia dans laquelle V est une partie de molécule ayant l'une des structures suivantes :

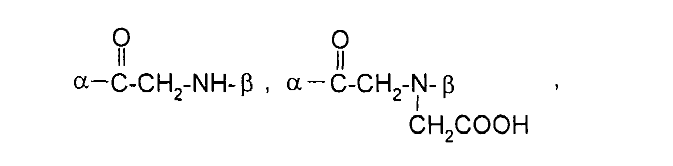

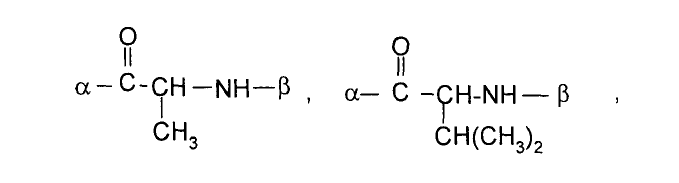

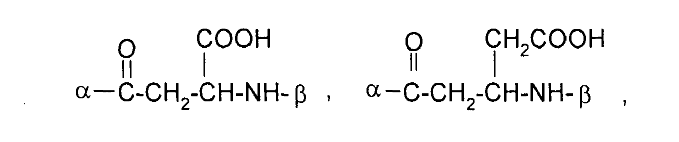

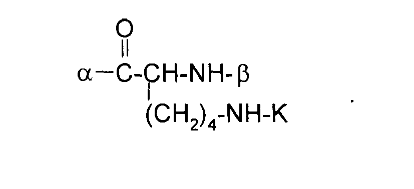

16. Utilisation selon la revendication 12, **caractérisée en ce qu'**on utilise les composés de formule générale Ia dans laquelle K représente un complexe de formule générale Va, VIa, VIIa ou VIIIa 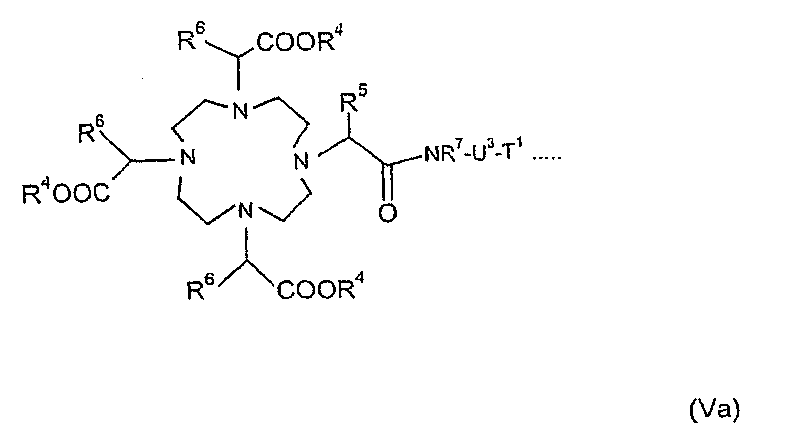 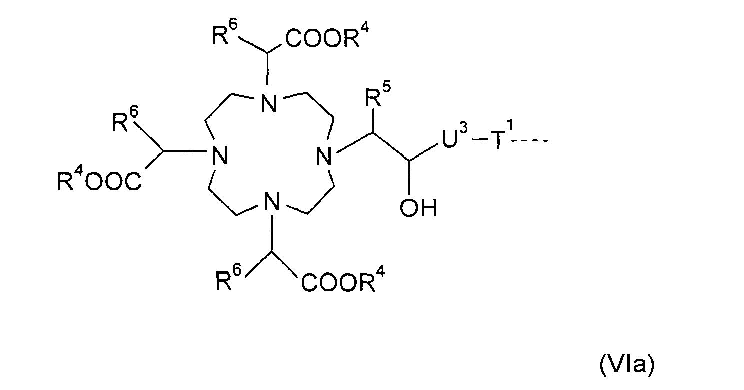 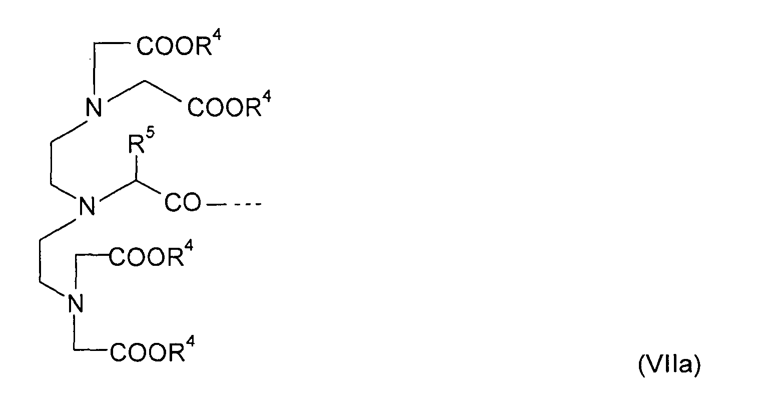 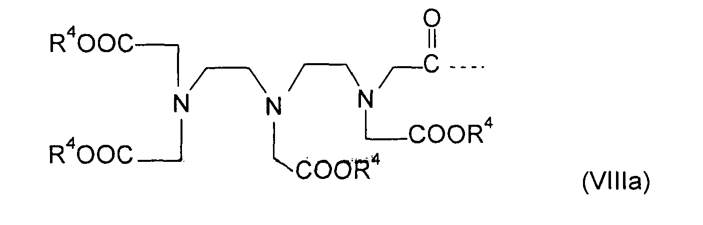 dans lesquelles
• les radicaux R⁴ représentent chacun indépendamment des autres un atome d'hydrogène ou un équivalent d'un ion métallique des éléments ayant les numéros atomiques 23-29, 42-46 ou 58-70,
• R⁵ est un atome d'hydrogène ou une chaîne alkyle en C₁ à C₃₀ droite ou ramifiée, saturée ou insaturée, qui est éventuellement substituée par 1 à 5 groupes hydroxy, 1 à 3 groupes carboxy ou un groupe phényle, et/ou est éventuellement interrompue par 1 à 10 atomes d'oxygène, un groupe phénylène ou un groupe phénylène-oxy,
• R⁶ est un atome d'hydrogène, un radical alkyle en C₁ à C₇ à chaîne droite ou ramifiée, un radical phényle ou benzyle,
• R⁷ est un atome d'hydrogène, un groupe méthyle ou éthyle, qui est éventuellement substitué par un groupe hydroxy ou carboxy,
• U³ est un groupe alkylène en C₁ à C₂₀ à chaîne droite ou ramifiée, saturé ou insaturé, contenant éventuellement 1 à 5 groupes imino, 1 à 3 groupes phénylène, 1 à 3 groupes phénylène-oxy, 1 à 3 groupes phénylène-imino, 1 à 5 groupes amido, 1 à 2 groupes hydrazido, 1 à 5 groupes carbonyle, 1 à 5 groupes éthylène-oxy, 1 groupe urée, 1 groupe thiourée, 1 à 2 groupes carboxyalkylimino, 1 à 2 groupes ester, 1 à 10 atomes d'oxygène, 1 à 5 atomes de soufre et/ou 1 à 5 atomes d'azote, et/ou éventuellement substitué par 1 à 5 groupes hydroxy, 1 à 2 groupes mercapto, 1 à 5 groupes oxo, 1 à 5 groupes thioxo, 1 à 3 groupes carboxy, 1 à 5 groupes carboxyalkyle, 1 à 5 groupes ester et/ou 1 à 3 groupes amino, les groupes phénylène éventuellement contenus pouvant être substitués par 1 à 2 groupes carboxy, 1 à 2 groupes sulfone ou 1 à 2 groupes hydroxy,
• T¹ représente un groupe -CO-β, -NHCO-β ou -NHCS-β, β étant le point de liaison à V.

17. Utilisation selon la revendication 16, **caractérisée en ce que** la chaîne alkylène en C₁ à C₂₀ représentant U³ contient les groupes -CH₂NHCO-, -NHCOCH₂O-, -NHCOCH₂OC₆H₄-, -N(CH₂CO₂H)-, -CH₂OCH₂-, -NHCOCH₂C₆H₄-, -NHCSNHC₆H₄-, -CH₂OC₆H₄-, -CH₂CH₂O-, et/ou est substituée par les groupes -COOH, -CH₂COOH.

18. Utilisation selon la revendication 16, **caractérisée en ce que** U³ est un groupe -CH₂-, -CH₂CH₂-, -CH₂CH₂CH₂-, -C₆H₄-, C₆H₁₀-, -CH₂C₆H₄-, -CH₂NHCOCH₂CH(CH₂CO₂H)-C₆H₄-, -CH₂NHCOCH₂OCH₂-, -CH₂NHCOCH₂C₆H₄-.

19. Utilisation selon la revendication 12, **caractérisée en ce qu'**on utilise les composés de formule générale Ia dans laquelle K a l'une des structures suivantes :

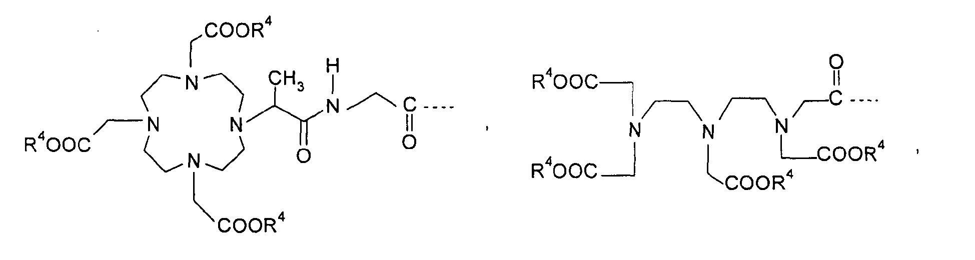

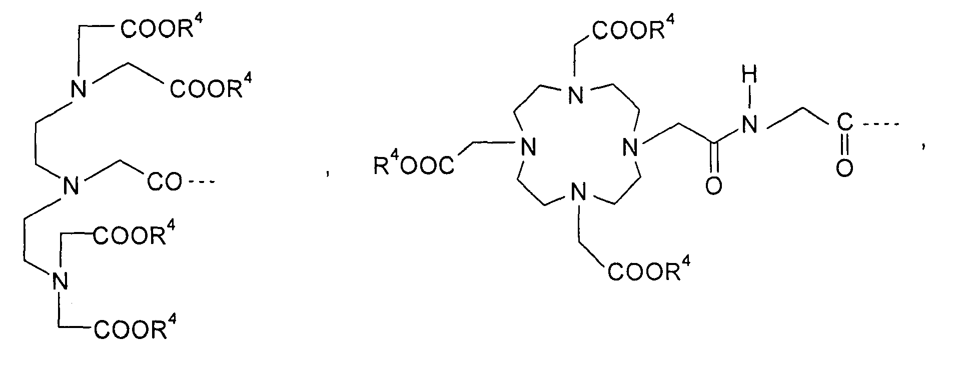

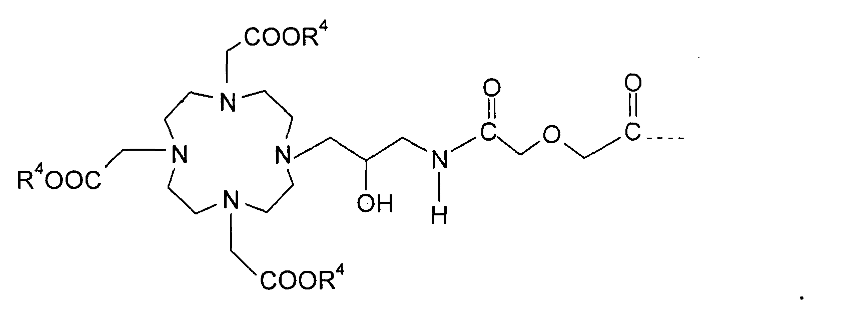

20. Utilisation selon l'une des revendications 12 à 19, **caractérisée en ce qu'**on utilise les composés de formule générale Ia dans laquelle la chaîne perfluoralkyle R^{F} est -C₆F₁₃, -C₈F₁₇, -C₁₀F₂₁ ou -C₁₂F₂₅.

21. Utilisation selon l'une des revendications 12 à 20, **caractérisée en ce qu'**on utilise le complexe de gadolinium du 1,4,7-Tris{complexe de Gd du 1,4,7-tris(N-(carboxylatométhyl)-10-[N-1-méthyl-3,6-diaza-2,5,8-trioxooctane-1,8-diyl)]-1,4,7,10-tétraazacyclododécane}-10-[N-2H,2H,4H,4H,5H,5H-3-oxa-perfluorotridécanoyl]-1,4,7,10-tétraazacyclododécane.

22. Utilisation selon l'une des revendications 1 à 7, **caractérisée en ce qu'**on utilise en tant que complexes métalliques perfluoralkylés les composés de formule générale Ib 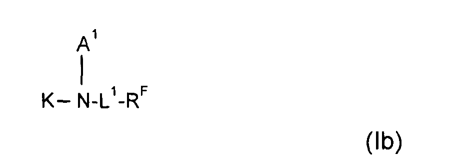 dans laquelle
K est un complexant ou un complexe métallique de formule générale IIb 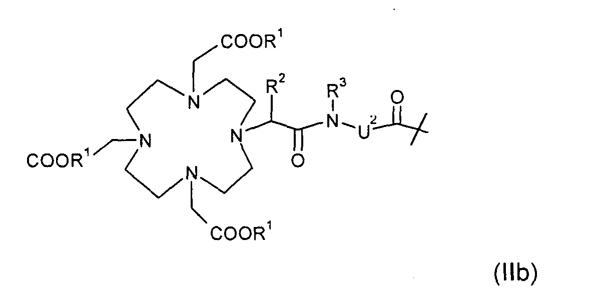 dans laquelle
R¹ est un atome d'hydrogène ou un équivalent d'un ion métallique ayant les numéros atomiques 23 à 29, 42 à 46 ou 58 à 70,
R² et R³ représentent chacun un atome d'hydrogène, un groupe alkyle en C₁ à C₇, un groupe benzyle, un groupe phényle, -CH₂OH ou -CH₂-OCH₃,
U² est le radical L¹, mais où L¹ et U² peuvent, indépendamment l'un de l'autre, être identiques ou différents,
A¹ est un atome d'hydrogène, un groupe alkyle en C₁ à C₃₀ à chaîne droite ou ramifiée, qui est éventuellement interrompu par 1 à 15 atomes d'oxygène et/ou est éventuellement substitué par 1 à 10 groupes hydroxy, 1 à 2 groupes COOH, un groupe phényle, un groupe benzyle et/ou 1 à 5 groupes OR^{g}, R^{g} ayant pour signification un atome d'hydrogène ou un radical alkyle en C₁ à C₇, ou encore -L¹-R^{F},
L¹ est un groupe alkylène en C₁ à C₃₀ à chaîne droite ou ramifiée, qui est éventuellement interrompu par 1 à 10 atomes d'oxygène, 1 à 5 groupes -NH-CO, par 1 à 5 groupes -CO-NH, par un groupe phénylène éventuellement substitué par un groupe COOH, par 1 à 3 atomes de soufre, par 1 à 2 groupes -N(B¹)-SO₂ et/ou 1 à 2 groupes -SO₂-N(B¹), B¹ ayant les significations de A¹ et/ou étant éventuellement substitué par le radical R^{F}, et
R^{F} est un radical alkyle perfluoré à chaîne droite ou ramifiée de formule CₙF₂ₙE dans laquelle n est un nombre de 4 à 30 et E est un atome de fluor, de chlore, de brome ou d'iode terminal, ou un atome d'hydrogène,
et les groupes acides éventuellement présents peuvent éventuellement se présenter sous forme de sels de bases organiques et/ou inorganiques ou d'acides aminés, ou d'amides d'acides aminés.

23. Utilisation selon la revendication 22, **caractérisée en ce qu'**on utilise les composés de formule générale Ib dans laquelle R², R³ et R^{g} représentent chacun indépendamment des autres un atome d'hydrogène ou un groupe alkyle en C₁ à C₄.

24. Utilisation selon la revendication 22,
**caractérisée en ce qu'**on utilise les composés de formule générale Ib dans laquelle A¹ est un atome d'hydrogène, un radical alkyle en C₁ à C₁₅, les radicaux
C₂H₄-O-CH₃,
C₃H₆-O-CH₃,
C₂H₄-O-(C₂H₄-O)ₜ-C₂H₄-OH,
C₂H₄-O-(C₂H₄-O)ₜ-C₂H₄-OCH₃,
C₂H₄-OH,
C₃H₆OH,
C₄H₈OH,
C₅H₁₀OH,
C₆H₁₂OH,
C₇H₁₄OH,
CH(OH)CH₂OH,
CH(OH)CH(OH)CH₂OH,
CH₂[CH(OH)]ᵤ¹CH₂OH,
CH[CH₂(OH)]CH(OH)CH₂OH,
C₂H₄CH(OH)CH₂OH,
(CH₂)ₛCOOH,
C₂H₄-O-(C₂H₄-O)ₜ-CH₂COOH ou
C₂H₄-O-(C₂H₄-O)ₜ-C₂H₄-CₙF₂ₙE,
où
s est un nombre entier de 1 à 15,
t est un nombre entier de 0 à 13,
u¹ est un nombre entier de 1 à 10,
n est un nombre entier de 4 à 20, et
E est un atome d'hydrogène, de fluor, de chlore, de brome ou d'iode,
et aussi, si cela est possible, leurs isomères ramifiés.

25. Utilisation selon la revendication 22,
**caractérisée en ce qu'**on utilise les composés de formule générale Ib dans laquelle A¹ est un atome d'hydrogène, un groupe alkyle en C₁ à C₁₀,
C₂H₄-O-CH₃,
C₃H₆-O-CH₃,
C₂H₄-O-(C₂H₄-O)ₓ-C₂H₄-OH,
C₂H₄-O-(C₂H₄-O)ₓ-C₂H₄-OCH₃,
C₂H₄OH,
C₃H₆OH,
CH₂[CH(OH)]_{y}CH₂OH,
CH[CH₂(OH)]CH(OH)CH₂OH,
(CH₂)_{w}COOH,
C₂H₄-O-(C₂H₄-O)ₓ-CH₂COOH,
C₂H₄-O-(C₂H₄-O)ₓ-C₂H₄-CₙF₂ₙE,
où
x est un nombre entier de 0 à 5,
y est un nombre entier de 1 à 6,
w est un nombre entier de 1 à 10,
n est un nombre entier de 4 à 15, et
E est un atome de fluor, et aussi, si cela est possible, leurs isomères ramifiés.

26. Utilisation selon la revendication 22, **caractérisée en ce qu'**on utilise les composés de formule générale Ib dans laquelle L¹ est
α-(CH₂)ₛ-β
α-CH₂-CH₂-(O-CH₂-CH₂-)_{y}-β
α-CH₂-(O-CH₂-CH₂-)_{y}-β,
α-CH₂-NH-CO-β
α-CH₂-CH₂-NH-SO₂-β
α-CH₂-NH-CO-CH₂-N(CH₂COOH)-SO₂-β
α-CH₂-NH-CO-CH₂-N(C₂H₅)-SO₂-β
α-CH₂-NH-CO-CH₂-N(C₁₀H₂₁)-SO₂-β
α-CH₂-NH-CO-CH₂-N(C₆H₁₃)-SO₂-β
α-CH₂-NH-CO-(CH₂)₁₀-N(C₂H₅)-SO₂-β
α-CH₂-NH-CO-CH₂-N(-CH₂-C₆H₅)-SO₂-β
α-CH₂-NH-CO-CH₂-N(-CH₂-CH₂-OH)SO₂-β
α-CH₂-NHCO-(CH₂)₁₀-S-CH₂CH₂-β
α-CH₂NHCOCH₂-O-CH₂CH₂-β
α-CH₂-CH₂NHCOCH₂-O-CH₂CH₂-β
α-CH₂-(CH₂-CH₂-O)ₜ-(CH₂)₃NHCO-CH₂-O-CH₂CH₂-β
α-CH₂NHCO(CH₂)₁₀-O-CH₂CH₂-β
α-CH₂CH₂NHCO(CH₂) ₁₀-O-CH₂CH₂-β
α-CH₂-C₆H₄-O-CH₂CH₂-β où le groupe phénylène est lié en positions 1,4 ou 1,3
α-CH₂-O-CH₂-C(CH₂-OCH₂CH₂-C₆F₁₃)₂-CH₂-OCH₂-CH₂-β
α-CH₂-NHCOCH₂CH₂CON-CH₂CH₂NHCOCH₂N(C₂H₅)SO₂C₈F₁₇β
α-CH₂-CH₂NHCOCH₂N(C₂H₅)-SO₂-β
α-CH₂-O-CH₂-CH(OC₁₀H₂₁)-CH₂-O-CH₂CH₂-β
α-(CH₂NHCO)₄-CH₂O-CH₂CH₂-β
α-(CH₂NHCO)₃-CH₂O-CH₂CH₂-β
α-CH₂-OCH₂C(CH₂OH)₂-CH₂-O-CH₂CH₂-β 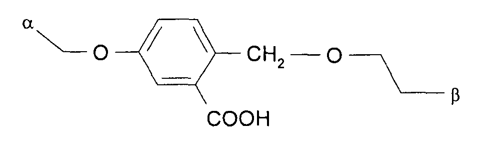 α -CH₂NHCOCH₂N(C₆H₅)-SO₂-β
α-NHCO-CH₂-CH₂-β
α-NHCO-CH₂-O-CH₂CH₂-β
α-NH-CO-β
α-NH-CO-CH₂-N(CH₂COOH)-SO₂-β
α-NH-CO-CH₂-N(C₂H₅)-SO₂-β
α-NH-CO-CH₂-N(C₁₀H₂₁)-SO₂-β
α-NH-CO-CH₂-N(C₆H₁₃)-SO₂-β
α-NH-CO-(CH₂)₁₀-N(C₂H₅)-SO₂-β
α-NH-CO-CH₂-N(-CH₂-C₆H₅)-SO₂-β
α-NH-CO-CH₂-N(-CH₂-CH₂-OH)SO₂-β
α-NH-CO-CH₂-β
α-CH₂-O-C₆H₄-O-CH₂-CH₂-β
α-CH₂-C₆H₄-O-CH₂-CH₂-β
α-N(C₂H₅)-SO₂-β
α-N(C₆H₅)-SO₂-β
α-N(C₁₀H₂₁)-SO₂-β
α-N(C₆H₁₃)-SO₂-β
α-N(C₂H₄OH)-SO₂-β
α-N(CH₂COOH)-SO₂-β
α-N(CH₂C₆H₅)-SO₂-β
α-N([CH(CH₂OH)₂]-SO₂-β
α-N-[CH(CH₂OH)CH (OH) (CH₂OH)]-SO₂-β
où
s est un nombre entier de 1 à 15, et
y est un nombre entier de 1 à 6.

27. Utilisation selon la revendication 22, **caractérisée en ce qu'**on utilise les composés de formule générale Ib dans laquelle L¹ est
α-CH₂-O-CH₂CH₂-β,
α-CH₂-CH₂-(O-CH₂-CH₂-)_{y}-β,
α-CH₂-(O-CH₂-CH₂-)_{y}-β,
α-CH₂-CH₂-NH-SO₂-β, exemple 10
α-CH₂NHCOCH₂-O-CH₂CH₂-β,
α-CH₂-CH₂NHCOCH₂-O-CH₂CH₂-β,
α-CH₂-(CH₂-CH₂-O)_{y}-(CH₂)₃NHCO-CH₂-O-CH₂CH₂-β,
α-CH₂NHCO(CH₂)₁₀-O-CH₂CH₂-β,
α-CH₂CH₂NHCO(CH₂)₁₀-O-CH₂CH₂-β,
α-CH₂-O-CH₂-CH(OC₁₀H₂₁)-CH₂-O-CH₂CH₂-β
α-CH₂-O-C₆H₄-O-CH₂-CH₂-β ou
α-CH₂-C₆H₄-O-CH₂-CH₂-β,
où
y est un nombre entier de 1 à 6.

28. Utilisation selon la revendication 22, **caractérisée en ce qu'**on utilise les composés de formule générale Ib dans laquelle R^{F} est un radical alkyle perfluoré à chaîne droite ou ramifiée de formule CₙF₂ₙE, dans laquelle n est un nombre de 4 à 15 et E est un atome de fluor terminal.

29. Utilisation selon l'une des revendications 22 à 28, **caractérisée en ce qu'**on utilise les composés suivants :
- Complexe de gadolinium du 1,4,7-tris(carboxylatométhyl)-10-[N-(2,3-dihydroxypropyl)-N-(1H,1H,2H, 2H,4H,4H,5H,5H-3-oxa)-perfluorotridécyl)-amide de l'acide (3-aza-4-oxo-hexan-5-ylique)]-1,4,7,10-tétraazacyclododécane,
- Complexe de gadolinium du 1,4,7-tris(carboxylatométhyl)-10-{N-(3,6,9,12,15-pentaoxa)-hexadécyl)-(1H,1H,2H,2H,4H,4H,5H,5H-3-oxa)-perfluorotridécyl]-amide de l'acide (3-aza-4-oxo-hexan-5-ylique)}-1,4,7,10-tétraazacyclododécane,
- Complexe de gadolinium du 1,4,7-tris(carboxylatométhyl)-10-{N-(5-hydroxy-3-oxa-pentyl)-N-(1H,1H, 2H,2H,4H,4H,5H,5H-3-oxa)-perfluorotridécyl]-amide de l'acide (3-aza-4-oxo-hexan-5-ylique)}-1,4,7,10-tétraazacyclododécane,
- Complexe de gadolinium du 1,4,7-tris(carboxylatométhyl)-10-{[N-3,6,9,15-tétraoxa-12-aza-15-oxo-C₁₇-C₂₆-hepta-décafluoro)hexacosyl]-amide de l'acide (3-aza-4-oxo-hexan-5-ylique) }-1,4,7,10-tétraazacyclododécane,
- Complexe de gadolinium du 1,4,7-tris(carboxylatométhyl)-10-[N-(2-méthoxyéthyl)-N-(1H,1H,2H,2H,4H, 4H,5H,5H-3-oxa)-perfluorotridécyl]-amide de l'acide (3-aza-4-oxo-hexan-5-ylique]}-1,4,7,10-tétraazacyclododécane.

30. Utilisation selon l'une des revendications 1 à 7, **caractérisée en ce qu'**on utilise en tant que complexes métalliques perfluoralkylés les composés comportant des résidus sucre de formule générale Ic 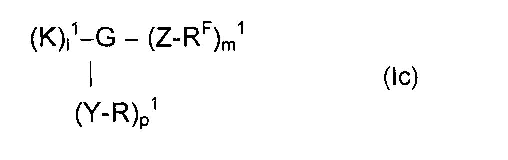 dans laquelle
R est un résidu mono- ou oligosaccharidique lié par l'intermédiaire de la position 1-OH ou 1-SH,
R^{F} est une chaîne carbonée perfluorée droite ou ramifiée de formule -CₙF₂ₙE dans laquelle E est un atome de fluor, de chlore, de brome, d'iode ou d'hydrogène terminal, et n est un nombre de 4 à 30,
K est un complexe métallique de formule générale IIc 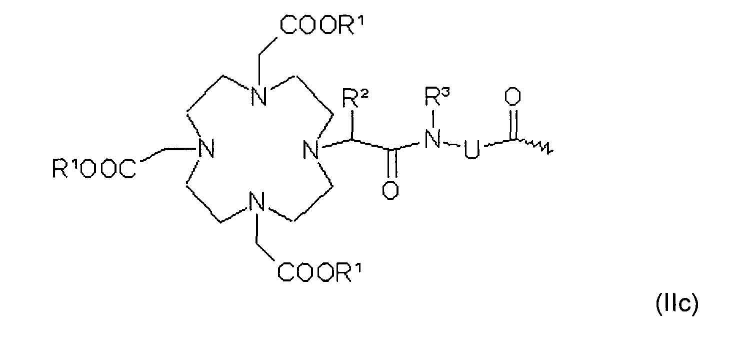 dans laquelle
R¹ est un atome d'hydrogène ou un équivalent d'un ion métallique ayant les numéros atomiques 23 à 29, 42 à 46 ou 58 à 70, à la condition qu'au moins deux radicaux R¹ soient des équivalents d'ions métalliques,
R² et R³ représentent chacun indépendamment de l'autre un atome d'hydrogène, un groupe alkyle en C₁ à C₇, benzyle, phényle, -CH₂OH ou -CH₂OCH₃, et
U est un groupe -C₆H₄-O-CH₂-ω, -(CH₂)₁₋₅-ω, un groupe phénylène, -CH₂-NHCO-CH₂-CH(CH₂COOH)-C₆H₄-ω-, -C₆H₄-OCH₂CH₂)₀₋₁-N(CH₂COOH)-CH₂-ω, ou un groupe alkylène en C₁ à C₁₂ ou (C₇ à C₁₂)-C₆H₄-O-, éventuellement interrompu par un ou plusieurs atomes d'oxygène, 1 à 3 groupes -NHCO-, 1 à 3 groupes -CONH- et/ou substitué par 1 à 3 groupes -(CH₂)₀₋₅COOH, ω représentant le point de liaison à -CO-,
ou
de formule générale IIIc 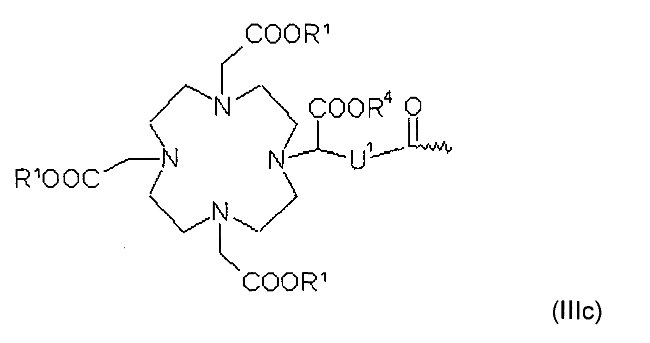 dans laquelle R¹ a les significations données ci-dessus, R⁴ est un atome d'hydrogène ou représente un équivalent d'un ion métallique mentionné pour R¹, et U¹ est -C₆H₄-O-CH₂-ω-, ω étant le point de liaison à -CO-,
ou de formule générale IVc 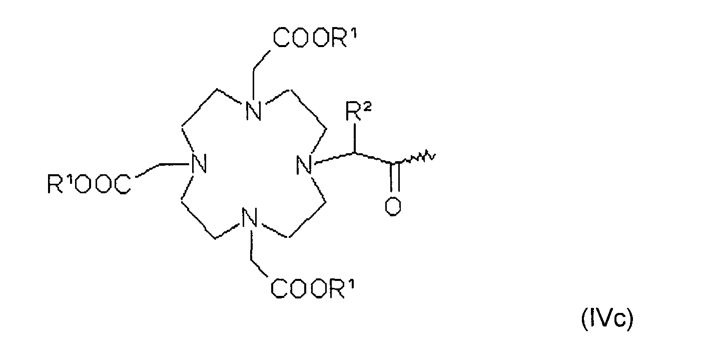 dans laquelle R¹ et R² ont les significations données ci-dessus,
ou de formules générales VcA ou VcB 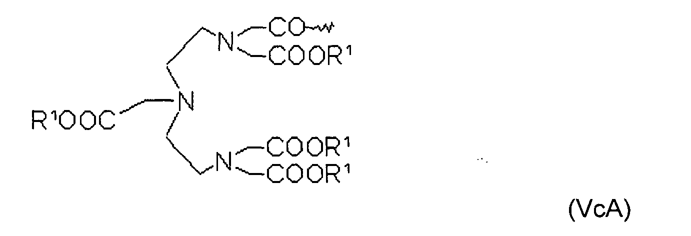 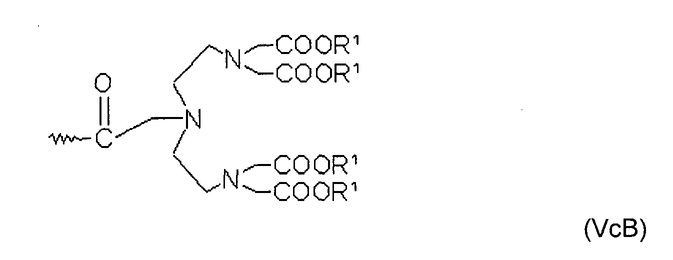 dans lesquelles R¹ a les significations données ci-dessus,
ou de formule générale VIc 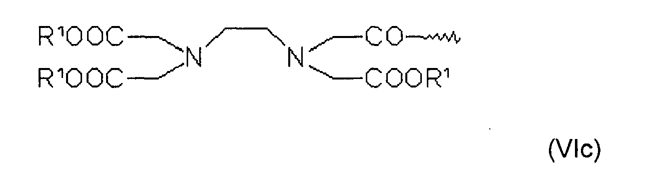 dans laquelle R¹ a les significations données ci-dessus,
ou de formule générale VIIc 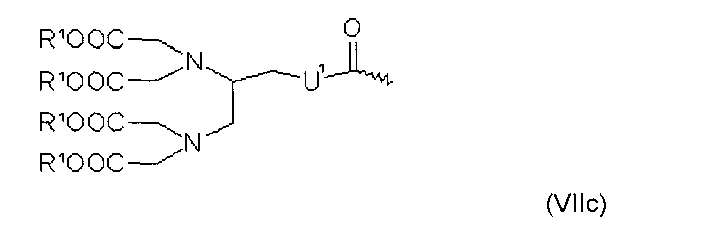 dans laquelle R¹ a les significations données ci-dessus, et U¹ est -C₆H₄-O-CH₂-ω-, ω étant le point de liaison à -CO-,
ou de formule générale VIIIc 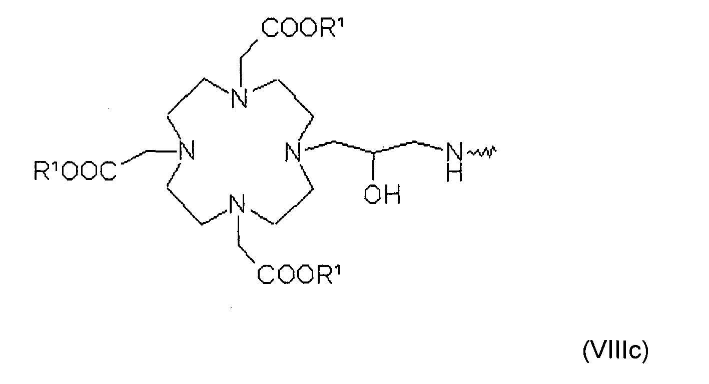 dans laquelle R¹ a les significations données ci-dessus,
et les groupes acides libres éventuellement présents dans le radical K peuvent se présenter éventuellement sous forme de sels de bases organiques et/ou inorganiques ou d'acides aminés,
ou d'amides d'acides aminés,
dans le cas dans lequel K désigne les complexes métalliques IIc à VIIc, représente un radical au moins trifonctionnalisé, choisi parmi les radicaux a) à j) ci-après : 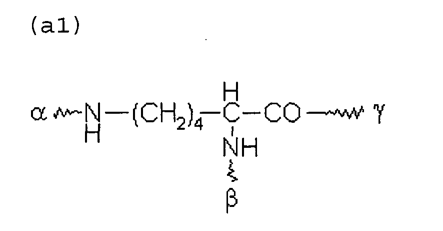 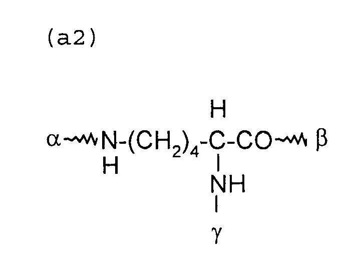 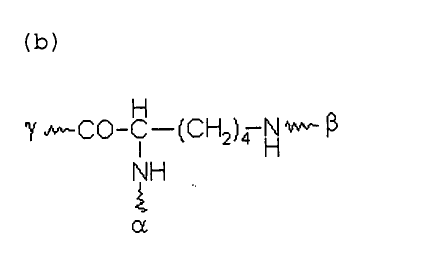 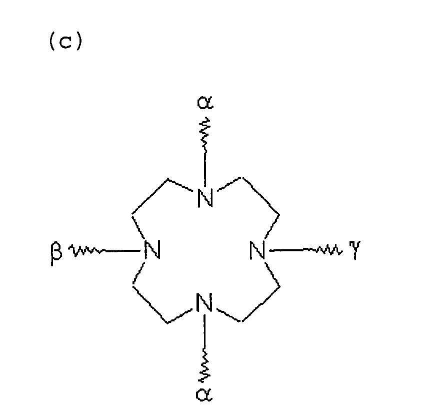 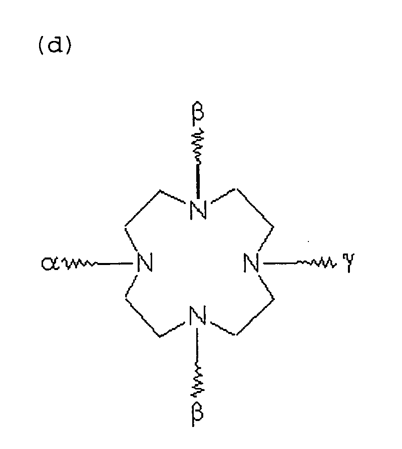 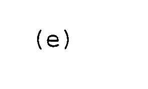 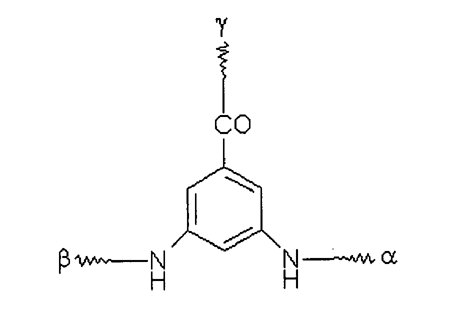 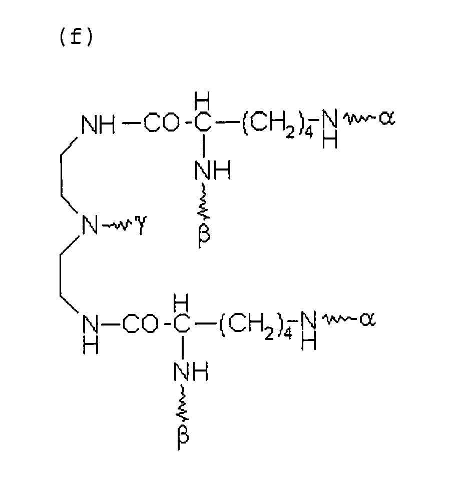 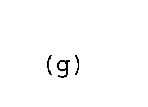 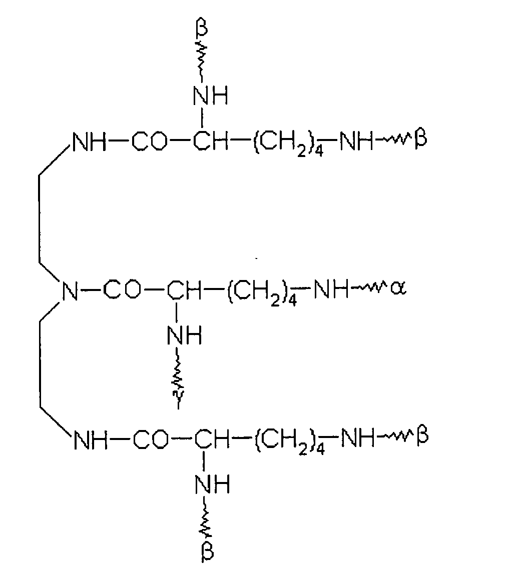 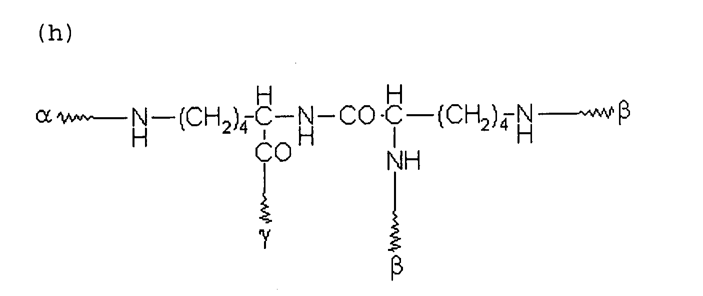 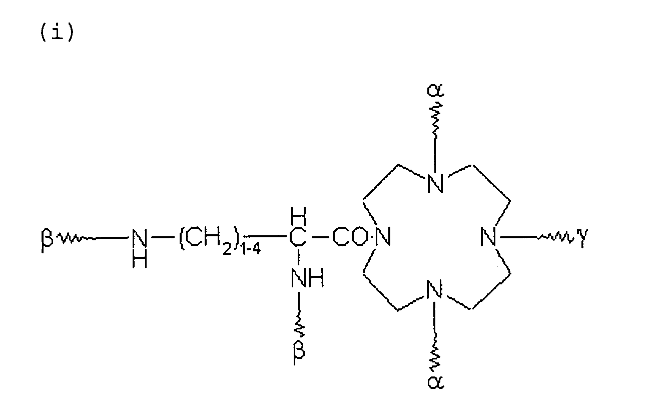 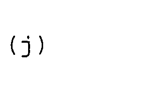 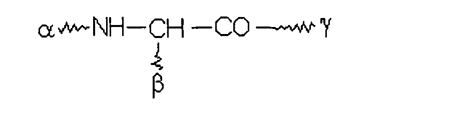 et
G, dans le cas dans lequel K est le complexe métallique VIIIc, représente un radical au moins trifonctionnalisé choisi parmi (k) ou (1), 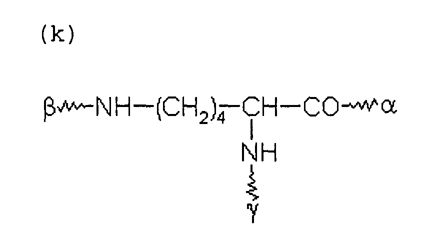 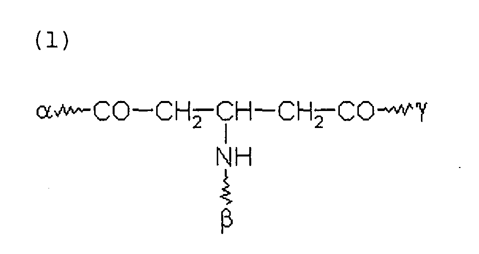 où α est le point de liaison de G au complexe K, β est le point de liaison de G au radical Y, et γ est le point de liaison de G au radical Z,
Y est -CH₂-, δ- (CH₂)₍₁₋₅₎CO-β, β- (CH₂)₍₁₋₅₎CO-δ, δ-CH₂-CHOH-CO-β ou δ-CH(CHOH-CH₂OH)-CHOH-CHOH-CO-β, où δ est le point de liaison au résidu sucre R, et β est le point de liaison au radical G,
Z est 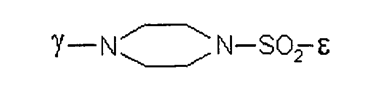 γ -COCH₂-N(C₂H₅)-SO₂-ε,
γ-COCH₂-O-(CH₂)₂-SO₂-ε, 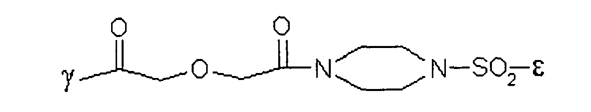 ou
γ-NHCH₂CH₂-O-CH₂CH₂-ε
où γ est le point de liaison de Z au radical G et ε est le point de liaison de Z au radical perfluoré R^{F},
et
l¹ et m¹ représentent chacun indépendamment de l'autre le nombre entier 1 ou 2, et
p¹ est un nombre entier de 1 à 4.

31. Utilisation selon la revendication 30, **caractérisée en ce qu'**on utilise les composés de formule générale Ic dans laquelle R est un résidu monosaccharidique ayant 5 à 6 atomes de carbone ou son composé désoxy, de préférence le glucose, le mannose ou le galactose.

32. Utilisation selon la revendication 30, **caractérisée en ce qu'**on utilise les composés de formule générale Ic dans laquelle R² et R³ représentent chacun indépendamment de l'autre un atome d'hydrogène ou un groupe alkyle en C₁ à C₄, et/ou E, dans la formule -CₙF₂ₙE, est un atome de fluor.

33. Utilisation selon la revendication 30, **caractérisée en ce qu'**on utilise les composés de formule générale Ic dans laquelle G représente le résidu lysine (a) ou (b).

34. Utilisation selon la revendication 30, **caractérisée en ce qu'**on utilise les composés de formule générale Ic dans laquelle Z est 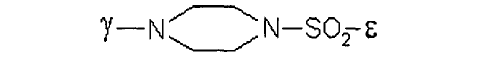 où γ est le point de liaison de Z au radical G et ε est le point de liaison de Z au radical perfluoré R^{F} et/ou Y est δ-CH₂CO-β où δ est le point de liaison au résidu sucre R et β est le point de liaison au radical G.

35. Utilisation selon la revendication 30, **caractérisée en ce qu'**on utilise les composés de formule générale Ic dans laquelle U, dans le complexe métallique K, est -CH₂- ou -C₆H₄-O-CH₂-ω, où ω est le point de liaison à -CO-.

36. Utilisation selon la revendication 30, **caractérisée en ce qu'**on utilise le complexe de gadolinium du 6-N-[1,4,7-tris(carboxylatométhyl)-1,4,7,10-tétraazacyclododécane-10-N-(pentanoyl-3-aza-4-oxo-5-méthyl-5-yl)]-2-N-[1-O-α-D-carbonylméthyl-mannopyrannose]-L-lysine-[1-(4-perfluoroctylsulfonyl)-pipérazine]-amide.

37. Utilisation selon l'une des revendications 1 à 7, **caractérisée en ce qu'**on utilise en tant que complexes métalliques perfluoralkylés les composés comportant des radicaux polaires de formule générale Id 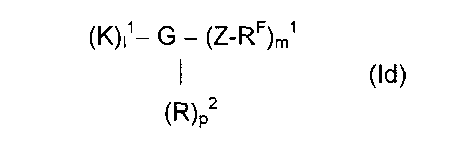 dans laquelle
R^{F} est une chaîne carbonée perfluorée droite ou ramifiée de formule -CₙF₂ₙE dans laquelle E est un atome de fluor, de chlore, de brome, d'iode ou d'hydrogène terminal, et n est un nombre de 4 à 30,
K est un complexe métallique de formule générale IId
dans laquelle
R¹ est un atome d'hydrogène ou un équivalent d'un ion métallique ayant les numéros atomiques 23 à 29, 42 à 46 ou 58 à 70, à la condition qu'au moins deux radicaux R¹ soient des équivalents d'ions métalliques,
R² et R³ représentent chacun indépendamment de l'autre un atome d'hydrogène, un groupe alkyle en C₁ à C₇, benzyle, phényle, -CH₂OH ou -CH₂OCH₃, et
U est -C₆H₄-O-CH₂-ω-, - (CH₂)₁₋₅-ω, un groupe phénylène, -CH₂-NHCO-CH₂-CH(CH₂COOH)-C₆H₄-ω-, -C₆H₄(OCH₂CH₂)₀₋₁*-*N(CH₂COOH)-CH₂-ω, ou un groupe alkylène en C₁ à C₁₂ ou (C₇ à C₁₂)-C₆H₄-O éventuellement interrompu par un ou plusieurs atomes d'oxygène, par 1 à 3 groupes -NHCO-, par 1 à 3 groupes -CONH- et/ou substitué par 1 à 3 groupes -(CH₂)₀₋₅COOH, ω étant le point de liaison à -CO-,
ou
de formule générale IIId 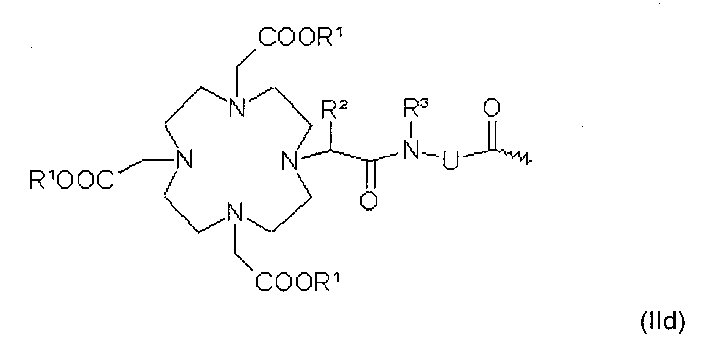 dans laquelle R¹ a les significations données ci-dessus, R⁴ est un atome d'hydrogène ou représente un équivalent d'un ion métallique mentionné à propos de R¹, et U¹ est -C₆H₄-O-CH₂-ω-, ω étant le point de liaison à -CO-,
ou de formule générale IVd 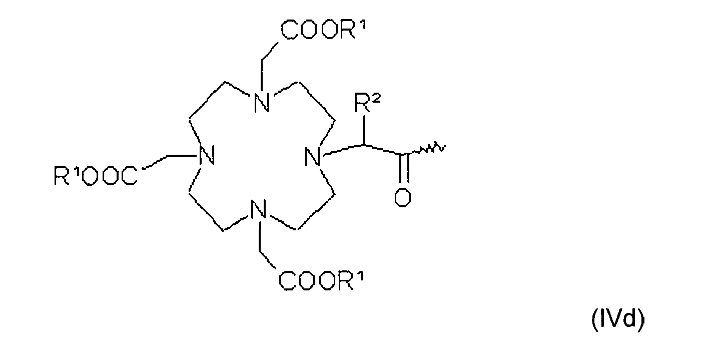 dans laquelle R¹ et R² ont les significations données ci-dessus,
ou de formule générale VdA ou VdB 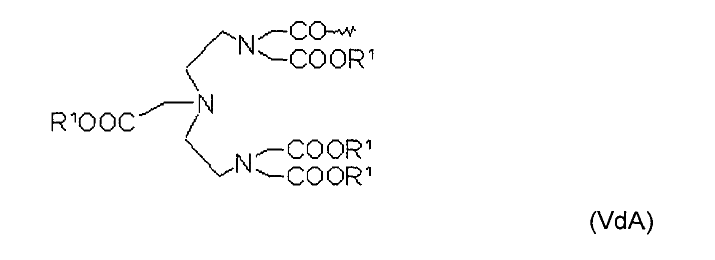 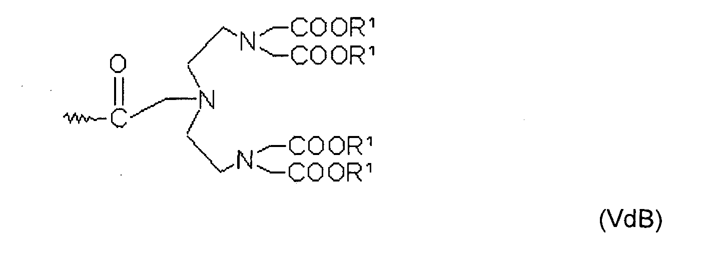 dans laquelle R¹ a les significations données ci-dessus,
ou de formule générale VId 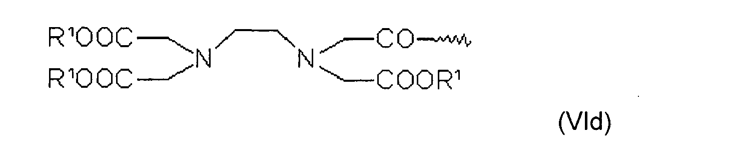 dans laquelle R¹ a les significations données ci-dessus,
ou de formule générale VIId 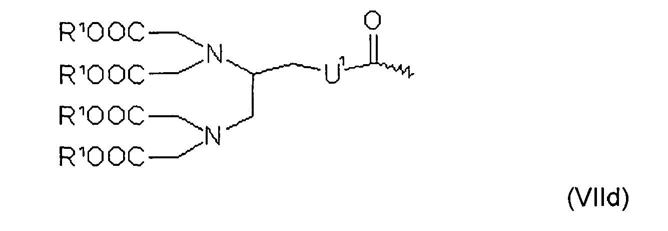 dans laquelle R¹ a les significations données ci-dessus, et U¹ est -C₆H₄-O-CH₂-ω-, ω étant le point de liaison à -CO-,
et les groupes acides libres éventuellement présents dans le radical K peuvent éventuellement se présenter sous forme de sels de bases organiques et/ou inorganiques ou d'acides aminés,
ou d'amides d'acides aminés,
G représente un radical au moins trifonctionnalisé choisi parmi les radicaux a) à i) ci-après : 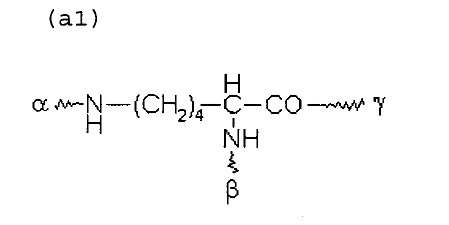 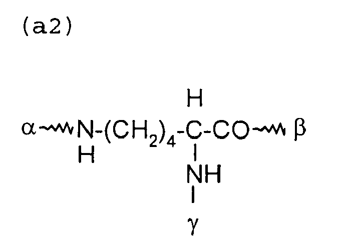 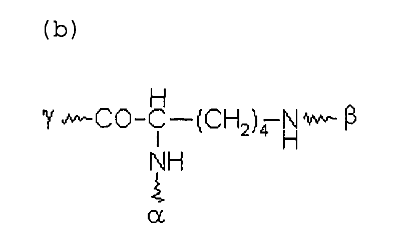 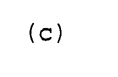 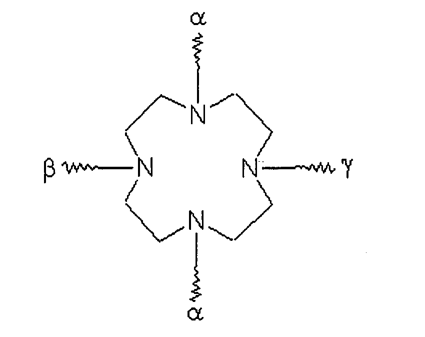 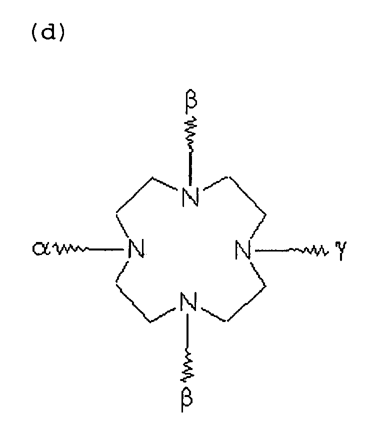 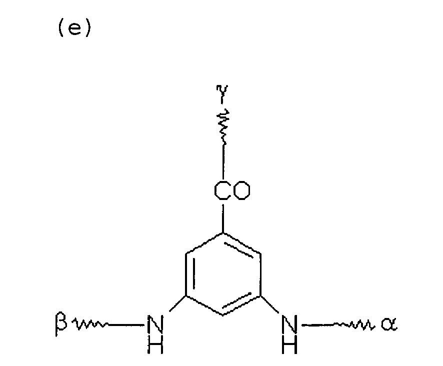 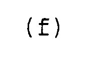 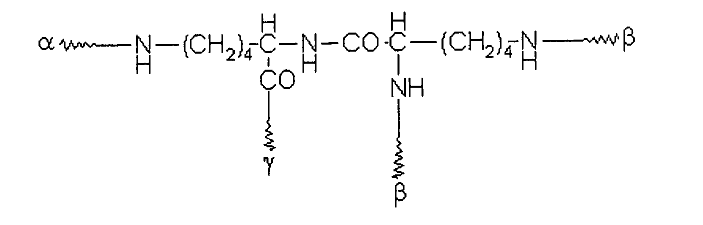 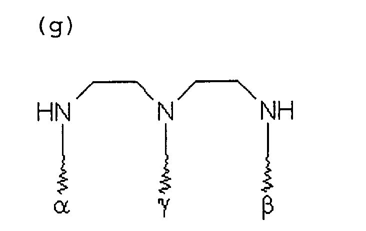 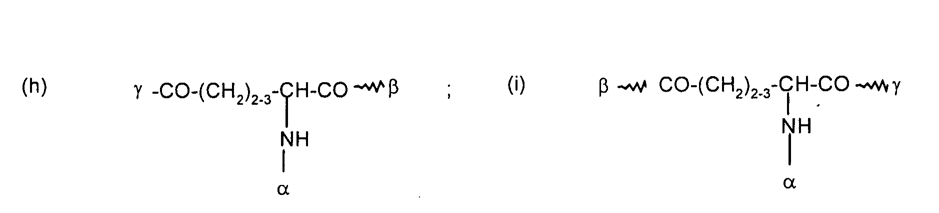 où α est le point de liaison de G au complexe K, β est le point de liaison de G au radical R, et γ est le point de liaison de G au radical Z,
Z représente 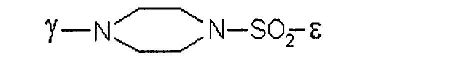 γ -C(O)CH₂O(CH₂)₂-ε,
où γ est le point de liaison de Z au radical G, et ε est le point de liaison de Z au radical perfluoré R^{F},
R est un radical polaire choisi parmi les complexes K de formules générales IId à VIId, où R¹ représente ici un atome d'hydrogène ou un équivalent d'un ion métallique ayant les numéros atomiques 20, 23 à 29, 42 à 46 ou 58 à 70,
et les radicaux R², R³, R⁴, U et U¹ ont les significations données ci-dessus,
ou
le résidu de l'acide folique,
ou
une chaîne carbonée ayant 2 à 30 atomes de carbone, droite ou ramifiée, saturée ou insaturée, liée au radical G par l'intermédiaire de -CO-, de SO₂- ou d'une liaison directe, éventuellement interrompue par 1 à 10 atomes d'oxygène, 1 à 5 groupes -NHCO-, 1 à 5 groupes -CONH-, 1 à 2 atomes de soufre, 1 à 5 groupes -NH- ou 1 à 2 groupes phénylène, qui éventuellement peuvent être substitués par 1 à 2 groupes OH, 1 à 2 groupes NH₂, 1 à 2 groupes -COOH ou 1 à 2 groupes -SO₃H,
ou
éventuellement substituée par 1 à 8 groupes OH, 1 à 5 groupes -COOH, 1 à 2 groupes SO₃H, 1 à 5 groupes -NH₂, 1 à 5 groupes alcoxy en C₁ à C₄,
et
l¹, m¹ et p² représentent chacun indépendamment des autres le nombre entier 1 ou 2.

38. Utilisation selon la revendication 37, **caractérisée en ce qu'**on utilise les composés de formule générale Id dans laquelle K est un complexe métallique de formule générale IId, IIId, VdB ou VIId.

39. Utilisation selon la revendication 37, **caractérisée en ce qu'**on utilise les composés de formule générale Id dans laquelle le radical polaire R a les significations du complexe K, de préférence des complexes K ayant les formules générales IId, IIId, VdA ou VIId.

40. Utilisation selon la revendication 37, **caractérisée en ce qu'**on utilise les composés de formule générale Id dans laquelle le radical polaire R a les significations suivantes :
- C(O)CH₂CH₂SO₃H
- C (O) CH₂OCH₂CH₂OCH₂CH₂OH
- C(O)CH₂OCH₂CH₂OH
- C(O)CH₂OCH₂CH(OH) CH₂OH
- C(O)CH₂NH-C(O)CH₂COOH
- C(O)CH₂CH(OH)CH₂OH
- C (O)CH₂OCH₂COOH
- SO₂CH₂CH₂COOH
- C(O)-C₆H₃-(m-COOH)₂
- C(O)CH₂O(CH₂)₂-C₆H₃-(m-COOH)₂
- C(O)CH₂O-C₆H₄-m-SO₃H
- C(O)CH₂NHC(O)CH₂NHC(O)CH₂OCH₂COOH
- C(O)CH₂OCH₂CH₂OCH₂COOH
- C(O)CH₂OCH₂CH(OH)CH₂O-CH₂CH₂OH
- C (O)CH₂OCH₂CH(OH) CH₂OCH₂-CH(OH)-CH₂OH
- C(O)CH₂SO₃H
- C(O)CH₂CH₂COOH
- C(O)CH(OH)CH(OH)CH₂OH
- C(O)CH₂O[(CH₂)₂O]₁₋₉-CH₃
- C(O)CH₂O[(CH₂)₂O]₁₋₉-H
- C(O)CH₂OCH(CH₂OH)₂
- C(O)CH₂OCH(CH₂OCH₂COOH)₂
- C (O)-C₆H₃-(m-OCH₂COOH)₂
- CO-CH₂O-(CH₂)₂O(CH₂)₂O(CH₂)₂O(CH₂)₂OCH₃
de préférence -C(O)CH₂O[(CH₂)₂O]₄-CH₃.

41. Utilisation selon la revendication 37, **caractérisée en ce qu'**on utilise les composés de formule générale Id dans laquelle le radical polaire R est le résidu de l'acide folique.

42. Utilisation selon la revendication 37, **caractérisée en ce qu'**on utilise les composés de formule générale Id dans laquelle G est le résidu lysine (a) ou (b).

43. Utilisation selon la revendication 37, **caractérisée en ce qu'**on utilise les composés de formule générale Id dans laquelle U, dans le complexe métallique K, représente le groupe -CH₂- ou -C₆H₄-O-CH₂-ω, ω étant le point de liaison à -CO-.

44. Utilisation selon l'une des revendications 37 à 43, **caractérisée en ce qu'**on utilise le complexe de gadolinium du 2,6-N,N'-bis[1,4,7-tris(carboxylatométhyl)-1,4,7,10-tétraazacyclododécane-10-(pentanoyl-3-aza-4-oxo-5-méthyl-5-yl)]-lysine-[1-(4-perfluoroctylsulfonyl-pipérazine]-amide.

45. Utilisation selon l'une des revendications 1 à 7, **caractérisée en ce qu'**on utilise en tant que complexes métalliques perfluoralkylés des formulations galéniques qui contiennent des complexes métalliques perfluoralkylés paramagnétiques ayant les formules générales I, Ia, Ib, Ic et/ou Id et des substances perfluoralkylées diamagnétiques, de préférence dissoutes dans un solvant aqueux.

46. Utilisation selon la revendication 45, **caractérisée en ce qu'**on utilise en tant que substances perfluoralkylées diamagnétiques celles de formule générale XX :
R^{F}-L²-B² (XX)
dans laquelle R^{F} est un radical perfluoralkyle à chaîne droite ou ramifiée ayant 4 à 30 atomes de carbone, L² est un élément de liaison et B² est un groupe hydrophile.

47. Utilisation selon la revendication 46, **caractérisée en ce que** l'élément de liaison L² est une liaison directe, un groupe -SO₂ ou une chaîne carbonée droite ou ramifiée ayant jusqu'à 20 atomes de carbone, qui peut être substituée par un ou plusieurs groupes -OH, -COO⁻, -SO₃, et/ou contient éventuellement un ou plusieurs groupes -O-, -S-, -CO-, -CONH-, -NHCO-, -CONR⁹-, -NR⁹CO-, -SO₂-, -PO₄⁻-, -NH-, -NR⁹-, un noyau aryle ou une pipérazine, où R⁹ est un radical alkyle en C₁ à C₂₀, lequel pour sa part peut contenir un ou plusieurs atomes d'oxygène et/ou peut être substitué par des groupes -COO⁻ ou SO₃.

48. Utilisation selon la revendication 46, **caractérisée en ce que** le groupe hydrophile B² est un mono- ou un disaccharide, un ou plusieurs groupes -COO⁻ ou -SO₃⁻voisins, un acide dicarboxylique, un acide isophtalique, un acide picolique, un acide benzène-sulfonique, un acide tétrahydropyranedicarboxylique, un acide 2,6-pyridinedicarboxylique; un ion ammonium quaternaire, un acide aminopolycarboxylique, un acide aminodipolyéthylèneglycolsulfonique, un groupe aminopolyéthylèneglycol, un groupe SO₂-(CH₂)₂-OH, une chaîne polyhydroxyalkyle ayant au moins deux groupes hydroxyle ou une ou plusieurs chaînes polyéthylèneglycol ayant au moins deux motifs glycol, les chaînes polyéthylèneglycol étant terminées par un groupe -OH ou -OCH₃.

49. Utilisation selon la revendication 45, **caractérisée en ce qu'**on utilise en tant que substances perfluoralkylées diamagnétiques des conjugués d'α-, de β- ou de γ-cyclodextrine et des composés de formule générale XXII
A²-L³-R^{F} (XXII)
dans laquelle A² est une molécule d'adamantane, de biphényle ou d'anthracène, L³ est un élément de liaison et R^{F} est un radical perfluoralkyle à chaîne droite ou ramifiée ayant 4 à 30 atomes de carbone ; et où l' élément de liaison L³ est une chaîne hydrocarbonée droite ayant 1 à 20 atomes de carbone, qui peut être interrompue par un ou plusieurs atomes d'oxygène, un ou plusieurs groupes CO, SO₂, CONH, NHCO, CONR¹⁰, NR¹⁰CO, NH, NR¹⁰, ou par une pipérazine, R¹⁰ étant un radical alkyle en C₁ à C₅.

50. Utilisation selon la revendication 45, **caractérisée en ce qu'**on utilise en tant que substances perfluoralkylées diamagnétiques celles de formule générale XXI
R^{F}-X¹ (XXI)
dans laquelle R^{F} est un radical perfluoralkyle à chaîne droite ou ramifiée ayant 4 à 30 atomes de carbone, et X¹ est un radical choisi dans l'ensemble des radicaux suivants (n est alors un nombre compris entre 1 et 10) :
